# EUROPEAN PATENT SPECIFICATION

(11) **EP 2 265 187 B1**
(45) Date of publication and mention of the grant of the patent: **29.07.2020**
(21) Application number: 09719018.5
(22) Date of filing: 13.03.2009
(51) Int. Cl.: A61B 17/04, A61B 90/92, A61B 90/94, A61B 17/06, A61B 17/02, A61B 17/11, A61B 50/00

(54) **SECUREMENT APPARATUS**
SICHERUNGSGERÄT
APPAREIL DE FIXATION

(30) Priority: 14.03.2008 US 36916 P; 14.03.2008 US 36903 P; 18.04.2008 US 106204; 29.08.2008 US 202073; 26.09.2008 US 100636 P; 13.03.2009 US 404227
(43) Date of publication of application: 29.12.2010
(73) Proprietor: Merit Medical Systems, Inc., South Jordan UT 84095 (US)
(72) Inventor: LAMPROPOULOS, Fred, P, Salt Lake City UT 84117 (US); CLARK, Timothy, W, Philadelphia PA 19128 (US); MCARTHUR, Gregory, R, Sandy UT 84093 (US); ALBO, Dominic, Dr., Salt Lake City UT 84102 (US)
(74) Representative: Bradley, Adrian
(86) International application number: PCT/US2009/037168
(87) International publication number: WO 2009/114811

(56) References cited:
- WO-A2-2005/122912
- US-A- 5 800 447
- US-A- 5 911 728
- US-A1- 2001 031 873
- US-A1- 2003 028 203
- US-A1- 2004 087 099
- US-A1- 2005 235 778
- US-A1- 2006 030 886
- US-A1- 2007 142 788
- US-A1- 2007 218 487
- US-A1- 2007 219 467

## Description

### BACKGROUND OF THE INVENTION

### Field of the Invention

The present disclosure relates to a suture securement apparatus. In more particular, the present relates to a suture securement apparatus which is utilized in connection with a threading assembly to provide desired securement of a percutaneous catheter insertion site and/or a drainage catheter apparatus. Additionally, the present disclosure relates to a surgical securement apparatus configured to provide desired gripping of a securement cord that is positioned around one or more intracorporeal structures during a surgical procedure. Additionally, the present disclosure relates to a securement and marking system configured to facilitate securement, isolation, and identification of intracorporeal structures. Lastly, the present disclosure relates to a configuration for efficient and effective shipping and storage of a securement apparatus.

### Relevant Technology

Some of the challenges encountered with a percutaneous catheter insertion relate to the maintaining of the catheter within the catheter insertion site and also the proper maintenance of hemostasis subsequent to removal of the catheter from the catheter insertion site. A variety of different suturing techniques have been utilized in connection with the puncture wound of percutaneous catheter insertion sites and the maintenance of catheters within the percutaneous catheter insertion sites. However, inadvertent slipping or removal of catheters and desired closure of puncture wounds of percutaneous catheter insertion sites subsequent to removal of such catheters has resulted in undesirable patient discomfort and at times a partial or total loss of hemostasis.

One of the techniques often utilized with percutaneous catheter insertion sites is the use of a purse string suture. A purse string suture is formed by inserting a suture, such as a monofilament or braided thread into the patient's skin at a position adjacent the insertion site. The practitioner then forms a circular series of continuous stitches which parallel the edges of the wound in a substantially circular pattern. The configuration of the stitches results in a plurality of segments of suture which alternate between being threaded under a portion of the patient's skin and being positioned over the surface of the patient's skin such that the stitch has between three and four segments or stitches which are positioned above the skin around the edges of the insertion site. A tail of the suture is left both at the initial insertion site of the suture and also at the emergent site of the suture such that the purse string suture appears to have two tails which emerge somewhat close in proximity to one another at a predetermined point around the edges of the insertion site.

The purse string sutures allow a practitioner to close the puncture wound of the insertion site by simply pulling on the two ends of the suture, thus tightening the suture which is then threaded around the perimeter of the insertion site effectively closing the puncture wound of the insertion site and providing the desired hemostasis. In this manner, excessive compression and other techniques which are typically utilized to stop puncture wound bleeding are not needed.

One of the challenges which has been experienced with purse string suture closure methods and the desired maintenance of hemostasis utilizing the purse string suture relates to the knotting or other techniques for maintaining tension on the adjacent ends of the purse string suture. One mechanism which has been utilized employs a knot at one end of one suture which engages the other suture to maintain the desired tension of the overall length of the purse string suture. However, the knot utilized in connection with such techniques can compromise the integrity of the purse string suture. Additionally, the knot can make subsequent loosening and retightening of the purse string suture difficult if not impractical. Additionally, non-doctor practitioners may be unwilling or uncomfortable with removing such sutures at the end of the procedure. As a result, the doctor must see the patient to remove the suture, or the patient may be required to come in for an office visit that may be inconvenient or otherwise unnecessary.

Another technique which has been utilized is to attempt to utilize a secondary securement apparatus to secure the ends of the suture. However, such techniques utilizing existing secondary securement apparatus often result in excessive tension on the purse string suture which can cause puckering or unnatural distortion of the patient's skin adjacent the purse string suture site. As a result, unnecessary discomfort and/or damage to the patient tissue can result. Moreover, existing secondary securement apparatus can compromise the integrity of the suture, giving rise to the potential that the suture will break. If the suture breaks, there is a risk that hemostasis at the insertion site will be lost and/or slipping of the catheter will occur resulting in partial or total removal of the catheter from the insertion site.

Another application of a securement apparatus relates to surgical procedures. A variety of surgical procedures require a practitioner to gain access to a patient's thoracic cavity, abdominal cavity or another location within the patient. These procedures often necessitate separating, identifying, and grouping various intracorporeal structures, such as nerves, blood vessels (arteries and veins), and tendons. Sorting the intracorporeal structures allows the practitioner to identify the particular structures to be repaired and to guard against accidental operation on, or damage to, such structures not involved with the procedure. During the course of a surgery, the practitioner may isolate one or more target intracorporeal structures to perform particularized procedures on the structures. A practitioner may also identify various intracorporeal structures that are not being repaired and tie them together or otherwise secure them safely on the periphery of the surgical site. In this manner accidental cutting or otherwise damaging such structures can be avoided.

A variety of techniques have been utilized to secure and, at times, to isolate nerves, vessels, and tendons during a surgical procedure. The techniques generally involve providing a securement loop around the structures to be secured. Typically the securement loop is made of a soft woven tape or cord, such as umbilical tape, that will not inadvertently cut, slice, or otherwise damage the structures. Moreover, the techniques can include the use of ties and clamps to secure the securement cord around the structures.

One particular technique that has traditionally been utilized is simply looping a short section of cord around a target structure and securing the cord utilizing a knot. For description purposes, the cord and knot can be referred to as a tie. A significant shortcoming of this technique is the possibility of the tie falling into the surgical area. Blood and fluids in the surgical area can cover or otherwise obscure the tie such that the practitioner may fail to remove the tie at the end of the procedure. Leaving a tie inside a patient after a surgery can cause discomfort and damage and increase the possibility of infection. Another significant shortcoming of a simple tie arrangement is that the knot of the tie can be difficult to release once the procedure is completed. Leaving the ends of the cord long enough to extend outside the surgical site can help prevent accidental loss of the tie. However, the additional length of cord introduces challenges in initially forming the knot. Furthermore, the additional length of cord does not help resolve the problems associated with releasing the knot. In light of these shortcomings other techniques have been developed to secure intracorporeal structures during a medical procedure.

Another technique used to secure structures from outside the surgical site employs a section of tubing in connection with a securement cord. The securement cord is threaded in a loop around a target structure. After looping the securement cord around the target structure, the ends of the cord are then threaded through the section of tubing. A distal end of the tubing can be positioned near the structure and engage the cord to enable cinching or tightening of the loop around the structure. A hemostat or clamp can then be placed at the proximal end of the tubing to secure the position of tubing along the loop of cord. The proximal end of the tubing and the hemostat or clamp can remain safely outside the surgical site and the distal end of the section of tubing can hold the loop of securement cord taut around the structure.

The use of this type of securement arrangement utilizing tubing and a hemostat or another clamp, can have several shortcomings. The securement cord loop cannot be pre-threaded through the tubing due to the fact that many of the target structures do not have identifiable or accessible endings around which a pre-formed loop could be threaded. Moreover, quickly and efficiently threading the securement cord through the tubing during the surgical procedure is challenging. For example, introducing one or both ends of the securement cord into a section of tubing with a small diameter can be complicated and cumbersome. Another shortcoming is that the weight of the hemostat or other clamping device can potentially result in excessive strain on the structures. US5911728 relates to a cannula purse string suture clamping device with shortcomings such as those discussed above.

### BRIEF SUMMARY

The invention provides a suture securement apparatus according to claim 1. Further embodiments of the invention are provided in the dependent claims. The present disclosure relates to a suture securement apparatus for selectively securing one or more ends of a suture while allowing adjustments in the tension or a full release of the sutures intermittently after a prolonged period of time. The suture securement apparatus can be particularly adapted for use with a purse-string suture to close a percutaneous catheter puncture site without causing puckering or distortion of the skin at the purse string suture site. The suture securement apparatus permits a practitioner to subsequently modify the amount of tensioning or permits full release of the sutures at the catheter insertion site. The present disclosure also relates to a threading assembly for use with the suture securement apparatus which is adapted to facilitate threading of sutures along the length of the suture securement apparatus.

According to one embodiment of the present disclosure, the suture securement apparatus includes an extension tube for facilitating tensioning of the sutures at the suture insertion site. The length and shape of the extension tube also permits positioning of the tip of the extension tube adjacent the suture insertion site. According to another embodiment of the present disclosure, a threading assembly is provided in connection with the suture securement assembly. The threading assembly allows a practitioner to quickly and simply thread one or more tails of the suture from the exterior of the suture securement apparatus and through a lumen of the suture securement apparatus such that desired engagement of such sutures is facilitated.

According to one embodiment of the present disclosure, a clasp and suture loop are provided as part of the threading assembly. The clasp prevents the accidental pulling of the suture loop through the suture securement apparatus as a result of the tension exerted on the portion of the suture loop positioned distal to the suture securement apparatus. Additionally, the clasp provides a simple and ergonomic mechanism to allow a user to control functioning of the threading assembly and of the suture securement loop.

The present disclosure also relates to a suture securement apparatus and adhesive suture securement devices which can be utilized to provide desired securement of a drainage catheter apparatus and/or closure of a percutaneous catheter insertion site. A suture securement apparatus can be configured to selectively secure one or more ends of a suture while allowing adjustments in the tension or a full release of the suture in a quick, simple and effective manner. The suture securement apparatus can be utilized in connection with a purse string suture at a percutaneous catheter insertion site to achieve desired hemostasis. A threading assembly can be utilized to quickly and simply thread one or more tails of the purse string suture through a lumen of the suture securement apparatus to enable engagement of the tails of the suture. The extension tube of the suture securement apparatus can be configured to facilitate drawing the tails of the purse string suture together to achieve and maintain a desired degree of tension to close the insertion site and achieve hemostasis. Moreover, pressing the release button of the suture securement apparatus can release the suture to permit a practitioner to subsequently modify the amount of tension, to alter the position of the suture securement apparatus relative to the patient, or to fully release the suture.

The present disclosure also relates to a threading assembly which can be utilized in connection with a suture securement apparatus to thread a suture through a lumen of the suture securement apparatus and/or a suture ring of a catheter hub. The threading assembly comprises a clasp and a wire. The wire has one or more pre-formed bends to form an insertion assembly having a pointed tip and one or more angular portions defining an opening through which sutures can easily be inserted. The threading assembly is inserted through the lumen of the suture securement apparatus such that at least a portion of the opening of the insertion assembly is positioned distal to the suture securement apparatus. The ends of a suture can be inserted through the opening, such that as the threading assembly is drawn through the lumen of the suture securement apparatus the ends of the suture are also drawn through the lumen of the suture securement apparatus.

The present disclosure also relates to an adhesive suture securement device for providing securing sutures which can be utilized during a procedure without requiring insertion of the sutures through the patient's skin. The adhesive suture securement device has an adhesive base and one or more sutures secured to the adhesive base by an anchor. A practitioner can secure the adhesive base to the skin of a patient and the one or more sutures can then be utilized, for example, to secure a suture ring of a catheter hub relative to the patient. A suture securement apparatus and threading assembly can be utilized to thread the sutures through the ring of the catheter hub and thereby secure the catheter hub relative to the patient.

The present disclosure also relates to an adhesive securement device having a cradle in which a hub of a catheter can be secured. The adhesive securement device has an adhesive pad and a cradle configured to receive a hub of a catheter. The cradle can be secured to the adhesive base by a cradle base. The cradle can be configured such that the hub can snap into the cradle, thereby securing the hub relative to the skin of the patient.

The present disclosure also relates to a surgical securement apparatus configured to selectively secure one or more ends of a cord for securing various intracorporeal structures while allowing adjustments in the tension or a full release of the cord in a quick, simple and effective manner. The surgical securement apparatus can be particularly adapted for use with a cord that is utilized to sort and secure various intracorporeal structures such as nerves, blood vessels, and tendons during a surgical procedure. The body of the surgical securement apparatus remains outside the surgical site while an extender tube enables securement of structures within the surgical site. The surgical securement apparatus permits a practitioner to subsequently modify the amount of tension or to fully release the cord. According to one embodiment of the present disclosure, the surgical securement apparatus includes an extender tube, which enables tensioning of the cord adjacent to intracorporeal structures within a surgical site while allowing the body of the surgical securement apparatus to remain outside the surgical site.

The present disclosure also relates to a release button having a shipping configuration that enables effective transport and storage of the surgical securement apparatus while reducing or eliminating strain on the internal biasing element until the release button is engaged during a surgical procedure. According to one embodiment of the present disclosure, a release button is provided having an actuator to activate the release button from a shipping configuration to an activated configuration. The shipping configuration reduces or eliminates strain on an internal biasing element to preserve the strength of the biasing force. The activated configuration positions the button and the internal biasing element such that forces from the internal biasing element can be utilized to secure a cord within the body of the surgery securement apparatus.

The present disclosure also relates to a threading assembly for use with the surgical securement apparatus that is adapted to facilitate threading of the cord along the length of the surgical securement apparatus. The threading assembly allows a practitioner to quickly and simply thread one or more tails of the securement cord from the exterior of the surgical securement apparatus, through a lumen of the extender tube and the body of the surgical securement apparatus such that desired engagement of the securement cord is facilitated.

The present disclosure also relates to a surgical securement apparatus having an extender tube trimming device for easily adjusting the length of the extender tube to an appropriate length prior to using the surgical securement apparatus and without cutting a threading loop that is threaded through the extender tube. The trimming device allows for easy adjustment of the length of the extender tubing. A practitioner can trim the extender tube without cutting a pre-threaded threading loop of a threading assembly.

The present disclosure also relates to a system for marking and securing intracorporeal structures. The system provides an indicator that enables a practitioner to readily identify which intracorporeal structure is associated with a particular securement apparatus. For example, according to one aspect of the present disclosure, non-textual indicia such as color is provided on the body of a surgical securement apparatus to allow a practitioner to identify the structures associated with that surgical securement apparatus. According to one embodiment of the present disclosure, a surgical securement and marking system is provided to enable a practitioner to readily identify various intracorporeal structures. The system includes one or more surgical securement apparatus having integrated non-textual indicia, such as color, to convey to the practitioner information to allow the practitioner to readily identify an associated sorted and secured intracorporeal structure without needing to independently observe or examine the particular structure. Other non-textual indicia that may be integrated with the surgical securement apparatus include symbols and patterns. The surgical securement system can provide an indication of a particular category, structure name, or other distinguishing characteristic of the structure utilizing indicia on, or the characteristics of, the individual surgical securement apparatus within the system. For example, in one embodiment, a plurality of surgical securement apparatus are provided. Non-textual indicia such as color-coding, is provided on each of the surgical securement apparatus. A practitioner can secure structures utilizing color-coding that allows ready determination that a surgical securement apparatus of a particular color is associated with a particular structure. The invention is described by reference to the claims.

### BRIEF DESCRIPTION OF THE DRAWINGS

Embodiments of the suture securement apparatus according to the present invention as defined in the appended claims are described in conjunction with Figures 1 to 9. Other figures that are described herein illustrate additional components or provide further context for the invention.
Fig. 1 is a perspective view of a suture securement apparatus utilized in connection with a threading assembly for maintaining hemostasis at a puncture site, according to one embodiment of the present disclosure.
Fig. 2 is a cross-sectional side view of the suture securement apparatus illustrating operation of the threading assembly for threading sutures through the suture securement apparatus according to one aspect of the present disclosure.
Fig. 3 is a perspective view of the suture securement apparatus illustrating the manner in which the suture securement apparatus maintains a desired degree of tension on the purse string sutures at a puncture wound site.
Fig. 4 is a close-up perspective view of an extension tube illustrating the juxtaposition of the extension tube relative to the tails of the suture at a puncture wound site.
Figs. 5 and 5A illustrate a suture securement apparatus having a threading assembly adapted for use with a suture ring of a catheter hub according to one embodiment of the present disclosure.
Fig. 6 illustrates the manner in which the threading assembly of the suture securement apparatus is utilized to draw the suture tails through the suture ring of the hub assembly and through the lumen of the suture securement apparatus.
Fig. 7 illustrates the suture securement apparatus operating to secure the suture, and thereby secure the suture ring to maintain the position of the catheter hub relative to the patient.
Fig. 8 is an end view of the suture securement apparatus according to one embodiment of the present disclosure.
Fig. 9 is an end view of the suture securement apparatus of Fig. 8 illustrating the manner by which the suture securement apparatus engages the tails of the suture positioned within a lumen of the suture securement apparatus according to one embodiment of the present disclosure.
Fig. 10 is a perspective view of an adhesive suture securement device for providing securing sutures which can be utilized to secure a suture ring of a catheter hub without requiring insertion of the sutures through the patient's skin.
Fig. 11 is a perspective view of an adhesive suture securement device according to an alternative embodiment of the present disclosure.
Fig. 12 is a perspective view of an adhesive securement device having a basket securement member for securing a catheter hub relative to a patient without requiring insertion of a suture into a patient's skin according to another embodiment of the present disclosure.
Fig. 13 is a perspective view of a surgical securement apparatus utilized in connection with a threading assembly to secure intracorporeal structures during a surgical procedure.
Fig. 14 is an exploded view of a surgical securement apparatus.
Fig. 15A is a perspective view of a body of a surgical securement apparatus having a release button in a transport configuration.
Fig. 15B is a perspective view of a body of a surgical securement apparatus, the release button being activated.
Fig. 15C is a perspective view of a body of a surgical securement apparatus having an activated release button.
Fig. 16A is a cross-sectional side view of a body of a surgical securement apparatus having a release button in a transport configuration.
Fig. 16B is a cross-sectional side view of a body of a surgical securement apparatus, the release button being activated.
Fig. 16C is a cross-sectional side view of a body of a surgical securement apparatus having an activated release button.
Fig. 17A is a cross-sectional front view of a body of a surgical securement apparatus having a release button in a transport configuration.
Fig. 17B is a cross-sectional front view of a body of a surgical securement apparatus, the release button being activated.
Fig. 17C is a cross-sectional front view of a body of a surgical securement apparatus having an activated release button.
Fig. 18A is a cross-sectional side view of a surgical securement apparatus illustrating a threading assembly positioned within the surgical securement apparatus.
Fig. 18B is a cross-sectional side view of a surgical securement apparatus in which the release button is depressed and threading assembly is being utilized to engage a securement cord.
Fig. 18C is a cross-sectional side view of a surgical securement apparatus in which the securement cord is being threaded through the surgical securement apparatus.
Fig. 18D is a cross-sectional side view of a surgical securement apparatus illustrating a securement cord being secured within the surgical securement apparatus.
Fig. 19A is a perspective front view of an extender tube trimming device according to one embodiment of the present disclosure.
Fig. 19B is another perspective front view of the extender tube trimming device of Fig. 19A.
Fig. 19C is cross-sectional front view of the extender tube trimming device of Fig. 19A in an open position.
Fig. 19D is cross-sectional front view of the extender tube trimming device of Fig. 19A in a closed position.
Fig. 20A is a perspective front view of an extender tube trimming device according to another embodiment of the present disclosure.
Fig. 20B is another perspective front view of the extender tube trimming device of Fig. 20A.
Fig. 20C is cross-sectional front view of the extender tube trimming device of Fig. 20A.
Fig. 20D is another cross-sectional front view of the extender tube trimming device of Fig. 20A with blades engaged and cutting an extender tube positioned through the extender tube trimming device.
Fig. 21A is a perspective front view of an extender tube trimming device according to another embodiment of the present disclosure.
Fig. 21B is another perspective front view of the extender tube trimming device of Fig. 21A.
Fig. 22 is a perspective view of a surgical securement apparatus being utilized in connection with a surgical procedure.
Fig. 23 is a perspective view of a surgical securement apparatus positioned to secure a securement cord with a desired degree of tension around a structure to be secured during a surgical procedure.
Fig. 24 is a perspective view of a surgical securement apparatus utilized in connection with a threading assembly to secure intracorporeal structures during a surgical procedure.
Fig. 25 is an exploded view of a surgical securement apparatus.
Fig. 26A is a cross-sectional side view of a surgical securement apparatus illustrating a threading assembly positioned within the surgical securement apparatus.
Fig. 26B is a cross-sectional side view of a surgical securement apparatus in which the release button is depressed and threading assembly is being utilized engage a securement cord.
Fig. 26C is a cross-sectional side view of a surgical securement apparatus in which the securement cord is being threaded through the surgical securement apparatus.
Fig. 27 is a perspective view of a surgical securement apparatus being utilized in connection with a surgical procedure.
Fig. 28 is a perspective view of a surgical securement apparatus positioned to secure a securement cord with a desired degree of tension around a structure to be secured during a surgical procedure.
Fig. 29 is a perspective view of a surgical securement and marking system employing color-coded surgical securement apparatus to sort and secure various intracorporeal structures during a surgical procedure.

Reference signs in the claims are not to be construed as limiting the extent of the matter protected by the claims.

### DETAILED DESCRIPTION

Some of the challenges encountered with percutaneous catheter insertion relate to the maintaining of the catheter within the catheter insertion site and also the proper maintenance of hemostasis of the puncture wound of the insertion site subsequent to removal of the catheter from the insertion site. In some cases, the drainage catheter is left in place for a period of time, remaining inserted for use during subsequent procedures. When the drainage catheter remains inserted for extended periods, it can be important to secure the end or hub of the catheter relative to the skin of the patient to guard against inadvertent movement of the catheter or removal of the catheter from the insertion site. Moreover, achieving hemostasis at the insertion site while the catheter is inserted and after the catheter is removed guards against blood loss and transfer of disease both to and from the patient.

A variety of different suturing techniques have been utilized in connection with percutaneous catheter insertion sites and the maintenance of catheters within the percutaneous catheter insertion sites. However, inadvertent slipping or removal of catheters and closure of percutaneous catheter insertion sites subsequent to removal of such catheters has resulted in undesirable patient discomfort and at times a partial or total loss of hemostasis. As an example, practitioners commonly utilize a purse string suture at an insertion site as a technique to secure a drainage catheter and/or maintain hemostasis at the insertion site both while the catheter is inserted and after the catheter is removed. A purse string suture allows a practitioner to close the insertion site by simply pulling on the two ends or tails of the suture, thus tightening the suture. However, a challenge experienced when utilizing a purse string suture is maintaining tension on the ends of the purse string suture. Knotting the ends of the suture is problematic because the purse string cannot be easily loosened and subsequently retightened. Use of an existing secondary securement apparatus to secure the ends of the suture often results in excessive tension on the purse string suture, which can cause puckering or unnatural distortion of the patient's skin adjacent the purse string suture site. Moreover, existing secondary securement apparatus can be awkward to manipulate and can compromise the integrity of the suture.

An additional challenge of using a purse string suture is encountered when the condition of the patient's skin is poor (e.g., due to injury, disease, neglect, etc.). In these situations, the patient's skin near the insertion site may be thin or of such poor quality that the skin cannot accommodate the purse string suture and tears as a result of tension on the suture when the suture is tightened. Merely securing a drainage catheter, to guard against inadvertent slipping and prevent additional damage to the skin, becomes a significant challenge. The invention is described by reference to the claims.

The present disclosure relates to a suture securement apparatus for selectively securing one or more ends of a suture while allowing adjustments in the tension or a full release of the sutures intermittently after a prolonged period of time. The suture securement apparatus can be particularly adapted for use with a purse-string suture to close a percutaneous catheter puncture site without causing puckering or distortion of the skin at the purse string suture site. The suture securement apparatus permits a practitioner to subsequently modify the amount of tensioning or full release of the sutures at the catheter insertion site. The present disclosure also relates to a threading assembly for use with the suture securement apparatus and being adapted to facilitate threading of sutures along the length of the suture securement apparatus.

According to one embodiment of the present disclosure, the suture securement apparatus includes an extension tube for facilitating tensioning of the sutures at the suture insertion site. The length and shape of the extension tube also permits positioning of the tip of the extension tube adjacent the suture insertion site. According to another embodiment of the present disclosure, a threading assembly is provided in connection with the suture securement apparatus. The threading assembly allows a practitioner to quickly and simply thread one or more tails of the suture from the exterior of the suture securement apparatus and through a lumen of the suture securement apparatus such that desired engagement of such suture tails is effectuated.

According to one embodiment of the present disclosure, a clasp and suture loop are provided as part of the threading assembly. The clasp prevents the accidental pulling of the suture loop through the suture securement apparatus as a result of the tension exerted on the portion of the suture loop positioned distal to the suture securement apparatus. Additionally, the clasp provides a simple and ergonomical mechanism to allow a user to control functioning of threading assembly and of the suture securement loop.

The present disclosure also relates to a suture securement apparatus and adhesive suture securement devices that can be utilized in connection with a threading assembly to provide desired securement of a drainage catheter apparatus and/or maintain hemostasis at a percutaneous catheter insertion site.

Another application of a securement apparatus according to one embodiment of the present disclosure, relates to surgical procedures. Surgical procedures accessing the thoracic or abdominal cavity can require separating, sorting, and grouping various intracorporeal structures, including nerves, blood vessels (arteries and veins), and tendons. This process of separating, sorting, and grouping structures allows the practitioner to identify the particular target structures to be repaired. The process also allows the practitioner to separate peripheral structures not involved with the procedure to guard against accidental puncture, incision, or damage of such peripheral structures. The present disclosure enables a practitioner, who is performing a medical procedure on one or more particular intracorporeal structures, to identify such one or more target structures and separate them from other structures using a securement cord. The practitioner may also identify various intracorporeal structures that are not being repaired and secure them safely on the periphery of the surgical site.

The present disclosure relates to a surgical securement apparatus to selectively secure one or more ends of a securement cord adapted to secure intracorporeal structures. The selective securement of the cord allows adjustments in the tension or a full release of the cord in a simple and effective manner. The body of the surgical securement apparatus provides the securement function. The body can remain outside the surgical site while an extender tube extends into the surgical site to enable securement of intracorporeal structures within the surgical site. This permits a practitioner to adjust the tension or to fully release the cord from outside the surgical site.

In one embodiment, a release button is provided that has an actuator that can activate the release button from a shipping configuration to an activated configuration. The shipping configuration reduces or eliminates tension on an internal biasing element and preserves the strength and the integrity of the biasing element until the surgical securement apparatus is to be utilized. This pressures the biasing force to grip and secure the cord when the release button is activated. The activated configuration, by contrast, is adapted to attempt to maximize the force applied by the biasing element to secure a cord within the body of the surgical securement apparatus.

A threading assembly for use with the surgical securement apparatus is also provided. The threading assembly is adapted to facilitate threading of the cord along the length of the surgical securement apparatus. The threading assembly can be pre-threaded through the length of the surgical securement apparatus. The threading assembly can then be used to capture the ends of a securement cord, pull the cord, and thereby thread the cord along the length of the surgical securement apparatus.

An extender tube trimming device is also provided that can be used to trim extender tube to a desired length. The extender tube trimming device can be coupled to the extender tube to be readily accessible to a practitioner and to enable quick, efficient and safe trimming of the extender tube. The extender tube trimming device can have one or more blades adapted to cut the tubing without cutting the pre-threaded sutures positioned in the extender tube.

Also provided is a system which provides an indicator that enables a practitioner to readily identify which intracorporeal structures are associated with a particular surgical securement apparatus. The system can comprise one or more surgical securement apparatus with non-textual indicia such as color integrated thereon. The color or other non-textual indicia convey to a practitioner information about the associated secured intracorporeal structure(s), enabling the practitioner to readily identify associated structure(s). The non-textual indicial allows the practitioner to identify the associated structures without requiring independent observation or examination of the associated structures.

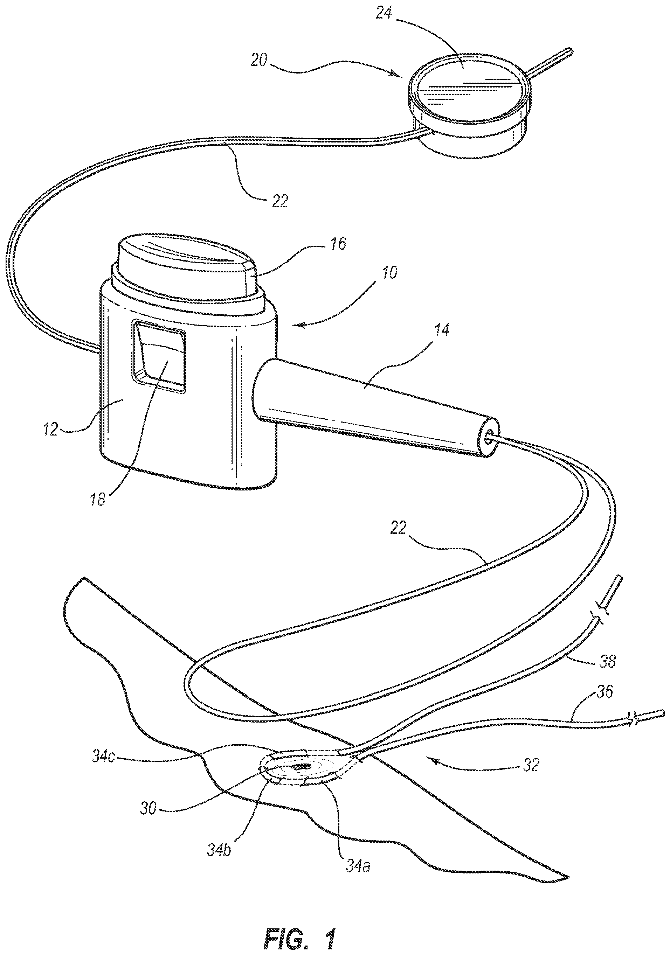

Fig. 1 is a perspective view of a suture securement apparatus according to one embodiment of the present disclosure. Suture securement apparatus 10 is utilized to selectively and releasably secure the ends of the suture provided in connection with various different types of securement procedures. In the illustrated embodiment, the sutures are utilized with a purse string suture to maintain hemostasis and close a percutaneous catheter puncture site. Suture securement apparatus 10 comprises a body 12, an extension tube 14, release button 16, and an assembly window 18. Body 12 comprises a housing for securing the other components of suture securement apparatus 10 and for maintaining desired operability of the components of suture securement apparatus 10. Extension tube 14 extends distally from body 12. Extension tube 14 provides a mechanism for engaging the sutures positioned adjacent a wound 30 without interruption or interference from body 12 of suture securement apparatus 10. In other words, the length of extension tube 14 allows the tip to be positioned adjacent the suture insertion site to maintain a desired degree of tension on the sutures without interruption from body 12 of suture securement apparatus 10.

Release button 16 is positioned within body 12 of suture securement apparatus 10. Release button 16 allows a practitioner to selectively secure or release sutures positioned within suture securement apparatus 10. For example, in the illustrated embodiment when release button 16 is in a released position, suture securement apparatus is securely gripping any sutures positioned within suture securement apparatus 10. When the user depresses release button 16, the grip exerted by suture securement apparatus 10 on any sutures positioned within suture securement apparatus 10 is loosened, and manipulation of the sutures or adjustment of the suture securement apparatus 10 relative to the sutures can be effectuated. The ability to selectively release and reposition the suture securement apparatus 10 relative to sutures for which the suture securement apparatus 10 is to be utilized can be desirable to the extent the practitioner desires to adjust the tension, reposition the suture securement apparatus 10 relative to the patient, or perform other desired activities relative to the sutures and/or the suture securement apparatus 10.

Assembly window 18 comprises an aperture in the sidewall of body 12 of suture securement apparatus 10. Assembly window 18 allows for the quick snap fit assembly of release button 16 relative to body 12 of suture securement apparatus 10. In the illustrated embodiment, release button 16 includes a biasing flange having a transverse length approximating the width of assembly window 18. During assembly, release button 16 is lowered into body 12 of suture securement apparatus 10. As release button 16 is urged downward, the biasing flange is received within assembly window 18 preventing inadvertent removal of release button 16 from body 12.

In the illustrated embodiment, suture securement apparatus 10 is utilized with threading assembly 20. Threading assembly 20 allows a practitioner to quickly and simply thread one or more tails of suture 32 from the exterior of suture securement apparatus 10 and through a lumen of the suture securement apparatus 10 such that desired engagement of such sutures is effectuated. In the illustrated embodiment, threading assembly 20 comprises a suture loop 22 and a clasp 24. Suture loop 22 extends from a proximal portion of suture securement apparatus 10, along the length of a lumen of suture securement apparatus, and extends distally from an end of extension tube 14.

In the illustrated embodiment, clasp 24 includes a first lateral side and second lateral side. Cooperative engagement of the first lateral side and second lateral side secures a portion of suture loop 22 within clasp 24. Clasp 24 secures the end of suture loop 22 positioned on the proximal side of suture securement apparatus 10. Clasp 24 prevents the accidental pulling of suture loop 22 through suture securement apparatus 10 as a result of tension exerted on the portion of suture loop 22 positioned distal to suture securement apparatus 10. Additionally, clasp 24 provides a simple and ergonomic mechanism to allow a user to control functioning of threading assembly and of suture securement loop 22. According to one embodiment of the present disclosure, clasp 24 includes texturing or finger grips to allow for desired gripping of clasp 24.

In the illustrated embodiment, a suture 32 is utilized in connection with a puncture site 30. As will be appreciated by those skilled in the art, a variety of types and configurations of sutures can be utilized with a variety of types of puncture sites. For example, a percutaneous catheter puncture site can result in a round or substantially circular wound similar to that depicted as puncture site 30. Suture 32 is configured as a purse string suture. As a result, a plurality of purse string segments 34a, b, c are depicted being positioned around the perimeter of puncture site 30. A first tail is shown extending away from puncture 30. A second tail 38 is also depicted as extending away from puncture site 30. First tail 36 and second tail 38 are positioned adjacent to one another but in sufficient spatial relationship so as to allow the drawing of first tail 36 closer to second tail 38 to achieve a desired degree of tension on purse string segments 34a, b, c. In the illustrated embodiment, a user can utilize suture securement apparatus 10 to achieve a desired degree of tension on the purse string configuration of suture 32.

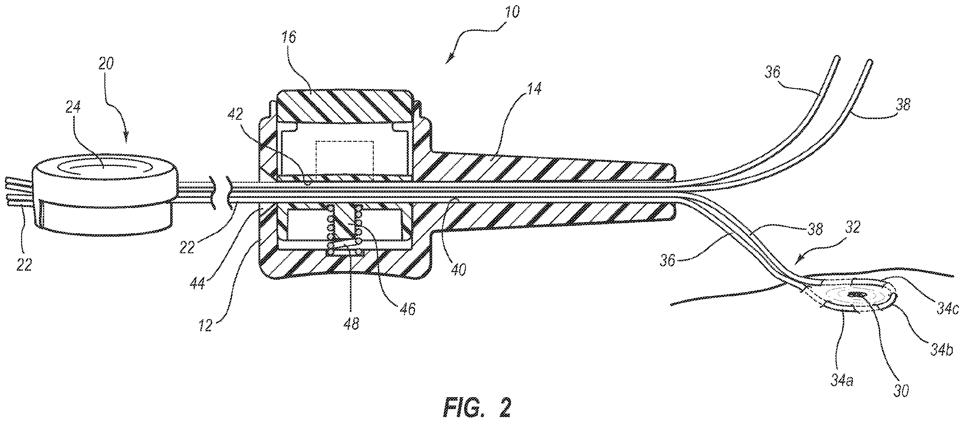

To utilize suture securement apparatus 10, a user simply threads the first tail 36 and second tail 38 through suture loop 22. Once the first tail 36 and second tail 38 are threaded through suture loop 22, the user grasps clasp 24 and begins to draw suture loop 22 in a proximal direction. As suture loop 22 is retracted in a proximal direction it begins to be drawn into extension tube 14. As suture loop 22 is drawn into extension tube, first tail 36 and second tail 38 are also drawn into extension tube 14. In this manner, complicated or cumbersome introduction of the tips of first tail 36 and second tail 38 into the somewhat small diameter of extension tube 14 is unnecessary. A more complete description of the manner in which suture loop 22 is utilized to draw first tail 36 and second tail 38 of the suture 32 into suture securement apparatus 10 will be described below with reference to Fig. 2.

As will be appreciate by those skilled in the art, a variety of types and configurations of the suture securement apparatus and threading assembly can be utilized without departing from the scope of the present invention. According to one embodiment of the present disclosure, the suture securement apparatus and threading assembly are provided as a unified component. According to another embodiment of the present disclosure, the suture securement apparatus and threading assembly are separate components that can be utilized alone or in combination. According to another embodiment of the present disclosure, the suture securement apparatus can be utilized with a suture configuration other than a purse string suture.

Fig. 2 is a cross-sectional side view of suture securement apparatus 10 according to one embodiment of the present disclosure. In the illustrated embodiment, suture securement apparatus 10 illustrates the use of threading assembly 20 to draw the first tail 36 and second tail 38 through suture securement apparatus 10. In the illustrated embodiment, a practitioner has grasped clasp 24 and is pulling clasp 24 in a rearward direction. First tail 36 and second tail 38 have been threaded through suture loop 22. As a result, when clasp 24 is retracted in a rearward direction, suture loop 22 is drawn into an extension tube lumen 40 of extension tube 14. Because first tail 36 and second tail 38 are threaded through suture loop 22, retraction of suture loop 22 pulls first tail 36 and second tail 38 into extension tube lumen 40.

As the practitioner continues to pull clasp 24 and suture loop 22 in a rearward direction, the end of suture loop 22 is drawn into a release button lumen 42. As a result, first tail 36 and second tail 38 are drawn into release button lumen 42. As will be appreciated by those skilled in the art, as the practitioner continues to retract threading assembly 20 in a rearward direction, the first tail 36 and second tail 38 are pulled along the entire length of suture securement apparatus until first tail 36 and second tail 38 extend out of a rear aperture 44 and from a proximal portion of suture securement apparatus 10. In this manner, threading assembly 20 facilitates the simple and quick threading of the first tail 36 and second tail 38 of suture 32 through suture securement apparatus 10.

In the illustrated embodiment, release button 16 includes a post 46 and a spring member 48. Spring member 48 is positioned adjacent post 46 such that spring member 48 exerts a biasing force urging release button 16 in an upward direction. When spring member 48 has urged post 46 in an upward direction such that release button 16 is at its upward most displacement relative to body 12, release button lumen 42 is placed slightly out of alignment with extension tube lumen 40. In this manner, first tail 36 and second tail 38 of suture 32 are compressed between the bottom surface of release button lumen 42 and the top surface of extension tube lumen 40. Similarly, first tail 36 and second tail 38 of suture 32 are compressed between the bottom surface of release button lumen 42 and the top surface of rear aperture 44. The cooperative engagement of the surfaces of extension tube lumen 40, release button 42, and rear aperture 44 provide an effective mechanism for securing a desired degree of tension on first and second tails 36 and 38 of suture 32 positioned along the length of suture securement apparatus 10.

When a practitioner desires to loosen the tension on first tail 36 and second tail 38 of suture 32, the practitioner simply depresses release button 16. As the practitioner depresses release button 16, the release button 16 is urged in a downward direction. As the release button 16 is urged in a downward direction, spring member 48 is depressed. Additionally, as the release button 16 is urged in a downward direction, the release button lumen 42 becomes aligned with extension tube lumen 40 and rear aperture 44. As a result, first tail 36 and second tail 38 of suture 32 are no longer cooperatively engaged between opposing surfaces of release button lumen 42, extension lumen 40, and rear aperture 44. This allows the practitioner to move suture securement apparatus laterally relative to first tail 36 and second tail 38. Releasing of this securement of suture 32 by depression of release button 16 allows the practitioner to loosen, tighten, or make other changes in the juxtaposition of suture securement apparatus 10 relative to suture 32.

In the illustrated embodiment, rear aperture 44 is positioned in substantial alignment with extension tube lumen 40. Additionally, rear aperture 44 has a tapered configuration which facilitates the loading of suture loop 22 along the length of suture securement apparatus 10. According to one embodiment of the present disclosure, a tapered extension tube is provided in place of rear aperture 44.

As will be appreciated by those skilled in the art, a variety of types and configurations of suture securement apparatus can be utilized without departing from the scope of the invention. For example, in one embodiment, the extension tube has a tapered distal aperture to facilitate loading of sutures into the suture securement apparatus. According to another embodiment of the present disclosure, the suture securement apparatus is provided without a threading assembly. According to yet another embodiment of the present disclosure, the juxtaposition and mechanism utilized to secure a suture relative to the suture securement apparatus other than a release button is utilized.

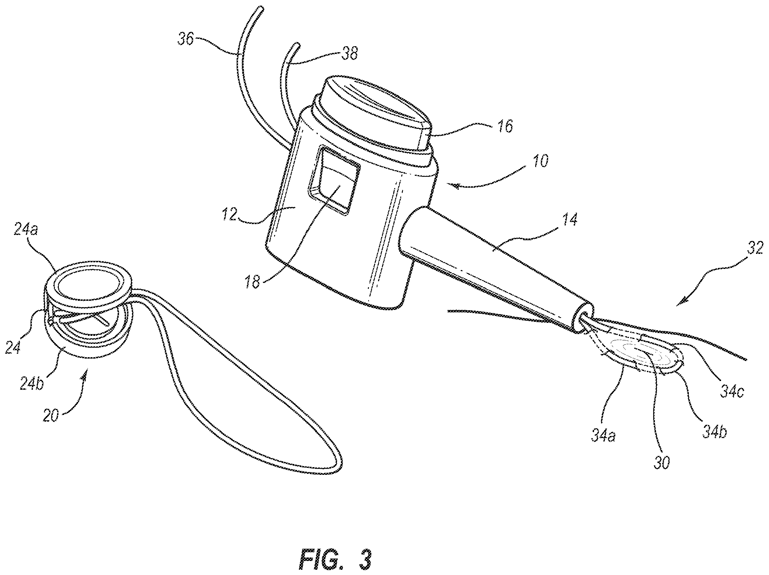

Fig. 3 is a perspective view of a suture securement apparatus 10 according to one embodiment of the present disclosure. In the illustrated embodiment, first tail 36, and second tail 38 have been threaded along the entire length of suture securement apparatus 10 such that first tail 36 and second tail 38 are extending out through rear aperture 44 (see Fig. 2). Suture securement apparatus 10 has been advanced forward such that the tip of extension tube 14 is positioned adjacent the insertion sites of first tail 36 and second tail 38 into the patient. In other words, first tail 36 and second tail 38 have been pulled tight relative to suture securement apparatus 10 such that a desire degree of tension is exerted on the purse string suture segments 34a, b, c. As a result, puncture site 30 is effectively closed and hemostasis at the puncture site 30 is effectuated.

In the illustrated environment, suture loop 22 has been fully withdrawn from suture securement apparatus 10. Additionally, clasp 24 is shown in an open configuration to illustrate the mechanism by which clasp 24 secures the configuration of suture loop 22. In the illustrated embodiment, clasp 24 has a clam shell configuration. Clasp 24 comprises a first clam shell component 24a and a second clam shell component 24b. The underlying surfaces of clam shell 24a and clam shell 24b have a profile which facilitates closing and gripping of the suture from which suture loop 22 is formed. Additionally, when the first clam shell component 24a and second clam shell component 24b are pressed together, a snap fit or other engagement mechanism is effectuated which secures the closed configuration of clasp 24.

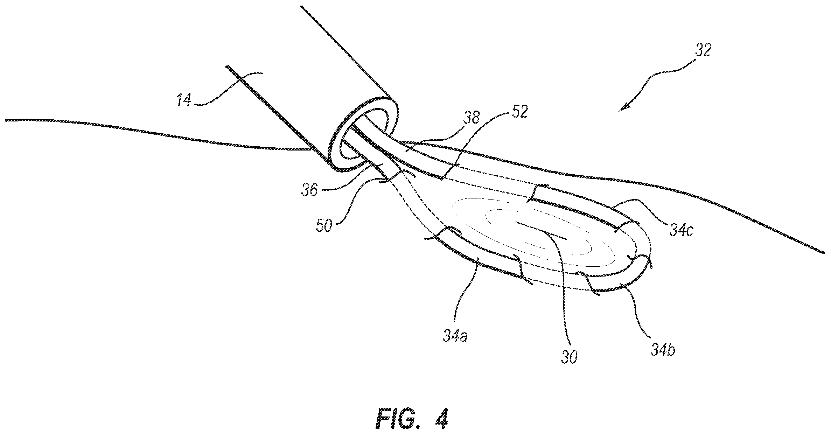

Fig. 4 is a close up perspective view of the tip of extension tube 14 relative to the purse string suture configuration of suture 32 positioned around puncture site 30. In the illustrated embodiment, first tail 36 and second tail 38 are threaded into extension tube lumen 40. It can be seen that the diameter of extension tube lumen 40 at the tip of extension tube 14 is smaller than the distance between the primary suture insertion point 50 at which first tail 36 is inserted into the patient and the secondary suture emergence point 52 at which second tail emerges from the patient. By having a diameter of extension tube lumen 40 that is smaller than the distance between primary suture insertion point 50 and secondary suture emergence point 52, tension exerted on first tail 36 and second tail 38 draws first tail 36 and second tail 38 together at the extension tube lumen 40. By drawing the first tail 36 and second tail 38 together, desired tensioning of the entire purse string suture along the perimeter of puncture site 30 is effectuated. This helps to minimize puckering or other distortion of the skin surrounding puncture site 30. As a result, desired closure of the puncture site 30 is effectuated and inadvertent damage from improper healing of the puncture site 30 is avoided.

As will be appreciated by those skilled in the art, a variety of types and configurations of securing a suture relative to the suture securement apparatus 10 can be utilized without departing from the scope of the present invention. For example, in one embodiment, only the very tip of the extension tube lumen 40 has a smaller diameter than the distance between the primary suture insertion point 50 and the secondary suture emergence point 52. According to another embodiment of the present disclosure, the entire length of the extension tube lumen 40 has a smaller diameter than the distance between the primary suture insertion point 50 and the secondary suture emergence point 52. According to yet another embodiment of the present disclosure, a method of tying or wrapping the first and second tails of the suture can be utilized. According to yet another embodiment of the present disclosure, the suture securement apparatus 10 is utilized with a suture arrangement other than a purse string suture.

As will be understood by those skilled in the art, utilization of the suture securement apparatus with the purse string suture is merely an exemplary embodiment illustrating operation of the suture securement apparatus relative to sutures and should in no means be considered to be limiting in nature. For example, the suture securement apparatus can be utilized with sutures that are not affixed to the skin of the patient. Additionally, the suture securement apparatus can be utilized for securing additional medical devices such as a suture ring of a catheter hub to a patient. According to another embodiment, the suture securement apparatus is utilized with sutures which are not utilized at a catheter insertion point.

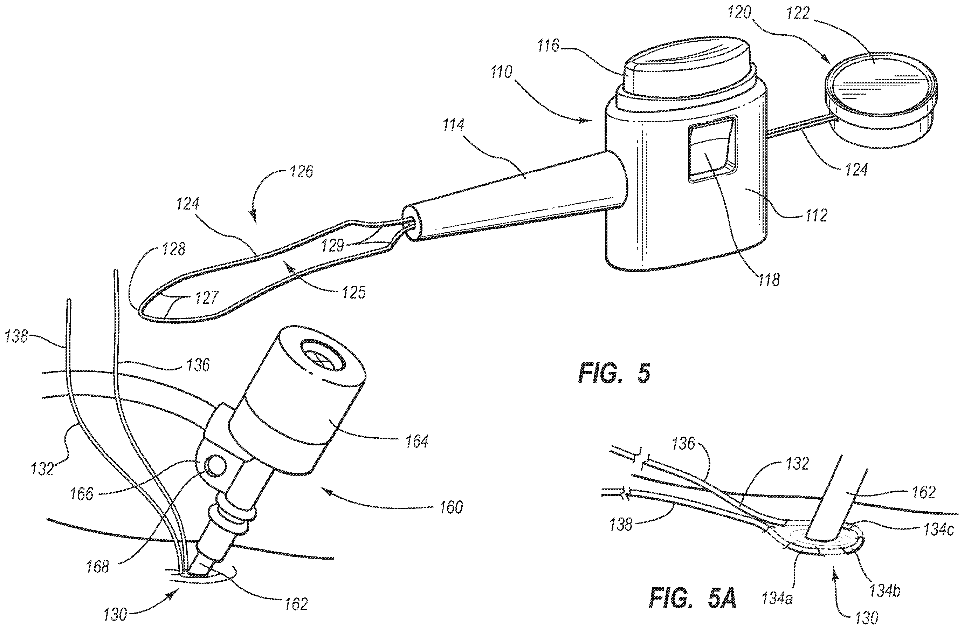

Fig. 5 is a perspective view of a suture securement apparatus according to one embodiment of the present disclosure. In the illustrated embodiment, suture securement apparatus 110 can be utilized to selectively and releasably secure the ends or tails 136, 138 of a suture utilized in various securement procedures. The suture is configured in a purse string suture configuration (as best depicted in Fig. 5A) to maintain hemostasis and close a percutaneous catheter insertion site 130. In the illustrated embodiment, suture securement apparatus 110 comprises a body 112, an extension tube 114, a release button 116, and an assembly window 118. Body 112 comprises a housing for securing the other components of suture securement apparatus 110 to maintain desired operability of the components of suture securement apparatus 110. An extension tube 114 extends distally from body 112. Extension tube 114 provides a mechanism for engaging suture 132 positioned adjacent insertion site 130 without interruption or interference from body 112 of suture securement apparatus 110.

Release button 116 is positioned within a cavity of body 112 of suture securement apparatus 110. Release button 116 allows a practitioner to selectively secure or release sutures positioned within suture securement apparatus 110. For example, in the illustrated embodiment when release button 116 is in a released position, suture securement apparatus 110 is securely gripping any sutures positioned within suture securement apparatus 110. When the user depresses release button 116, the grip exerted by suture securement apparatus 110 on any sutures positioned within suture securement apparatus 110 is loosened, and manipulation or adjustment of the suture securement apparatus 110 relative to the sutures can be effectuated. The ability to selectively release and reposition the suture securement apparatus 110 relative to sutures for which the suture securement apparatus 110 is to be utilized can be desirable to the extent the practitioner desires to adjust the tension, reposition suture securement apparatus 110 relative to the patient, or perform other desired activities relative to the sutures and/or suture securement apparatus 110. Assembly window 118 allows for the quick snap fit assembly of the release button 116 relative to body 112 of suture securement apparatus 110.

As illustrated in Fig. 5, suture securement apparatus 110 is utilized with a threading assembly 120. Threading assembly 120 allows a practitioner to quickly and simply thread one or more tails 136, 138 of suture 132 from the exterior of suture securement apparatus 110, through a lumen of suture securement apparatus 110 such that desired engagement of tails 136, 138 of suture 132 is effectuated. In the illustrated embodiment, threading assembly 120 comprises a clasp 122 and a wire 124. Clasp 122 is utilized to retain wire 124 in its desired position to prevent inadvertent advancement of the end of the wire 124 into suture securement apparatus 110.

Wire 124 is configured to form an insertion assembly 126. Insertion assembly 126 can comprise a pointed tip 128, an opening 125, and/or one or more angular portions 127 to allow insertion assembly 126 to be inserted through a narrow aperture, such as the lumen of suture securement apparatus 110. The pointed tip 128 and/or angular portions 127 allow for one or more sutures to be threaded through the opening provided by insertion assembly 126 and then drawn backward through the narrow aperture. In the illustrated embodiment, the insertion assembly 126 also includes rear angular portions 129 at the rear portion of the insertion assembly 126, proximal to extension tube 114 when insertion assembly 126 is fully inserted through the lumen of suture securement apparatus 110. The angular portions 127, 129 of the insertion assembly 126 are formed by pre-shaped bends in the wire 124 of the insertion assembly 126. The configuration of the pre-shaped bends in insertion assembly 126 allow for bending or pushing together of the opposing sides of the insertion assembly 126 as the insertion assembly 126 is drawn or pushed through narrow apertures. As a result, insertion assembly 126 can be easily threaded through a narrow aperture, such as the lumen of suture securement apparatus 110, and the insertion assembly 126 can readily be retracted back through the aperture.

In the embodiment of Fig. 5, a suture 132 is utilized in connection with a percutaneous catheter insertion site 130. A percutaneous drainage catheter 160 is inserted at the insertion site 130. Fig. 5A is a close-up view of insertion site 130 showing a dilator tube 162 of percutaneous drainage catheter 160 inserted therein. Suture 132 is configured as a purse string suture. As a result, a plurality of purse string segments 134a, b, c are depicted being positioned around the perimeter of insertion site 130. A first tail 136 is shown extending away from insertion site 130. A second tail 138 is also depicted as extending away from insertion site 130. First tail 136 and second tail 138 are positioned adjacent to one another but in sufficient spatial relationship so as to allow the drawing of first tail 136 closer to second tail 138 to achieve a desired degree of tension on purse string segments 134a, b, c.

In the illustrated embodiment, a user can utilize suture securement apparatus 110 to achieve a desired degree of tension on the purse string configuration of suture 132. To utilize suture securement apparatus 110, a user simply threads the first tail 136 and second tail 138 through opening 125 of insertion assembly 126 formed by wire 124. Once the first tail 136 and second tail 138 are threaded through opening 125 of insertion assembly 126, the user grasps clasp 122 of threading assembly 120 and begins to draw first tail 136 and second tail 138 into extension tube 114. In this manner, complicated or cumbersome introduction of the tips of first tail 136 and second tail 138 into the relatively small diameter of the lumen of extension tube 114 is eliminated. As will be appreciated by those skilled in the art, a variety of suture configurations can be utilized in connection with a variety of types and configurations of puncture wounds and insertion sites. The round or substantially circular wound of percutaneous catheter insertion site 130 as depicted is merely one example.

With continued reference to Fig. 5, drainage catheter 160 includes dilator tube 162, a hub 164 and suture ring 166. Dilator tube 162 of drainage catheter 160 is configured to be percutaneously inserted into the vasculature of a patient, or otherwise positioned within a patient's body. In some situations a practitioner may desire to secure the drainage catheter 160 relative to the patient to minimize the inadvertent movement or accidental full or partial removal of the drainage catheter 160 from its placement within the patient. For example, a drainage catheter 160 may be inserted into the patient with the intention to leave it in place for a significant period of time for repeated use during subsequent procedures.

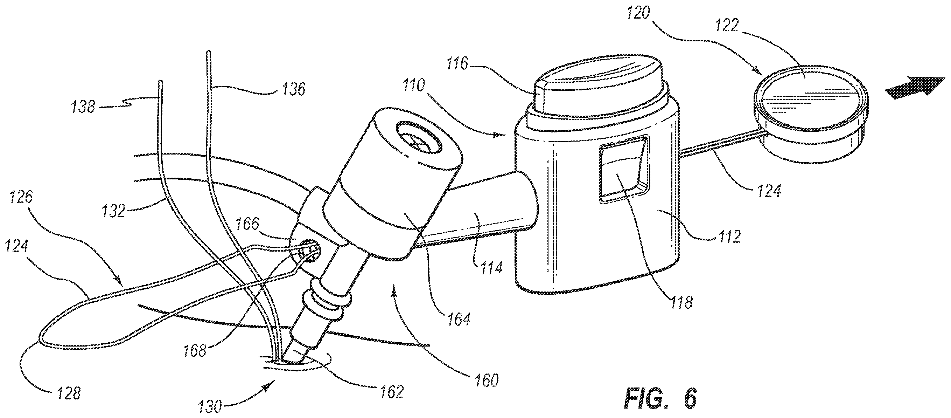

Suture ring 166 is provided adjacent hub 164 to enable the desired securement of the drainage catheter 160 relative to the body of the patient. Suture ring 166 includes a suture ring aperture 168. Traditionally, suture ring 166 is sutured or tied directly to the body of the patient to secure drainage catheter 160 relative to the patient's body. In the illustrated embodiment, a suture 132 having a first tail 136 and a second tail 138 are threaded into the skin or otherwise secured to the body of the patient. Suture securement apparatus 110 allows for the quick and efficient securement of the suture ring 166 and drainage catheter 160 to the patient, as depicted in Fig. 6 and describe in greater detail below with reference to the same.

As will be appreciated by those skilled in the art, a variety of configurations of threading assembly 120 can be utilized in connection with the suture securement apparatus 110 without departing from the scope of the present invention. According to one aspect of the present disclosure, the threading assembly utilizes a hook configuration rather than an insertion assembly 126. According to another embodiment of the present disclosure, the insertion assembly 126 includes only one or two preformed bends rather than a plurality of preformed bends. According to another embodiment of the present disclosure, the threading assembly 120 is operable from the distal portion of the suture securement apparatus 110 rather than from the proximal portion of the suture securement apparatus 110.

Fig. 6 illustrates utilization of threading assembly 120 to thread first tail 136 and second tail 138 of suture 132 through suture ring aperture 168 and through suture securement apparatus 110. In the illustrated embodiment, insertion assembly 126 is pushed through suture ring aperture 168 of suture ring 166. The first tail 136 and second tail 138 are then threaded through insertion assembly 126. The shape of insertion assembly 126 facilitates the passage or threading of first tail 136 and second tail 138 through insertion assembly 126 in a relatively straight forward and advantageous manner.

Once first tail 136 and second tail 138 have been threaded through insertion assembly 126, the practitioner can retract threading assembly 120 from suture securement apparatus 110. As the user begins to retract threading assembly 120 from suture securement apparatus 110, insertion assembly 126 is pulled through suture ring aperture 168. As insertion assembly 126 is pulled back through suture ring aperture 168, the preformed bends undergo deformation and the sides of insertion assembly 126 are biased together urging the two different sides of insertion assembly 126 together. This allows for insertion assembly 126 to easily be withdrawn through suture ring aperture 168.

As insertion assembly 126 is withdrawn through suture ring aperture 168, first tail 136 and second tail 138 are also pulled through suture ring aperture 168. In this manner, the practitioner does not have to engage in the sometimes tedious and often inconvenient process of threading the tips of first tail 136 and second tail 138 through suture ring 166. As the user continues to withdraw threading assembly 120 from suture securement apparatus 110, insertion assembly 126 is drawn into extension tube 14, along the length of suture securement apparatus 110, and out through the proximal aperture (not shown) of suture securement apparatus 110. In this manner, threading assembly 120 facilitates not only the drawing of first tail 136 and second tail 138 through suture ring 166, but also facilitates the drawing of first tail 136 and second tail 138 through suture securement apparatus 110.

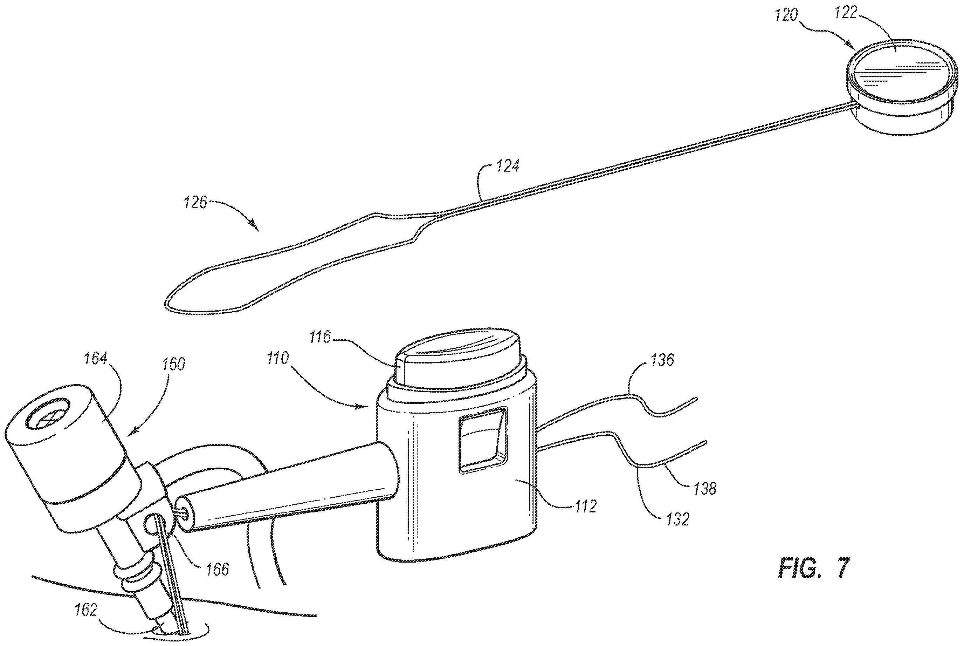

Fig. 7 illustrates suture securement apparatus 110 after first tail 136 and second tail 138 have been threaded along the length of suture securement apparatus 110. Fig. 7 also illustrates threading assembly 120 once it has been fully withdrawn from suture securement apparatus 110. In the illustrated embodiment, once first tail 136 and second tail 138 have been fully drawn through suture securement apparatus 110, the practitioner can grasp the free ends of first tail 136 and second tail 138 and exert tension on suture 132. To exert tension on suture 132, the practitioner simply depresses release buttons 16 while firmly grasping first tail 136 and second tail 138 and then urges suture securement apparatus 110 in the direction of suture ring 166 of drainage catheter hub 164. By utilizing an adjustable suture securement apparatus 110, the practitioner can increase or decrease the amount of tension of suture 132 and thus the amount of tension holding suture ring 166 relative to the body of the patient. In this manner, drainage catheter 160 is secured relative to the patient and dilator tube 162 is secured in position within the patient.

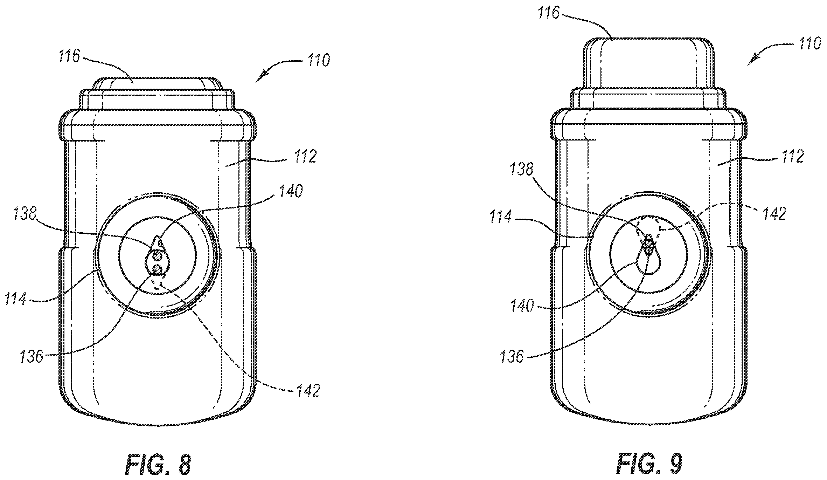

Figs. 8 and 9 illustrate an end view of suture securement apparatus 110 according to one aspect of the present disclosure. In the illustrated embodiment, extension tube 114 is positioned such that a user can look down the extension tube lumen 140. At least a portion of extension tube lumen 140 has a tear drop configuration having a larger or bulbous portion and a smaller width or pointed portion. Additionally, at least a portion of release button lumen 142 has a tear drop configuration, similarly having a bulbous portion and a pointed portion. When release button 116 is fully depressed as depicted in Fig. 8, the bulbous portions of the tear drops are aligned providing a desired larger circumference for accommodating first tail 136 and second tail 138 or also allowing for desired movement of first tail 136 and second tail 138 relative to suture securement apparatus 110.

As the user begins to release the pressure on release button 116, release button 116 moves upward relative to body 112 of suture securement apparatus 110. As a result, the release button lumen 142 begins to change its orientation relative to extension tube lumen 140. As a result, the larger or bulbous portion of the tear drop shape of the extension tube lumen 140 and release button lumen 142 move out of alignment and the pointed portion of the tear drops of extension tube lumen 140 and release button lumen 142 move into alignment as depicted in Fig. 9. This causes cooperative engagement of one or both of first tail 136 and second tail 138 between the pointed portions of the tear dropped configuration of extension tube lumen 140 and release button lumen 142.

The cooperative engagement of first tail 136 and second tail 138 relative to extension tube lumen 140 and release button lumen 142 provides a secure and desired gripping function which allows the practitioner to provide varying degrees of tensioning on first tail 136 and second tail 138. As will be appreciated by those skilled in the art, a variety of types and configurations of lumen cross sections can be provided in connection with extension tube lumen 140 and release button lumen 142 without departing from the scope of the present invention. For example, according to one embodiment of the present disclosure, extension tube lumen 140 and release button lumen 142 have a circular, triangular, curvilinear or elliptical shape. According to another embodiment of the present disclosure, the cross section of all or a portion of extension tube lumen 140 and release button lumen 142 are different from one another.

When a user desires to reposition or fully release first tail 136 and second tail 138, the user simply depresses release button 16 as depicted in Fig. 8. The user then can move suture securement apparatus 110 relative to first tail 136 and second tail 138. For example, the user can depress release button 16 and fully withdraw first tail 136 and second tail 138, for example, by pulling the suture securement apparatus 110 in a rearward direction until first tail 136 and second tail 138 are fully withdrawn from release button lumen 142 and extension tube lumen 140. Alternatively, the user can push release button 16 and draw the free ends of first tail 136 and second tail 138 in a rearward direction to increase the tension exerted on first tail 136 and second tail 138.

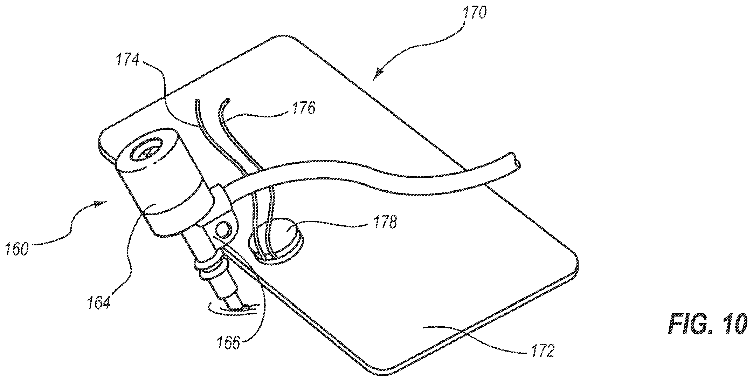

Fig. 10 is a perspective view of an adhesive suture securement device 170 according to one embodiment of the present disclosure. In the illustrated embodiment, adhesive suture securement device 170 comprises an adhesive base 172, a first suture 174, a second suture 176, and an anchor 178. Adhesive suture securement device 170 is utilized to secure sutures 174, 176 relative to a patient's body without requiring puncturing of the patient's skin to secure the sutures 174, 176 relative to the patient. By utilizing adhesive suture securement device 170, the practitioner can quickly and efficiently secure sutures 174, 176 on a patient's skin which can then be utilized for other aspects of the procedure to be performed.

In the illustrated embodiment, adhesive suture securement device 170 is being utilized in connection with a drainage catheter 160. Once the adhesive suture securement device 170 is secured on the skin of the patient, the sutures can then be readily secured to a suture ring 166 of hub 164 of drainage catheter 160. This can effectively secure drainage catheter 160 in a desired placement position relative to the patient. As will be appreciated by those skilled in the art, a single suture or a plurality of sutures comprising more than two sutures can be provided in connection with adhesive suture securement device 170.

In the illustrated embodiment, adhesive base 172 comprises a flexible or inflexible adhesive layer or pad which can be affixed directly to the skin of the patient by utilizing adhesive base 172. Adhesive suture securement device 170 can quickly and easily be placed in a desired position on the patient's skin without requiring a needle prick or other suturing into the patient's skin. By providing a sharpless suture securement solution, patients whose skin tissue is thin or may otherwise be easily damaged, or that may have a fear of needles, can have a suture readily positioned on their skin. Additionally, a sharpless suture securement device provides a mechanism to provide sutures at a percutaneous access site without the drawbacks of a skin puncture that may require healing or that may cause unnecessary concern or fear in the patient.

In the illustrated embodiment, first suture 174 and second suture 176 are secured to adhesive base 172 utilizing an anchor 178. Anchor 178 provides a secure anchoring of first suture 174 and second suture 176 relative to adhesive base 172. In this manner, any pulling or excessive tension exerted on first suture 174 and/or second suture 176 will not result in tearing or the accidental separation of one or more of first suture 174 and second suture 176 from adhesive base 172. In the illustrated embodiment anchor 178 comprises a clam shell type configuration which securely engages one or both first suture 174 and second suture 176 when opposing clam shell type faces of anchor 178 are snapped together. According to one embodiment of the present disclosure, first suture 174 and second suture 176 do not comprise first and second sutures but rather are opposing end of a continuous suture thread which is secured in the middle of the thread with the opposing tail ends being free such that they can be secured to a suture ring 166 of the drainage catheter 160.

As will be appreciated by those skilled in the art, a variety of types and configurations of anchors can be utilized with adhesive suture securement device 170 without departing from the scope of the present disclosure. According to one embodiment of the present disclosure, anchor 178 has a surface contact area with adhesive base 172 that is greater than four times the total contact area provided between the suture 170 and the adhesive base 172. According to another embodiment of the present disclosure, anchor 178 secures adhesive base 172 and at least a first suture 174 by sandwiching both adhesive base 172 and the first suture 174 between opposing faces of the anchor 178. According to an alternative embodiment of the present disclosure, the anchor 178 is positioned only on either the upper face surface or the under surface of the adhesive base 172.

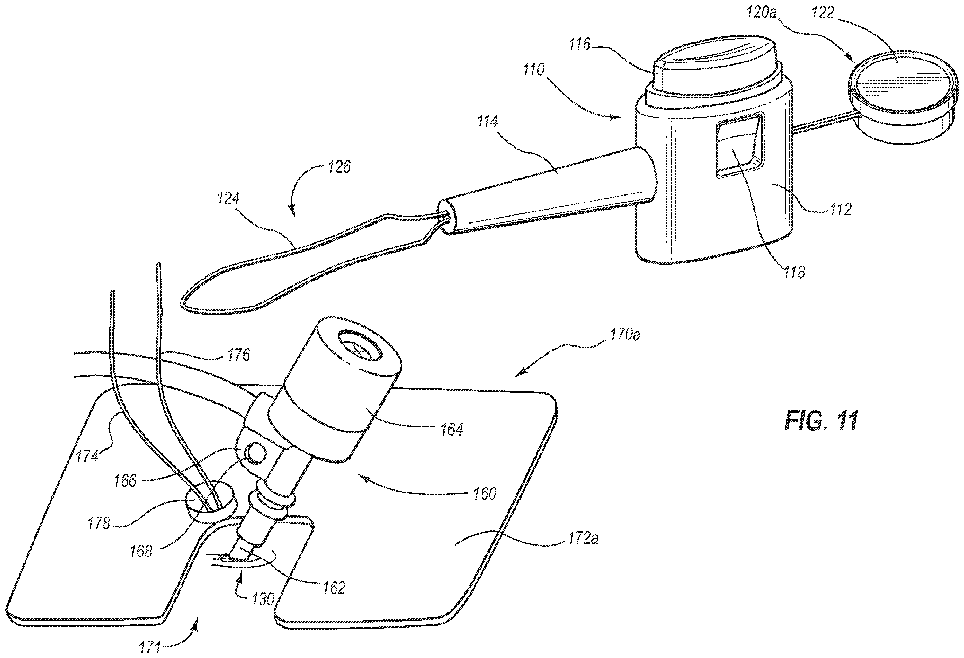

Fig. 11 is a perspective view of an adhesive suture securement device 170a according to another embodiment of the present disclosure. In the illustrated embodiment, adhesive base 172a of adhesive suture securement device 170a has a void 171 which accommodates an apparatus or other securement target while allowing the adhesive base 172a to be positioned around the insertion site 130 of the apparatus or securement target. In other words, void 171 permits adhesive base 172a to be positioned laterally adjacent on two or more lateral sides of dilator tube 162. In the illustrated embodiment, the adhesive suture securement device 170a is utilized in connection with a drainage catheter 160. Additionally in the illustrated embodiment, the adhesive base 172a is formed in a horseshoe-type or semi-circular configuration to define void 171. Anchor 178, first suture 174, and second suture 176 are positioned adjacent void 171 such that any tension on one or both of first suture 174 and second suture 176 are related to a desired position on adhesive base 172a. In the illustrated embodiment, anchor 178, first suture 174 and second suture 176 are positioned approximately at the center or a mid-point of adhesive base 172a such that lateral or other movement of drainage catheter 160 is well secured by adhesive base 172a and other components of adhesive suture securement apparatus 170a.

In the illustrated embodiment, a suture securement apparatus 110 is depicted. Suture securement apparatus 110 is similar to the suture securement apparatus 110 depicted in Figs. 5, 6 and 7. Suture securement apparatus 110 enables a practitioner to quickly thread first suture 174 and second suture 176 through the suture ring aperture 168 or suture ring 166 of drainage catheter 160 and then to releasably and adjustably secure the first suture 174 and second suture 176 relative to a medical apparatus, instrument, or other securement target.

As will be appreciated by those skilled in the art, adhesive suture securement device 170a can have a variety of shapes, configurations, uses and other modifications which will enable desired anchoring of the suture relative to a patient. For example, according to one embodiment of the present disclosure, a central hole is provided as an operative void rather than a partial semi-circular void 171 depicted in Fig. 11. This circular void or hole in the middle of the adhesive base can then be utilized to secure the sutures relative to the patient allowing for the insertion or securement of a medical apparatus position through the patient's skin at an insertion site within the particular void. According to another embodiment a contour, curve or other pad design is provided to allow for desired positioning of the pad relative to a catheter but or other apparatus to be secured.

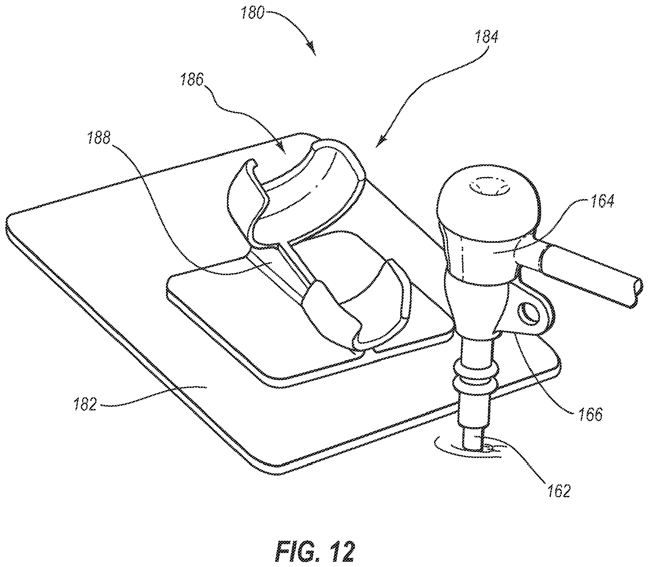

Fig. 12 is a perspective view of an adhesive securement device 180 having a basket securement member 184 for securing a medical instrument, according to one embodiment of the present disclosure. Basket securement member 184 comprises a cradle 186, which is secured to an adhesive base 182 utilizing a base 188. Adhesive base 182 is utilized to secure basket securement member 184 relative to a patient. The advantage of utilizing adhesive base 182 is similar to that described with reference to the adhesive base 172 of adhesive suture securement device 170, of Fig. 10, and base 172a of adhesive suture securement device 17a, of Fig. 11. More specifically, a practitioner can secure basket securement member 184 relative to a patient without puncturing the patient's skin.

Cradle 186 of basket securement member 184 is secured to adhesive base 182 utilizing a base 188. Cradle 186 is configured to receive the medical instrument or other securement target while base 188 is utilized to secure cradle 186 relative to adhesive base 182. Cradle 186 is configured to secure and maintain the position of a medical instrument relative to the patient on which adhesive securement device 180 is secured. For example, in the illustrated embodiment, a drainage catheter hub 164 can be positioned within cradle 186 of basket securement member 184. Cradle 186 of basket securement member 184 is shaped to conform to the dimensions and relief surface of hub 164 such that when a user snaps or urges hub 164 into cradle 186, inadvertent movement of hub 164 relative to the patient and relative to the components of adhesive securement device 180 is minimized. According to one embodiment of the present disclosure, cradle 186 also minimizes rotational movement of hub 164 such that the position of a suture ring 166 relative to cradle 186 is maintained. As will be appreciated by those skilled in the art, the shape of cradle 186 can be modified to the type of medical apparatus or securement target with which basket securement member 184 is to be utilized.

As will be appreciated by those skilled in the art, a variety of types and configurations of basket securement members 184 can be utilized without departing from the scope of the present disclosure. For example, according to one embodiment of the present disclosure, the basket securement member 184 includes a top clam shell or lid portion which facilitates securement of the securement target relative to the other components of adhesive securement device 180. According to another embodiment of the present disclosure, an adhesive overlay is provided which secures the components of adhesive securement device 180.

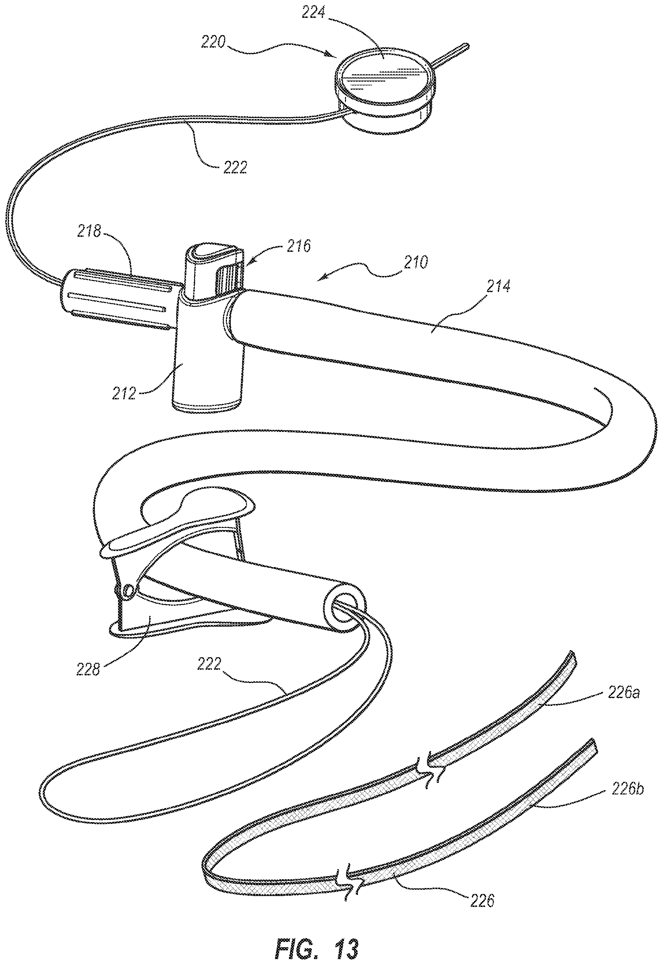

Fig. 13 is a perspective view of a surgical securement apparatus 210 according to one embodiment of the present disclosure. Surgical securement apparatus 210 comprises a body 212, an extender tube 214, a release button 216, and a finger grip tube 218. Surgical securement apparatus 210 can be used to selectively and releasable secure a securement cord 226, which is adapted to secure one or more intracorporeal structures during a surgical procedure. Securement cord 226 is threaded around one or more intracorporeal structures within the surgical site and is then threaded through surgical securement apparatus 210. The length of extender tube 214 of surgical securement apparatus 210 allows the distal end of extender tube 214 to be positioned adjacent to one or more intracorporeal structures within the surgical site while body 212 remains outside the surgical site. The distal end of the extender tube 214 is adapted to engage the securement cord adjacent to the one or more intracorporeal structures. Positioning extender tube 214 adjacent to the one or more intracorporeal structures allows securement cord 226 to be drawn taut around the one or more intracorporeal structures. A threading assembly 220 can also be provided to facilitate threading the securement cord 226 through the surgical securement apparatus 210. Threading assembly 220 can comprise a threading loop 222 and a clasp 224. An extender tube trimming device 228 can also be provided to enable the practitioner to quickly and conveniently adjust the length of extender tube 214.

Body 212 comprises a housing to secure other components of surgical securement apparatus 210 that are used to grip securement cord 226. In the illustrated embodiment, body 212 has a substantially oblong cross-section in the shape of an ellipse. Body 212 forms a cavity to receive and partially enclose release button 216. The cavity and release button both have an oblong cross-section substantially in the shape of an ellipse corresponding to the elliptical cross-section of body 212. As illustrated in Fig. 13, body 212 is oriented such that a cord threaded through body 212 and release button 216 extends substantially along the minor axis of the elliptical cross-section. This orientation provides a larger surface area, allowing a larger through hole through which a cord can be threaded. Accordingly, cords of larger thickness can be threaded through surgical securement apparatus 210. In addition to securing the components of the surgical securement apparatus 210, body 212 is also weighted so as to provide appropriate tension, such that the surgical securement apparatus can hang outside the surgical site without causing unnecessary strain on the intracorporeal structures being secured.

Release button 216 is positioned within body 212 of surgical securement apparatus 210. Release button 216 of the depicted embodiment has a shipping configuration and an activated configuration. In Fig. 13, release button 216 is depicted in an activated configuration. In the activated configuration, release button 216 is slidably moveable within the cavity of body 212. Accordingly, release button 216 in the activated configuration can be depressed against an internal biasing element to a depressed position or released to an undepressed position. Moreover, release button 216 in an activated configuration allows a practitioner to selectively secure or release a cord positioned within the surgical securement apparatus 210.

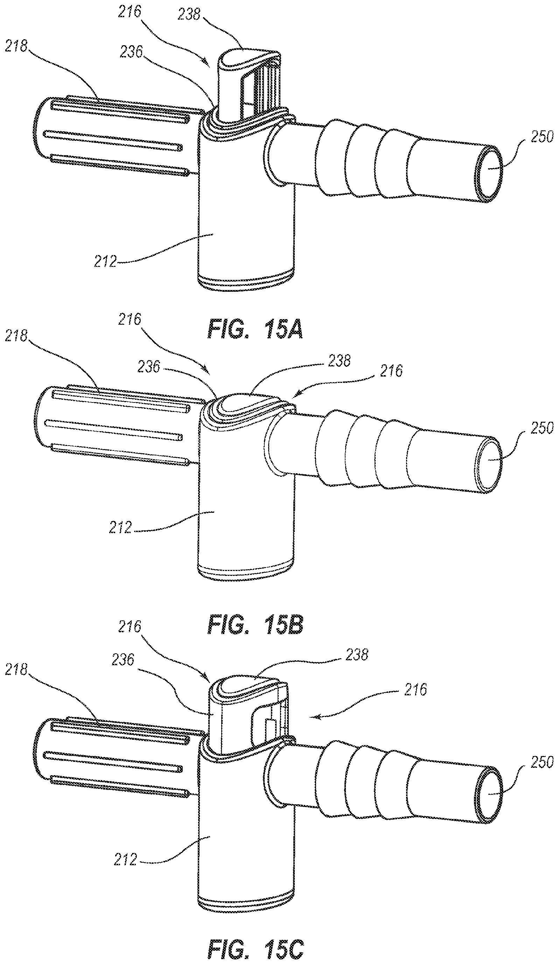

Selective securement of a cord by release button 216 is accomplished by depressing or releasing release button 216. In the illustrated embodiment, when release button 216 is in an undepressed position, surgical securement apparatus 210 grips a cord positioned therein. When the practitioner depresses release button 216, the grip of the surgical securement apparatus 210 on the cord is released and the practitioner can then manipulate the cord or adjust the surgical securement apparatus relative to the cord. The manner in which the cord is secured and released is discussed in greater detail below with reference to Figs. 15A-18C. The ability to selectively release and reposition the surgical securement apparatus 210 relative to a cord is desirable to the extent that the practitioner desires to adjust the tension of the cord, reposition the surgical securement apparatus relative to the patient, or perform other desired activities relative to the cord and/or surgical securement apparatus 210. Specifically, the practitioner can easily release the cord at the end of the surgical procedure and remove the cord prior to closing up the surgical site.

Extender tube 214 is coupled to body 212. Extender tube 214 comprises a resilient tubular member having a lumen formed therethrough. Extender tube 214 can be formed of PVC, silicone or other flexible material. Extender tube 214 extends distally from body 212 to access a surgical site. The length of extender tube 214 allows the distal end of extender tube 214 to be positioned within the surgical site while the proximal end, which is coupled to body 212, remains outside the surgical site. Securement cord 226 can be threaded in a loop or other configuration around the one or more intracorporeal structures within the surgical site. Then a first end 226a and a second end 226b of securement cord 226 can be threaded through extender tube 214 and through surgical securement apparatus 210. In this manner, securement cord 226 can be secured from outside the surgical site by surgical securement apparatus 210, thereby removing any potential for interference or interruption of the surgical site from body 212 of surgical securement apparatus 210.

Finger grip tube 218 is also coupled to body 212 in the illustrated embodiment. Finger grip tube 218 provides the practitioner an area to grip the surgical securement apparatus 210 without depressing release button 216. Finger grip tube 218 comprises a lumen (not depicted) through which a cord can be threaded. Finger grip tube 218 also can be configured to facilitate loading or pre-threading of threading assembly 220. For example, the lumen of finger grip tube 218 can be tapered to guide the threading assembly 220 into and through surgical securement apparatus 210.

A threading assembly 220 can be utilized in conjunction with the illustrated surgical securement apparatus 210. Threading assembly 220 facilitates threading a securement cord 226 through surgical securement apparatus 210. Threading assembly 220 comprises a threading loop 222 and a clasp 224. Threading loop 222 is threaded into the lumen of finger grip tube 218 and through surgical securement apparatus 210 such that threading loop 222 partially extends distally from the distal end of extender tube 214. First end 226a and second end 226b of securement cord 226 can be threaded through the portion of threading loop 222 that extends distally from extender tube 214. After the first end 226a and second end 226b are threaded through the distal portion of threading loop 222, a practitioner can pull threading assembly 220 through surgical securement apparatus 210, thereby pulling the first end 226a and second end 226b through surgical securement apparatus 210. The practitioner can grasp clasp 224 and retract threading assembly 220, drawing threading loop 222 in a proximal direction. As threading loop 222 is drawn in a proximal direction, threading loop 222 is drawn into extender tube 214. When threading loop 222 is drawn into extender tube 214, first tail 226a and second tail 226b of securement cord 226 are also drawn into extender tube 214. As the practitioner continues to retract threading assembly 220 in a proximal direction, threading loop 222 and the ends of securement cord 226 are pulled through the length of extender tube 214. Eventually threading assembly 220 is pulled through the entire length of surgical securement apparatus 210 positioning securement cord 226 such that it is threaded through the entire length of surgical securement apparatus 210.

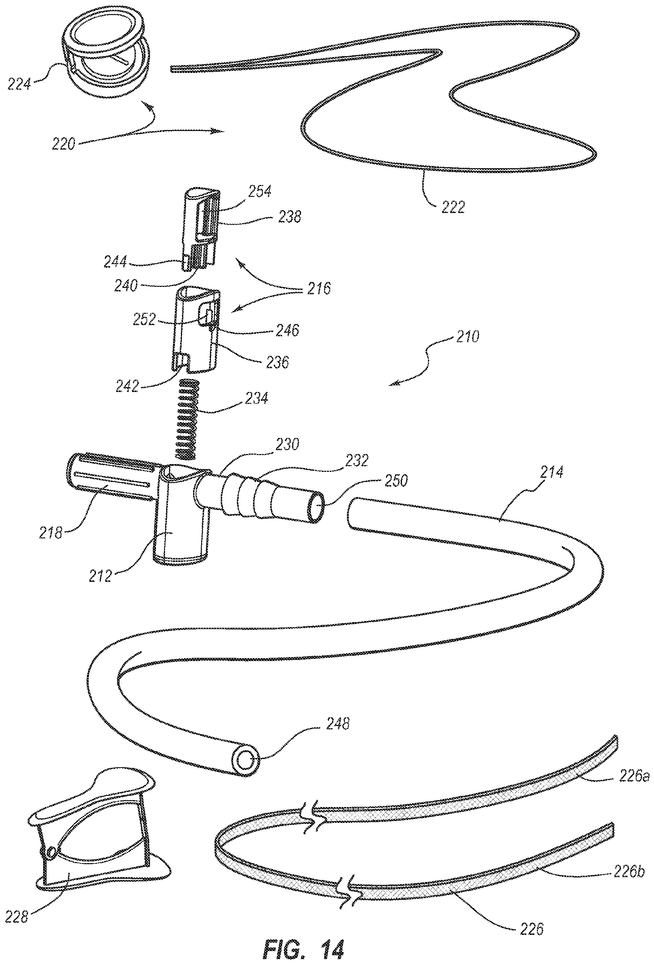

Fig. 14 is an exploded view of a surgical securement apparatus 210 of Fig. 13. In the illustrated embodiment, surgical securement apparatus 210 comprises body 212, extender tube 214, release button 216, and finger grip 218. Additionally, surgical securement apparatus 210 can be used in conjunction with a securement cord 226, a threading assembly 220, and an extender tube trimming device 228. The illustrated exploded view further illustrates that surgical securement apparatus 210 comprises a connector 230 to connect extender tube 214 to body 212. Connector 230 can further comprise a barbed connector tip 232 to facilitate coupling of extender tube 214 to connector 230. A biasing element 234 is positioned within a cavity of body 212 to bias release button 216 in an upward direction toward an undepressed state. In the illustrated embodiment, biasing element 234 comprises a spring. Additionally, in the illustrated embodiment, release button 216 comprises two components; a release button housing 236 and a release button actuator 238. Release button 216 is activated from the shipping configuration to the activated configuration by release button actuator 238.

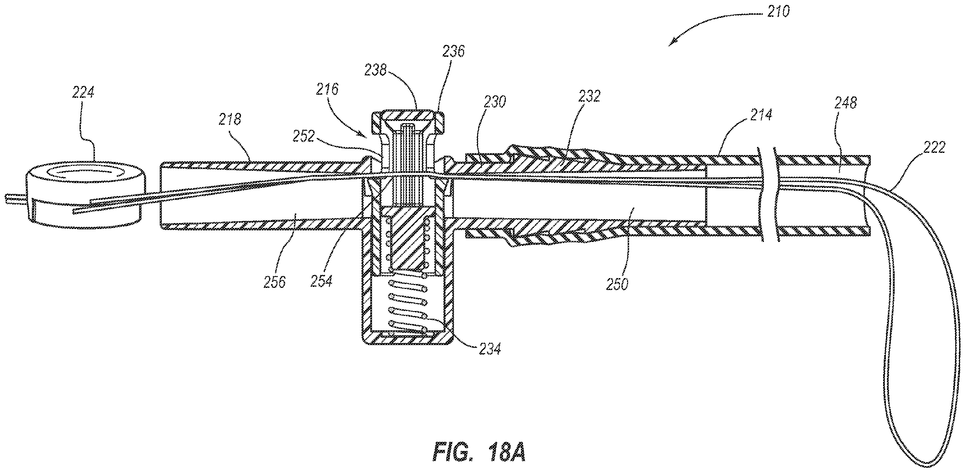

In the illustrated embodiment, connector 230 is coupled to or integrated with body 212. Connector 230 forms a connector lumen 250, which is adapted to provide a smooth transition from body 212 to extender tube lumen 248. As depicted, connector 230 can further comprise a barbed connector tip 232. Barbed connector tip 232 can be configured to allow extender tube 214 to easily slide in one direction on connector 230 while restricting sliding in the opposite direction. Barbs on barbed connector tip 232 can be configured so that extender tube 214 easily slides over the barbs in one direction but does not easily slide in the opposite direction. Barbed connector tip 232 is discussed in greater detail below with reference to Fig. 18A.

Biasing element 234 is adapted to be positioned within a cavity formed in body 212 such that biasing element 234 is in abutment with the underside of release button 216. Biasing element 234 is configured to provide a biasing force against release button 216. The biasing force provided by biasing element 234 urges release button 216 in an upward direction, such that release button 216 is positioned in an undepressed state. In the undepressed state, release button 216 grips a cord positioned through surgical securement apparatus 210, as described below in greater detail with reference to Figs. 18A-18D.

Release button housing 236 is configured to be received within a cavity of body 212 in abutment with biasing element 234. Release button housing 236 further comprises slots 242, release button housing opening 252, and stops 246. Slots 242 facilitate locking of release button actuator 238 with respect to release button housing 236. Opening 252 is formed through release button housing 238 to enable a cord to be threaded therethrough.

Fig. 14 depicts a release button housing 236 configured so that in the shipping configuration button housing 226 is initially positioned around biasing element 234. Accordingly, biasing element 234 exerts no biasing force on release button housing 236 until release button 216 is activated. When release button actuator 238 is activated, release button actuator 238 is locked with respect to release button housing 236. A biasing force exerted on release button actuator 238 when locked together with release button housing 236 is naturally transferred to release button housing 236. A biasing force exerted on release button actuator 238 when locked together with release button housing 236 is naturally transferred to release button housing 236. Release button housing 236 and release button actuator 238 locked together form a single component comprising release button 216.

In the activated configuration, biasing element 234 can exert a biasing force against release button 216 to urge release button 216, including release button housing 236, to an undepressed position. Stops 246 on release button housing 236 engage body 212 when release button 216 is at the uppermost undepressed position to secure release button 216 within the cavity of body 212. Stops 246 are configured to engage body 212 and/or connector lumen 250 and/or the lumen of finger grip tube 218 to prevent the biasing force of biasing element 234 from ejecting release button 216 from body 212.

The biasing force of biasing element 234 also enables securement of a cord threaded through surgical securement apparatus 210. The cord threaded through surgical securement apparatus 210 is threaded through opening 252 of release button housing 236. As release button 216 is biased to an undepressed state, the bottom surface of the opening 252 of release button housing 236 is configured to compress the cord against the top surface of connector lumen 250 and/or the top surface of the finger grip tube lumen (not depicted). The biasing force compressing the cord thereby secures the cord in a fixed position relative to the surgical securement apparatus. Securement of a cord is discussed in greater detail below with reference to Figs. 18A-18C.

Release button actuator 238 is adapted to fit within a cavity of release button housing 236. Release button actuator 238 further comprises locking tabs 244, a post 240, and a release button actuator opening 254. In the illustrated embodiment, release button actuator 238 in the shipping configuration protrudes from release button housing 236 and extends above release button housing 236. Release button 216 is activated when release button actuator 238 is depressed to a specified position within release button housing 236. Slots 242 are positioned to receive locking tabs 244 at the specified position. When release button actuator 238 is depressed to the specified position, locking tabs 244 spring outward and are received within slots 242 of release button housing 236. Locking tabs 244 are received into slots 242 to lock release button actuator 238 with respect to release button housing 236. Activation of release button 216 is discussed in greater detail below with reference to Figs. 15A-15C.

Post 240 is positioned on the underside of release button actuator 238, adjacent to and/or in abutment with biasing element 234. In the illustrated embodiment, biasing element 234 partially surrounds post 240 such that post 240 supports biasing element 234 in a proper position to exert a biasing force on release button 216. Biasing element 234 exerts a biasing force on post 240 and/or release button actuator 238 urging release button actuator 238 in an upward direction. When release button actuator 238 is locked with respect to release button housing 236, the biasing force urges release button 216 to an undepressed state. If release button actuator 238 is not locked with respect to release button housing 236, biasing element 234 simply urges release button actuator 238 in an upward direction. In this manner release button 216 is maintained in the shipping configuration until a practitioner depresses release button actuator 238 into release button housing 236 to the specified position.

Release button actuator opening 254 is formed through release button actuator 238 to enable a cord to be threaded through release button actuator 238. In the illustrated embodiment, the opening 254 through release button actuator 238 is somewhat elongate, such that opening 254 has a diameter that is greater than the diameter of opening 252 through release button housing 236. The elongate configuration of opening 254 is such that opening 254 does not compress a cord threaded through surgical securement apparatus 210. Because the elongate shaped opening 254 is configured to not compress a cord running therethrough, the release button actuator 238 can be urged to an uppermost position that allows biasing element 234 to expand or decompress (to a position of lesser tension) more than is possible when release button 216 is in the activated configuration. Allowing biasing element 234 to more fully expand reduces or even eliminates any strain on biasing element 234. Stated differently, in the shipping configuration biasing element 234 is in a position of little or no tension to preserve the integrity of biasing element 234. Shipping and storing surgical securement apparatus 210 with biasing element in a position of little or no tension, or in a position that reduces or eliminates strain on biasing element 234, preserves the strength of the biasing element 234 until the time when the surgical securement apparatus is utilized.

As will be appreciated by a person of skill in the art, a variety of structures, components, and configurations are possible without departing from the scope of the present disclosure. For example, in one embodiment a plunger can be used in place of a release button to secure a cord within a surgical securement apparatus. The plunger can be slidably moveable within the cavity of the body. Moreover, the plunger may comprise a plunger housing and a plunger actuator. A variety of forms of plungers are possible and are known in the art. A release button, as in the embodiment of Figs. 13 and 214, is an example of one embodiment of a plunger. However, a release button in other embodiments may not be a plunger. Rather, a release button may be a button that operates a separate mechanical mechanism configured to secure a cord within a surgical securement apparatus.

As another example, a release button, according to another embodiment, can comprise locking means other than locking tabs for locking the release button actuator with respect to the release button housing. A button, a clip, a clasp, a pin, and a detent are all examples of other means for locking the release button actuator to the release button housing.

As another example, the biasing element may be a spring configured to compress a cord threaded through the surgical securement apparatus by retracting from an expanded position toward a position of little or no tension. In the shipping configuration, the biasing spring is in a position of little or no tension. The release button is activated by an actuator causing the spring biasing element to be expanded such that it compresses the cord as it exerts a biasing force toward a position of little or no tension. In still another embodiment the biasing element may comprise a component other than a spring. The biasing element may comprise a cantilever beam or a piece of resilient, elastomeric material.

Fig. 15A-15C depict release button 216 of a surgical securement apparatus in a shipping configuration and in both a depressed state and undepressed state of an activated configuration. Fig. 15A is a perspective view of release button 216 in the shipping configuration. In the illustrated embodiment, release button actuator 238 protrudes above the top surface of release button housing 236 when release button 216 is in the shipping configuration. Additionally, in the shipping configuration, release button actuator 238 and release button housing 236 are not locked together. As depicted, release button housing 236 is positioned fully within the cavity of body 212 while release button actuator 238 is urged upward so that a portion of release button actuator protrudes out of release button housing 238.

In the shipping configuration, release button actuator 238 extends higher than when locked with respect to release button housing 236. This allows the biasing element to substantially decompress, thereby reducing or eliminating strain on the biasing element. Reducing or eliminating strain on the biasing element preserves the strength of the biasing force until the surgical securement apparatus is to be activated and utilized for a surgical procedure. When a practitioner wants to use the surgical securement apparatus, the practitioner can activate the release button by depressing release button actuator 238 into release button housing 236.

Fig. 15B is a perspective view of release button 216 at the position of initial activation. In the illustrated embodiment, release button actuator 238 is fully depressed into release button housing 236. When release button actuator 238 is depressed to a specified position within release button housing 236, the locking tabs 244 (see Fig. 14) of release button actuator 238 spring out and are received into the slots 242 (see Fig. 14) of release button housing 236. Engagement of locking tabs 244 and slots 242 locks release button actuator 238 with respect to release button housing 236.

In the embodiment of Fig. 15B, the position of initial activation is also the depressed state of the activated configuration. When the release button 216 is in the depressed state, a lumen of release button 216 is formed by the opening through release button actuator 238 and the opening through release button housing 236. In the depressed state, the lumen through release button 216 is aligned with connector lumen 250 and the lumen of finger grip tube 218. The surgical securement apparatus does not compress a cord threaded therethrough. A practitioner can freely manipulate the cord with respect to the surgical securement apparatus. When the practitioner releases release button 216, the locked configuration release button actuator 238 relative to release button housing 236, results in cooperative movement of release button actuator 238 and release button housing 236 to an undepressed state. The biasing element exerts a biasing force on release button actuator 238, which in turn transfers the biasing force provided by the biasing element to release button housing 236. Accordingly, in an undepressed state release button 216 grips a cord threaded through the surgical securement apparatus.

Fig. 15C is a perspective view of release button 216 in an undepressed state of the activated configuration. As illustrated, release button actuator 238 and release button housing 236 are locked together, and release button 216 can be urged upward to an undepressed state. When release button 216 is urged to the undepressed state, release button 216 can grip a cord threaded through the surgical securement apparatus. Thus, the cord is secured with respect to the surgical securement apparatus. Securement of a cord is discussed in greater detail below with reference to Figs. 18A-18C.

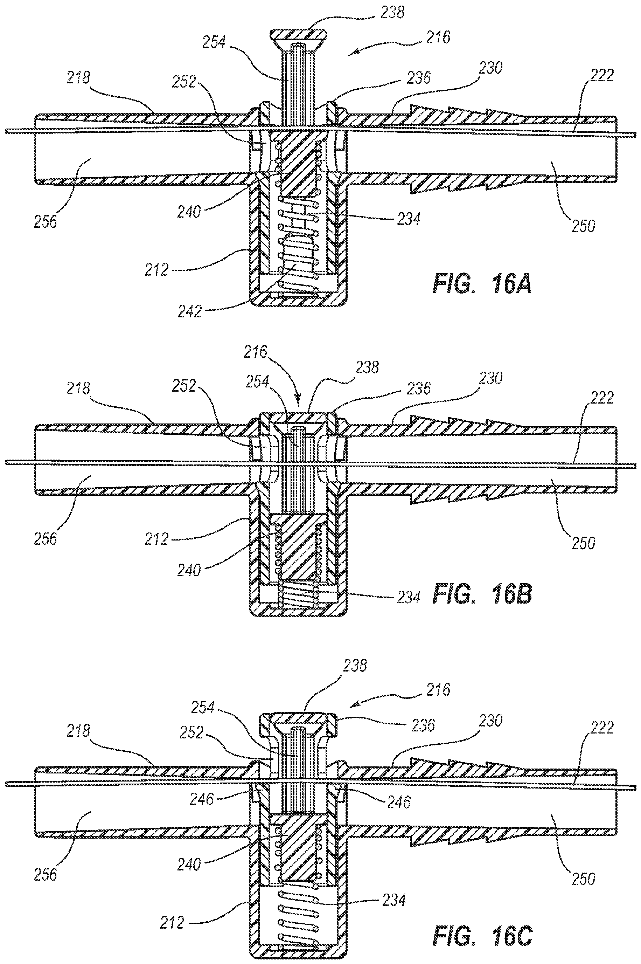

Fig. 16A-16C are cross-sectional side views depicting release button 216 in the shipping configuration and in both the depressed and undepressed state of the activated configuration. Fig. 16A is a cross-sectional side view of body 212 of a surgical securement apparatus when release button 216 is in the shipping configuration. In the illustrated embodiment, release button housing 236 is positioned within the cavity of body 212 when release button 216 is in the shipping configuration. The opening 252 through release button housing 236 is aligned with connector lumen 250. Release button actuator 238 is not locked with respect to release button housing 236. Accordingly, biasing element 234 urges release button actuator 238 to an extended position. Release button actuator 238 can have an elongate opening 254 that allows release button actuator 238 to be urged to an extended position while exerting little or no pressure on a cord, such as threading loop 222, threaded through the surgical securement apparatus. Opening 254 and biasing element 234 can be configured such that biasing element 234 can substantially expand without opening 254 compressing or exerting pressure on cord 222. In this manner, the shipping configuration preserves the integrity of biasing element 234 during shipping and storage of the surgical securement apparatus. Preserving the integrity of biasing element 234 also preserves the strength of the biasing force that the biasing element 234 can exert when release button 216 is activated and surgical securement apparatus is utilized. Release button 216 is activated when release button actuator 238 is depressed into release button housing 236 and locking tabs of release button actuator 238 lock into the locking slots 242 of release button housing 236.

Fig. 16B is a cross-sectional side view of a body of a surgical securement apparatus depicting actuation of release button 216. In Fig. 16B, release button actuator 238 is depressed into release button housing 236 to a specified position. When release button actuator 238 is depressed to the specified position within release button housing 236, locking tabs on release button actuator 238 extend or spring outwardly and engage slots on release button housing 236. In this manner, release button actuator 238 cooperatively engages release button housing 236 and release button 216 is activated. Moreover, as release button actuator 238 is depressed, biasing element 234 is loaded or compressed. When loaded, biasing element 234 is prepared to urge release button 216 to an undepressed position and thereby grip a cord threaded through the surgical securement apparatus.

In the embodiment illustrated in Fig. 16B, prior to activation, release button housing 236 is positioned within the cavity of body 212 similar to the positioning of release button housing 236 in the depressed state of the activated configuration. Release button housing 236 is positioned such that opening 252 through release button housing 236 is aligned with the connector lumen 250 and finger grip lumen 256. When release button actuator 238 is depressed into release button housing 236 to a specified position, release button actuator 238 locks with release button housing 236. Locking of release button actuator 238 with release button housing 236 activates release button 216 from the shipping configuration to the depressed state of the activated configuration.

As illustrated by Fig. 16B, at the point of initial activation, the lumen through release button 216, comprising release button actuator opening 254 and release button housing opening 252, is aligned with connector lumen 250 and finger grip lumen 256. There is no grip on cord 222. Accordingly, the practitioner can manipulate cord 222 with respect to surgical securement apparatus. Cord 222 may be a threading loop threaded through the surgical securement apparatus. Accordingly, in the depicted depressed state of the activated configuration, the practitioner can retract cord 222 pulling it through the surgical securement apparatus.

Fig. 16C is a cross-sectional side view of release button 216 in an undepressed state of the activated configuration. Release button 216 is gripping cord 222, thereby securing it with respect to surgical securement apparatus. Release button 216 grips cord 222 when biasing element 234 urges release button 216 in an upward direction to the undepressed state. As release button 216 is urged to the undepressed state, the bottom surface of the lumen of release button 216 compresses cord 222 against the top surface of lumen 250 and lumen 256. More specifically, the bottom edge of opening 252 through release button housing 236 engages cord 222 and compresses it against the top surface of connector lumen 250 and finger grip tube lumen 256. Stops 246 on release button housing 236 can facilitate in engaging cord 222. Stops 246 also engage the top surface of lumen 250 and lumen 256 to prevent release button housing, and accordingly release button 216, from being urged completely out of the cavity of body 212 by biasing element 234.

Comparing Fig. 16A to Fig. 16C illustrates how the strength of the biasing force is preserved by the shipping configuration of release button 216. More specifically, the advantages of a shipping configuration are observed by comparing the position of the bottom surface of opening 252 through release button housing 236 relative to the position of the top of biasing element 234. In Fig. 16A, the bottom surface of opening 252 is below the top of biasing element 234. Accordingly, biasing element 234 can substantially expand to a position of relatively little or no tension. By contrast, in Fig. 16C the bottom surface of opening 252 of release button housing 236 is positioned above the top of biasing element 234. Accordingly, biasing element 234 is in a position of greater tension. The position of less tension depicted in Fig. 16A preserves the integrity of biasing element 234, thereby preserving the strength of the biasing force for when release button 216 is activated.

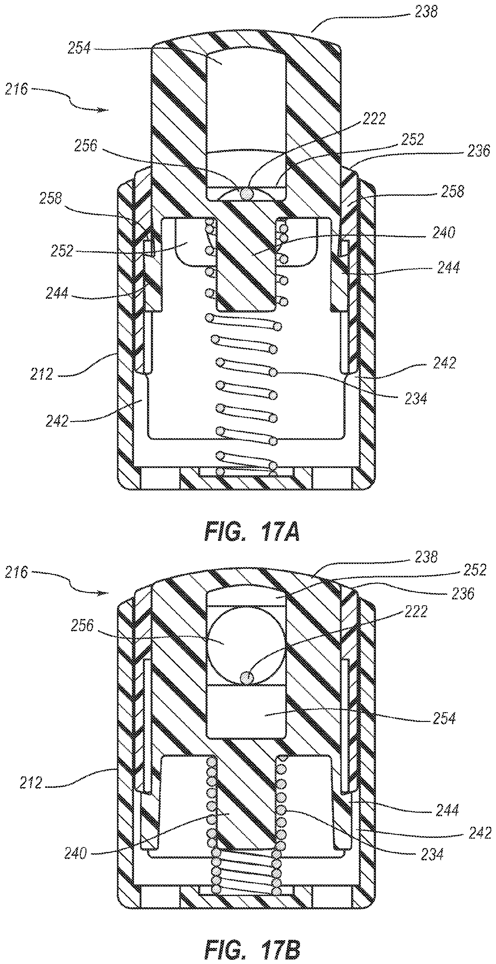

Figs. 17A-17C further illustrate activation of the release button and relative position of the bottom surface of the release button lumen relative to the top of biasing element 234. Furthermore, these figures further demonstrate how the two-parts of release button 216, namely the actuator and the housing, enable release button 216 to have a shipping configuration and an activated configuration. The figures further illustrate that the shipping configuration allows biasing element 234 to substantially decompress to a position of relatively little tension, which preserves the integrity of the biasing element and thereby preserves the strength of the biasing force that the biasing element 234 can exert when compressed.

Fig. 17A is a cross-sectional front view of release button 216 in the cavity of body 212. In Fig. 17A, release button 216 is in the shipping configuration. Release button actuator 238 is not yet locked with respect to release button housing 236. In the illustrated embodiment, release button housing 236 is at its lowest position within the cavity of body 212. Release button housing opening 252 is substantially aligned with finger grip tube lumen 256. By contrast, release button actuator 238 is at its extended position. Accordingly, biasing element 234 is in a position of little or no tension. Release button actuator opening 254 is substantially raised, but the elongate shape of opening 254 allows release button actuator 238 to be raised to an uppermost position without substantially compressing or gripping a cord threaded through opening 254. Biasing element 234 is substantially decompressed to a position of little or no tension, such that any biasing force is minimal. To ensure that release button actuator 238 is secured within release button housing, locking tabs 244 of release button actuator 238 engage stops 258 on the inner surface of release button housing 236 when release button actuator 238 is at its extended position.

Fig. 17B is a cross-sectional front view of release button 216 at the point of initial activation. Release button actuator 238 is depressed into release button housing 236 to a point where locking tabs 244 engage locking slots 242 to lock release button actuator 238 with respect to release button housing 236. Accordingly, as biasing element 234 exerts a biasing force on post 240 of release button actuator 238, both release button actuator 238 and release button housing 236 are urged together to an undepressed state.

In the illustrated embodiment of Fig. 17B, release button 216 is also in a depressed state of the activated configuration. When release button 216 is depressed as illustrated, the release button lumen, comprising release button housing opening 252 and release button actuator opening 254, aligns with finger grip tube lumen 256. In the depressed position, with the lumen of release button 216 aligned with finger grip tube lumen 256, release button 216 is not configured to secure a cord threaded through the surgical securement apparatus. Accordingly, a practitioner can manipulate a cord threaded through the surgical securement apparatus. The practitioner can also adjust positioning of the surgical securement apparatus relative to the cord.

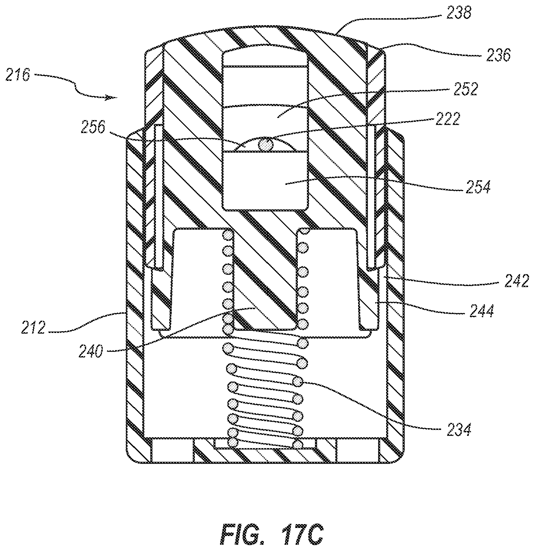

Fig. 17C is a cross-sectional front view of release button 216 in the undepressed position of the activated configuration. When the practitioner releases release button 216, biasing element 234 urges release button 216 to an undepressed position. In the illustrated embodiment, when release button 216 is in an undepressed position, the lumen of release button 216 moves out of alignment with finger grip tube lumen 256. More specifically, the bottom surface of release button housing opening 252 can compress a cord threaded therethrough against the top surface of finger grip tube lumen 256 and connector lumen (not depicted). Biasing element 234 exerts a biasing force urging release button 216 upward to compress the cord. The compression creates a grip on the cord that thereby secures the cord relative to the surgical securement apparatus.

A comparison of release button 216 in the undepressed state of the activated configuration as depicted in Fig. 17A with the shipping configuration as depicted in Fig. 17C again demonstrates how the shipping configuration preserves the strength of the biasing element during shipping and storage. In the shipping configuration, as illustrated in Fig. 17A, the bottom surface of release button housing opening 252 is lower than the top of biasing element 234. In the undepressed state of the activated configuration, as depicted in Fig. 17C, the bottom surface of release button housing opening 252 is higher than the top of biasing element 234. The shipping configuration depicted in Fig. 17A allows biasing element 234 to more fully expand to a position of little or no compression. Allowing biasing element 234 to more fully expand in the shipping configuration, preserves the strength of the biasing force that the biasing element can exert when compressed.

Comparing Fig. 17A with Fig. 17C also illustrates how the activated configuration seeks to maximize the biasing force of biasing element 234. The activated configuration maintains the biasing element in a slightly compressed state, displaced to a position of tension. Slightly displacing the biasing element concentrates the biasing force that the biasing element 234 exerts. Both preserving and concentrating the biasing force ensures that the surgical securement apparatus can adequately secure a cord threaded therethrough.

Figs. 18A- 18D illustrate how a threading assembly 220 is used in connection with a surgical securement apparatus 210 to thread a securement cord 226 through surgical securement apparatus 210. In the illustrated embodiment, release button 216 is in the activated configuration. The release button actuator 238 is depressed into, and secured with respect to, the release button housing 236. Accordingly, release button housing 236 and release button actuator 238 are functioning together as release button 216. A practitioner can depress release button 216 to release securement on a cord that is threaded through the surgical securement apparatus 210. The practitioner releases release button 216 to again secure a cord threaded through surgical securement apparatus 210.

Fig. 18A is a cross-sectional side view of surgical securement apparatus 210, according to one embodiment of the present disclosure. In the illustrated embodiment, a connector 230 includes a barbed connector tip 232 that engages and secures extender tube 214 relative to the other components of surgical securement apparatus 210. In the illustrated embodiment, barbed connector tip 232 comprises a "Christmas tree" connector. The barbs of barbed connector tip 232 are adapted to allow extender tube 214 to slide onto connector 230. Once the extender tube 214 slides past a barb, the barb restricts extender tube 214 from sliding in an opposite direction, such that that it is removed from connector 230, by engaging the inside of extender tube 214 if moved in the opposite direction. Barbed connector tip 232 engages and secures extender tube 214 in a manner that extender tube lumen 248 is in communication with connector lumen 250. Connector lumen 250 runs through the center of barbed connector tip 232. Barbed connector tip 232 is inserted and received into extender tube lumen 248, thus allowing a continuous lumen through both structures.

In the embodiment of Fig. 18A, release button 216 is depicted in an undepressed position of the activated configuration. Threading loop 222 extends through surgical securement apparatus 210, such that a portion of threading loop 222 projects distally from extender tube 214. The portion of threading loop 222 that extends distally of extender tube 214 is utilized to capture a securement cord and thread the securement cord through surgical securement apparatus 210.

When release button 216 of the illustrated embodiment is in an undepressed position, threading loop 222 is compressed between release button 216 and body 212. The compression of threading loop 222 restricts movement of threading loop 222 relative to surgical securement apparatus 210. Biasing element 234 exerts a biasing force to cause compression of threading loop 222. Biasing element 234 abuts with the underside of release button 216 and is positioned adjacent post 240 of release button 216, such that biasing element 234 exerts a biasing force that urges release button 216 in an upward direction. When release button 216 is urged upward relative to body 212, the release button lumen, comprising release button housing opening 252 and release button actuator opening 254, is shifted out of alignment with connector lumen 250 and finger grip tube lumen 256. In this manner, threading loop 222, or any cord threaded through the lumen of surgical securement apparatus 210, is compressed between the bottom surface of release button housing opening 252 and both the top surface of connector lumen 250 and the top surface of finger grip tube lumen 256. The cooperative engagement by the surfaces of connector lumen 250, release button housing opening 252, and finger grip tube lumen 256 provide an effective mechanism for securing a desired degree of tension on threading loop 222 or another cord threaded along the length of surgical securement apparatus 210.

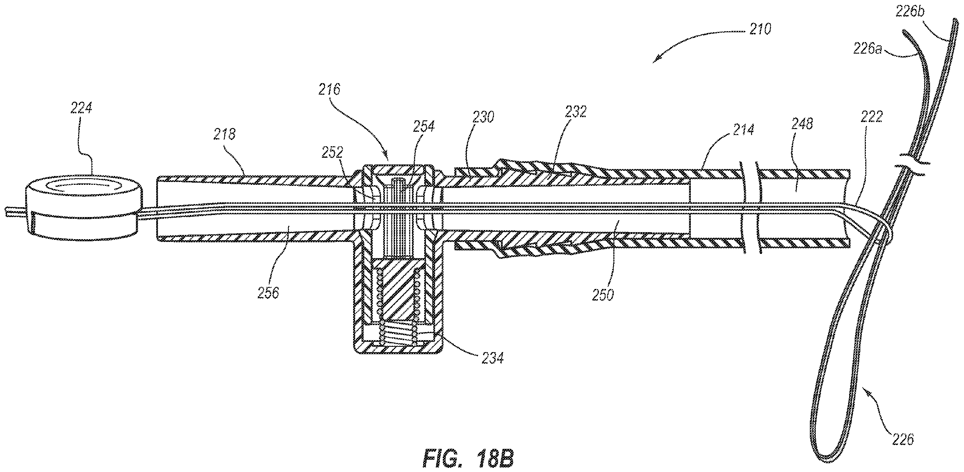

Fig. 18B is another cross-sectional side view of surgical securement apparatus 210 depicting release button 216 in a depressed position. The practitioner depresses release button 216 when loosening the tension on a cord is desired. As the practitioner depresses release button 216, the release button 216 is urged in a downward direction and biasing element 234 is compressed. Additionally, as release button 216 is urged in a downward direction, the release button lumen, comprising release button housing opening 252 and release button actuator opening 254, aligns with connector lumen 250 and finger grip tube lumen 256. As a result, threading loop 222 is no longer cooperatively engaged between opposing surfaces of release button housing opening 252, connector lumen 250, and finger grip tube lumen 256. When threading loop 222 is not cooperatively engaged, the grip of surgical securement apparatus 210 on threading loop 222 is released and threading loop 222 is moveable with respect to surgical securement apparatus 210.

When release button 216 is depressed and the grip on threading loop 222 is released, clasp 224 can be retracted in a rearward direction to pull threading loop 222. In Fig. 18B, first tail 226a and second tail 226b are threaded through the portion of threading loop 222 that extends distally of extender tube 214. As clasp 224 is retracted in a rearward direction, the portion of threading loop 222 projecting distally from extender tube 214 is drawn into extender tube lumen 248. Because first tail 226a and second tail 226b are threaded through threading loop 222, retraction of threading loop 222 pulls first tail 226a and second tail 226b into extender tube lumen 248. As clasp 224 is further retracted in a rearward direction, threading loop 222 is then drawn into connector lumen 250 of connector 230. Accordingly, first tail 226a and second tail 226b are also pulled into connector lumen 250. The distal end of connector tip 214 may be tapered to thereby facilitate guidance of first tail 226a and second tail 226b into connector lumen 250.

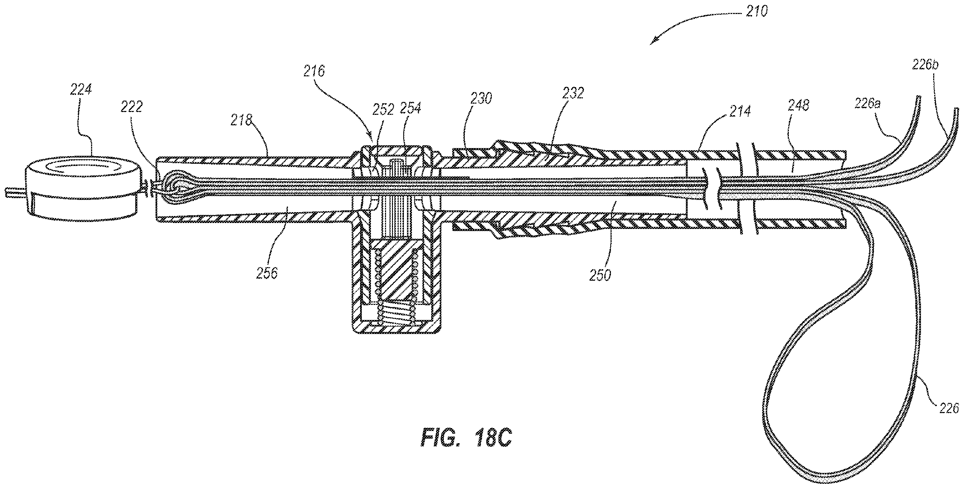

Fig. 18C is still another cross-sectional side view of surgical securement apparatus 210, illustrating the manner in which clasp 224 and threading loop 222 are utilized to draw securement cord 226 into surgical securement apparatus 210. As depicted, continued retraction of threading loop 222 draws the end of threading loop 222 through the release button lumen, comprising release button housing opening 252 and release button actuator opening 254, and into finger grip tube lumen 256. In Fig. 18C, release button 216 is in a depressed position, similar to the position of release button 216 in Fig. 15B. When release button 216 is in the depressed position, the release button lumen aligns with connector lumen 250 and finger grip tube lumen 256. As threading loop 222 is pulled in a rearward direction, first tail 226a and second tail 226b are also drawn through the release button lumen and into finger grip tube lumen 256. As will be appreciated by those skilled in the art, as the practitioner continues to retract threading assembly 220 in a rearward direction, the first tail 226a and second tail 226b are pulled along the entire length of surgical securement apparatus 210 until first tail 226a and second tail 226b extend out of finger grip tube 218 of surgical securement apparatus 210. In this manner, threading assembly 220 facilitates the quick, simple and intuitive threading of first tail 226a and second tail 226b of securement cord 226 through surgical securement apparatus 210.

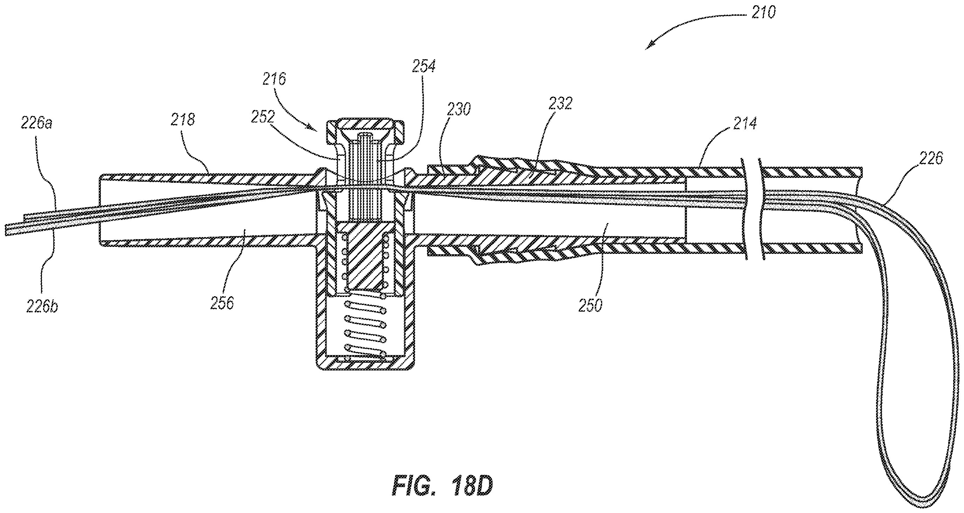

Fig. 18D is still another cross-sectional side view of surgical securement apparatus 210, illustrating first tail 226a and second tail 226b of securement cord 226 threaded completely through the length of surgical securement apparatus 210. Once securement cord 226 is completely threaded through surgical securement apparatus 210, a practitioner may desire to secure the position of securement cord 226 with respect to surgical securement apparatus 210. To secure securement cord 226, the practitioner simply allows release button 216 to release to an undepressed position. When release button 216 is released, biasing element 234 urges release button 216 to the undepressed position. As described above with reference to Fig. 18A, when release button 216 is in an undepressed position release button 216 engages a cord such as securement cord 226 positioned through the length of surgical securement apparatus 210. Release button 216 engages securement cord 226 by compressing securement cord 226 between the bottom surface of release button housing opening 252 and both the top surface of connector lumen 250 and the top surface of finger grip tube lumen 256. Thus, release button 216 provides selective securement of securement cord 226.

The practitioner can also release the grip on securement cord 226 by depressing release button 216. As described above with reference to Fig. 18B, as release button 216 is urged in a downward direction, the lumen of the release button, including release button housing opening 252 and release button actuator opening 254, aligns with connector lumen 250 and finger grip tube lumen 256. Securement cord 226 is no longer cooperatively engaged between opposing surfaces of release button housing opening 252, connector lumen 250, and finger grip tube lumen 256. Accordingly, the practitioner can move surgical securement apparatus 210 longitudinally relative to first tail 226a and second tail 226b of securement cord 226. Releasing of the grip of surgical securement apparatus 210 on securement cord 226 by depressing release button 216 allows the practitioner to loosen, tighten, or make other changes in the juxtaposition of surgical securement apparatus 210 relative to securement cord 226 and relative to the one or more intracorporeal structures thereby secured.

As will be appreciated by those skilled in the art, a variety of types and configurations of surgical securement apparatus can be utilized without departing from the scope of the disclosure. For example, the juxtaposition and mechanism utilized to secure a cord relative to the surgical securement apparatus can be configured differently. The release button may be on either side, rather than on the top. In another embodiment, two release buttons act cooperatively to engage and secure the cord. The two release buttons may be positioned on opposite sides of the body. In another embodiment, the cord may be compressed between a top surface of the release button lumen and both the bottom surface of the connector lumen and the bottom surface of the finger grip lumen.

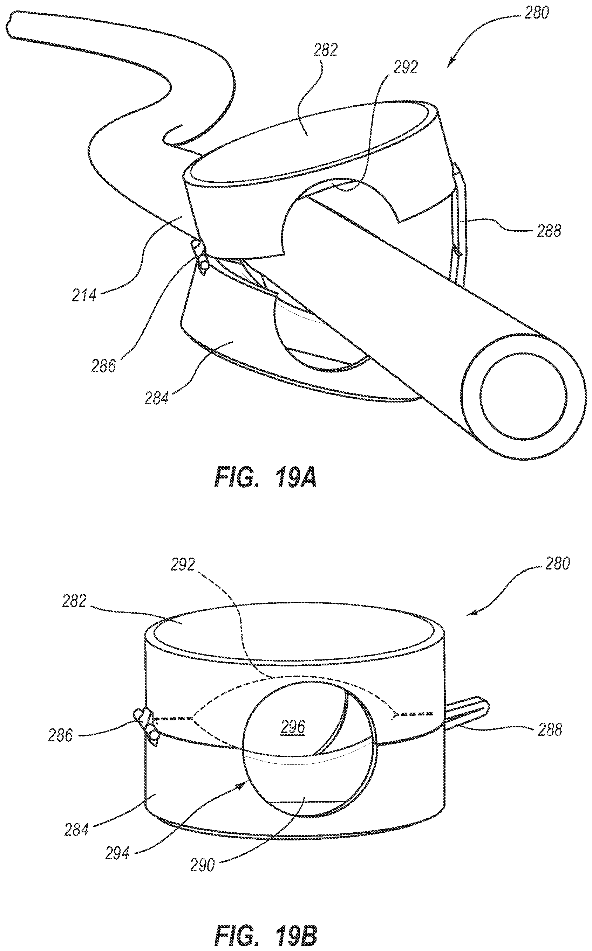

Figs. 19A-19D depict one embodiment of an extender tube trimming device 228, according to the present disclosure. Fig. 19A is a perspective front view of extender tube trimming device 280 coupled to an extender tube 214 of a surgical securement apparatus, according to one embodiment of the present disclosure. In the illustrated embodiment, extender tube trimming device 228 comprises a first housing portion 282 and a second housing portion 284 coupled together by a hinge 286 and a support member 288. Hinge 286 and support member 288 facilitate first housing portion 282 and second housing portion 284 rotating between an open position and a closed position. The illustrated embodiment is in an open position. When closed, first housing portion 282 and second housing portion 284 form an opening through extender tube trimming device 280, as described more fully below with reference to Fig. 19B. In the illustrated embodiment, first housing portion 282 encloses a first curved blade 292 and second housing portion 284 encloses a second curved blade 290.

In the illustrated embodiment, first housing portion 282 and second housing portion 284 are adapted to mate together, similar to a clamshell, to provide a support structure for extender tube trimming device 280. As depicted, first housing portion 282 and second housing portion 284 can be substantially disk-shaped. First housing portion 282 and second housing portion 284 are coupled together on one end by hinge 286. Hinge 286 allows first housing portion 282 and second housing portion 284 to rotate between an open position and a closed position.

Support member 288 is adapted to restrict full rotation of first housing portion 282 and second housing portion 284 about hinge 286, yet allows first and second housing portions 282, 284 to rotate between an open position and a closed position. Support member 288 is coupled to first housing portion 282 and second housing portion 284 substantially opposite hinge 286. Support member 288 can function as a biasing element, exerting a biasing force to urge first housing portion 282 and second housing portion 284 toward the closed position. The biasing force causes extender tube trimming device 280 to gently grip extender tube 214 when extender tube 214 is positioned as if threaded through lumen 294 of extender tube trimming device 280. The gentle gripping action of extender tube trimming device 280 substantially secures extender tube trimming device 280 to extender tube 214 without cutting extender tube 214.

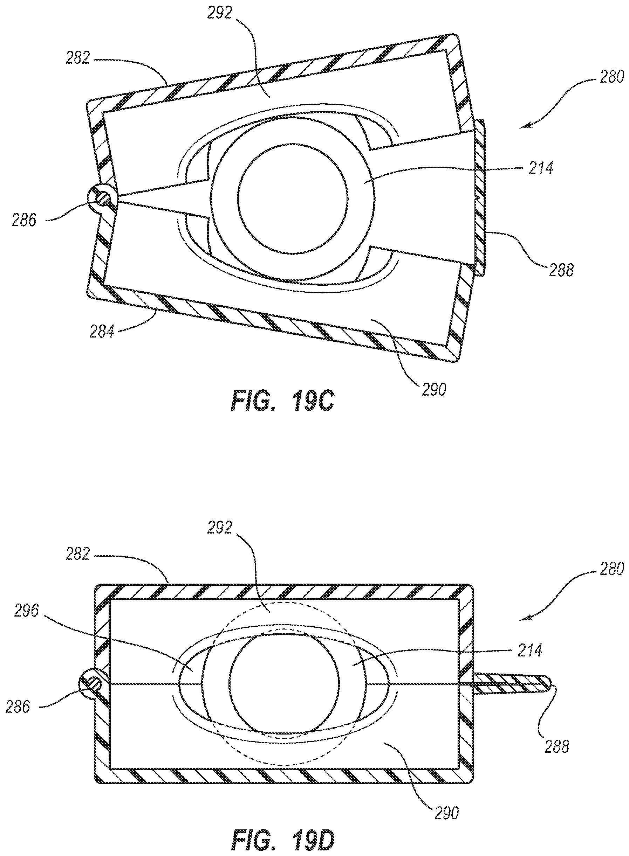

When a practitioner desires to cut off a portion of extender tube 214 to trim extender tube 214 to a desired length, the practitioner can adjust the position of extender tube trimming device 280 along extender tube 214 to the desired length and operate extender tube trimming device 280 to cut off the undesired length. The practitioner operates extender tube trimming device 280 by grasping first housing portion 282 and second housing portion 284 between the thumb and another finger and pressing first housing portion 282 and second housing portion 284 together. Pressing first housing portion 282 and second housing portion 284 together provide sufficient force for first blade 292 and second blade 290 to begin to cut extender tube 214. Blades 290, 292 have a curved shape, configured to cut extender tube 214 without cutting a cord threaded through extender tube 214 as discussed in greater detail below with reference to Figs. 19C and 19D. The closure of extender tube trimming device 280 may not fully sever extender tube 214. However, the practitioner can twist extender tube trimming device 280, rotating it about extender tube 214. The rotating action causes the sharp edges of blades 290, 292 to gradually cut through extender tube 214.

Fig. 19B is another perspective front view of the extender tube trimming device of Fig. 19A in the closed position and not coupled to an extender tube. When first housing portion 282 and second housing portion 284 are in the closed position, an opening 294 is formed through extender tube trimming device 280. Opening 294 is adapted to receive extender tube 214 and guide positioning of extender tube 214 while operating extender tube trimming device 280. Opening 294 runs longitudinally in a direction parallel with the axis of rotation of first housing portion 282 and second housing portion 284 about hinge 286. Opening 294 is also formed along the plane formed by the surface at which first housing portion 282 mates with second housing portion 284. In this manner, substantially half of opening 294 is formed by first housing portion 282 and substantially the other half of opening 294 is formed by second housing portion 284.

Blades 290, 292 in the illustrated embodiment are disposed within and partially intersect opening 294 so as to facilitate cutting of an extender tube. First blade 290 and second blade 292 come together to form an eyelet 296 when extender tube trimming device 280 is in the closed position, as depicted in Fig. 19B. In the illustrated embodiment, eyelet 296 is oblong and has a diameter that is smaller in at least one direction than the smallest diameter of opening 294 and the diameter of the lumen of an extender tube. The smaller diameter enables curved blades 290, 292 to cut through the extender tube. Moreover, eyelet 296 has a smallest diameter that is larger than the diameter of a cross section of a securement cord threaded through the extender tube. Accordingly, blades 290, 292 can cut the extender tube without cutting the securement cord.

Fig. 19C is cross-sectional front view of the extender tube trimming device 280 of Fig. 19A in an open position. As previously mentioned, support member 288 can be configured to exert a gentle biasing force urging first housing portion 282 and second housing portion 284 toward the closed position. Accordingly, blades 290, 292 gently grip extender tube 214 to prevent extender tube trimming device 280 from sliding off extender tube 214.

Fig. 19D is cross-sectional front view of the extender tube trimming device of Fig. 19A in a closed position. As a practitioner presses together first housing portion 282 and second housing portion 284, extender tube 214 is received within opening 294 and first and second curved blades 290, 292 engage the surface of extender tube 214. First blade 292 cuts through a first portion of extender tube 214, as depicted by phantom lines showing the outline of extender tube 214. Moreover, second blade 290 cuts through a second portion of extender tube 214, as also depicted by phantom lines. The practitioner can cut the remaining portions of extender tube 214 by maintaining the closed position of first housing portion 282 and second housing portion 284 and rotating extender tube trimming device 280 relative to extender tube 214. As extender tube trimming device 280 is rotated, first curved blade 292 and second curved blade 290 engage and cut the uncut portions of extender tube 214 to completely sever the portion of extender tube 214 distal to curved blades 290, 292. In this manner, extender tube 214 can be trimmed to a desired length.

As will be appreciated by those skilled in the art, a variety of types and configurations of extender tube trimming devices can be utilized without departing from the scope of the disclosure. For example, an extender tube trimming device having a first housing portion and a second housing portion shaped other than a disk is possible. First housing portion and second housing portion may be shaped to form a sphere when pressed together. First housing portion and second housing portion may also be shaped like a triangle, square, rectangle, or any desired shape that is capable of housing one or more blades and being pressed substantially together such that the blades cut an extender tube. As another example, the support member may comprise a spring configured to urge the first and second housing portions to a closed position. In another embodiment, the hinge can be spring-loaded and configured to urge the first and second housing portions to a closed position. In yet another embodiment, the blades may be configured differently. The blades can be straight, rather than curved, and adapted to not entirely close together so as to avoid severing a cord threaded through the extender tube. In still another embodiment, the blades can be straight and configured to engage and cut the extender tube at an angle so as to not cut a cord threaded through the extender tube.

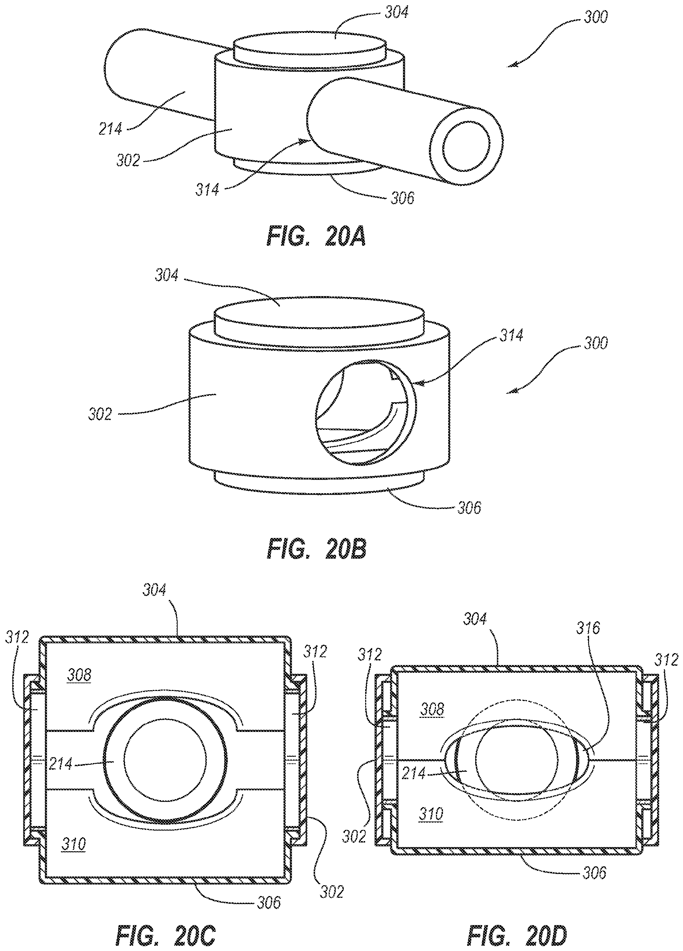

Figs. 20A-20D depict another embodiment of an extender tube trimming device 300 according to the present disclosure. Fig. 20A is a perspective front view of an extender tube trimming device 300 coupled to an extender tube 214 of a surgical securement apparatus. The illustrated extender tube trimming device 300 comprises a housing 302, a first button 304, and a second button 306. Housing 302 in the illustrated embodiment is disk-shaped. An opening 314 is formed through housing 302. Opening 314 is configured to receive extender tube 214. In the illustrated embodiment, at least a portion of opening 314 is circular and has substantially the same diameter as the outer surface of extender tube 214, such that friction between the circular portion of opening 314 and extender tube 214 somewhat restricts movement of extender tube trimming device 300 relative to extender tube 214.

Fig. 20B is another perspective front view of the extender tube trimming device of Fig. 20A without an extender tube positioned through opening 314. A first blade 308 (see Fig. 20c) is coupled to first button 304 and disposed within housing 302. First blade 308 initially does contact an extender tube positioned through opening 314. When first button 304 is depressed, first blade 308 is urged into opening 314 such that first blade 308 can engage the surface of an extender tube positioned through opening 314. A second blade 310 (see Fig. 20c) is coupled to second button 306 and disposed within housing 302 opposite first blade 308. When second button 306 is depressed, second blade 310 is urged into opening 314 such that second blade 310 can engage the surface of an extender tube positioned through opening 314.

Fig. 20C is cross-sectional front view of the extender tube trimming device of Fig. 20A with first button 304 and second button 306 in an undepressed position. One or more spring biasing members 312 are positioned within housing 302 and configured to urge first button 304 and second button 306 to an undepressed position. Accordingly, first blade 308 and second blade 310 are not engaged and slicing through extender tube 214.

Fig. 20D is another cross-sectional front view of the extender tube trimming device of Fig. 20A. First blade 308 and second blade 310 are engaged and cutting an extender tube positioned through the extender tube trimming device. As illustrated, first blade 308 and second blade 310 are configured to not cut completely through extender tube 214 individually. Rather, the blades 308, 310 come together to form an eyelet 316, similar to extender tube trimming device 280 of Figs. 19A-19D. Eyelet 316 has a smallest diameter that is greater than the diameter of a cord threaded through extender tube 214 so as to not cut the cord. Described in other terms, first blade 308 is configured to cut only a portion of extender tube 214 at a time so as to not cut a cord threaded through extender tube 214. Similarly, second blade 310 is configured to cut only a portion of extender tube at a time so as to not cut a cord threaded through extender tube 214. Completely severing extender tube 214 requires the practitioner to maintain one or both of first button 304 or second button 306 in a depressed position and rotate extender tube trimming device 300 around extender tube 214. Extender tube 214 can also be twisted with respect to extender tube trimming device 214 while first button 304 and second button 306 are depressed. In this manner, first blade 308 and/or second blade 310 are positioned to engage and slice all portions of extender tube 214.

As can be appreciated, first blade 308 and second blade 310 may or may not cut through a portion of extender tube 214 upon initial contact. Initial contact between a blade 304, 306 and extender tube 314 may simply cause extender tube 214 to collapse. However, extender tube 214 is formed of resilient flexible material, such that the outer surface will maintain contact with blades 308, 310 while first button 304 and second button 306 are depressed. Rotation of extender tube trimming device 300 about extender tube 214 with at least one of first button 304 or second button 306 depressed allows the sharp edge of first blade 308 and/or second blade 310 to engage extender tube 214 and gradually slice all portions around extender tube 214.

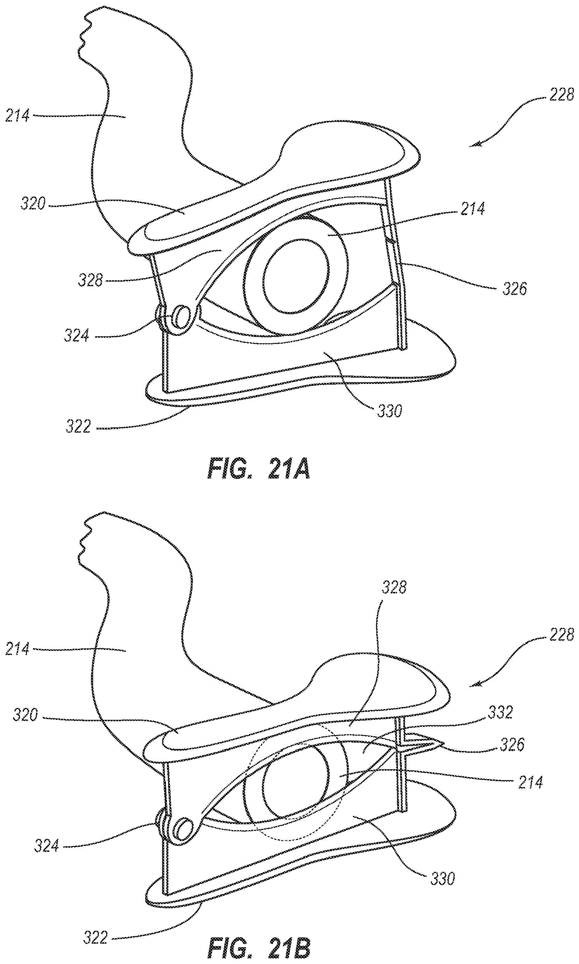

Fig. 21A is a perspective front view of an extender tube trimming device 228 according to another embodiment of the present disclosure. Extender tube trimming device 228 is also depicted in Figs. 13 and 14. Extender tube trimming device 228 comprises a first finger grip 320 coupled to a first blade 328, a second finger grip 322 coupled to a second blade 330, and a hinge 324 to couple together first blade 328 and second blade 330. First blade 328 and second blade 330 can rotate about hinge 324 between an open position and a closed position. In the illustrated embodiment, first blade 328 and second blade 330 are further coupled together opposite hinge 324 by a support member 326. Support member 326 is positioned to restrict full rotation of blades 328, 330 about hinge 324 while allowing blades 328, 330 to rotate between an open and closed position.

In the illustrated embodiment, hinge 324 can be spring-loaded so as to exert a slight biasing force urging first blade 328 and second blade 330 toward the closed position. The biasing force is sufficient to cause blades 328, 330 to engage extender tube 214, thereby restricting extender tube trimming device 228 from sliding off of extender tube 214. However, the biasing force is not sufficient to cause blades 328, 330 to slice extender tube 214. A practitioner desiring to trim extender tube 214 can grasp extender tube trimming device 228 between a thumb and other finger and press blades 328, 330 together. The thumb and finger can each grip first finger grip 320 or second finger grip 322. Finger grips 320, 322 can be adapted to provide a comfortable surface against which the practitioner can squeeze extender tube trimming device 228.

Fig. 21B is another perspective front view of the extender tube trimming device of Fig. 21A with first blade 328 and second blade 330 pressed together to a closed position. As illustrated, first blade 328 and second blade 330 have a slightly curved shape to facilitate cutting extender tube 214. Accordingly, first blade 328 and second blade 330 come together to form an eyelet 332 when extender tube trimming device 228 is in the closed position. In the illustrated embodiment, eyelet 332 is oblong and has a diameter that is smaller in at least one direction than the lumen of an extender tube. The smaller diameter of eyelet 332 enables curved blades 328, 330 to cut through extender tube 214. Moreover, eyelet 332 has a smallest diameter that is larger than the largest diameter of a cross-section of a securement cord threaded through extender tube 214. Accordingly, blades 328, 330 can cut the extender tube without cutting the securement cord. Furthermore, pressing blades 328, 330 together does not completely sever extender tube 214, similar to the embodiments described above. A combination of pressing blades 328, 330 together and rotating extender tube trimming device 228 around extender tube 214 can completely cut extender tube 214.

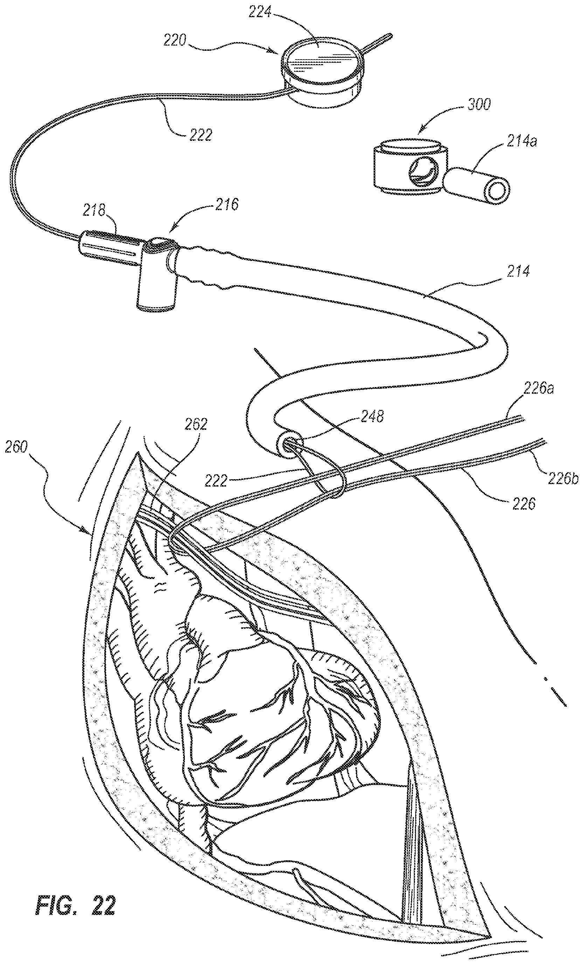

Fig. 22 is a perspective view of a surgical securement apparatus 210 being utilized in connection with a surgical procedure. Extender tube trimming device 228 has been utilized to cut off a portion of extender tube 214a, thereby trimming extender tube 214 to a desired length. Trimmed extender tube portion 214a has been cut from extender tube without severing threading loop 222. Fig. 22 also illustrates positioning of threaded loop 222 just prior to first tail 226a and second tail 226b of securement cord 226 being pulled into extender tube lumen 248. Threading assembly 220 is partially retracted and threading loop 222 is nearly drawn into extender tube lumen 248. Securement cord 226 is threaded around one or more intracorporeal structures 262 within a surgical site 260. First tail 226a and second tail 226b are threaded through threading loop 222. As threading loop 222 is further retracted, threading loop 222 will capture first tail 226a and second tail 226b and pull them into extender tube lumen 248.

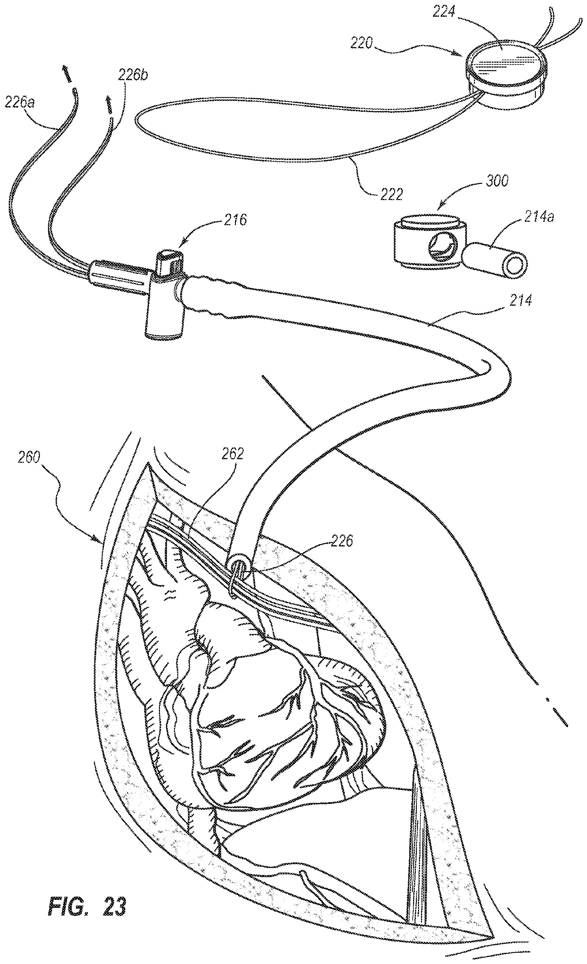

Fig. 23 is a perspective view of a surgical securement apparatus 210 positioned to secure a securement cord 226 with a desired degree of tension around various intracorporeal structures 262 to be secured during a surgical procedure. Threading assembly 220 has been fully retracted, such that threading loop 222 is withdrawn from surgical securement apparatus 210. Accordingly, first tail 226a and second tail 226b of securement cord 226 have been threaded along the entire length of surgical securement apparatus, through extender tube 214, body 212, and finger grip tube 218, such that first tail 226a and second tail 226b are extending out through the proximal end of finger grip tube 218. Furthermore, surgical securement apparatus 210, including extender tube 214, has been advanced forward along securement cord 226 such that the distal end of extender tube 214 is positioned adjacent intracorporeal structures 262 and the loop in securement cord 226 is taut. Although the figure depicts the securement cord 226 as somewhat loose, it can be appreciated by those skilled in the art that the tension of the securement cord can be adjusted as desired.

Fig. 23 illustrates how extender tube 214 enables securement of intracorporeal structures 262 within surgical site 260 while allowing body 212 of surgical securement apparatus 210 to remain outside surgical site 260. As described with reference to Fig. 21, the length of extender tube 214 can be adjusted using extender tube trimming device 228. The length of extender tube 214 can be adjusted such that the proximal end of extender tube 214 extends outside the surgical site. Moreover, the length of extender tube 214 allows the distal tip of extender tube 214 to be positioned immediately adjacent an intracorporeal structure 262 to be secured. The length of extender tube 214 is sufficient to extend away from the secured structure and provide displacement between the tip of extender tubing 214 and body 212 of surgical securement apparatus 210. The displacement allows tight securement at the tip of extender tubing while moving actual actuation of securement well outside the surgical site. Body 212 can hang out of the surgical site so as to not obstruct any structures or otherwise impede the surgical procedure. By hanging out of the surgical site, body 212 can also provide an appropriate amount of weight to stabilize and/or pull ancillary structures that are not a part of the procedure to the periphery of the surgical site. Moreover, body 212 is readily accessible for a practitioner to grasp to locate a desired structure.

As will be understood by those skilled in the art, utilization of the surgical securement apparatus with a securement cord to secure intracorporeal structures is merely an exemplary embodiment illustrating operation of the surgical securement apparatus relative to securing such intracorporeal structure and should in no means be considered to be limiting in nature. For example, the surgical securement apparatus can be utilized with sutures that are within the surgical site. Additionally, the surgical securement apparatus can be utilized for securing additional medical devices such as a suture ring of a catheter hub to a patient.

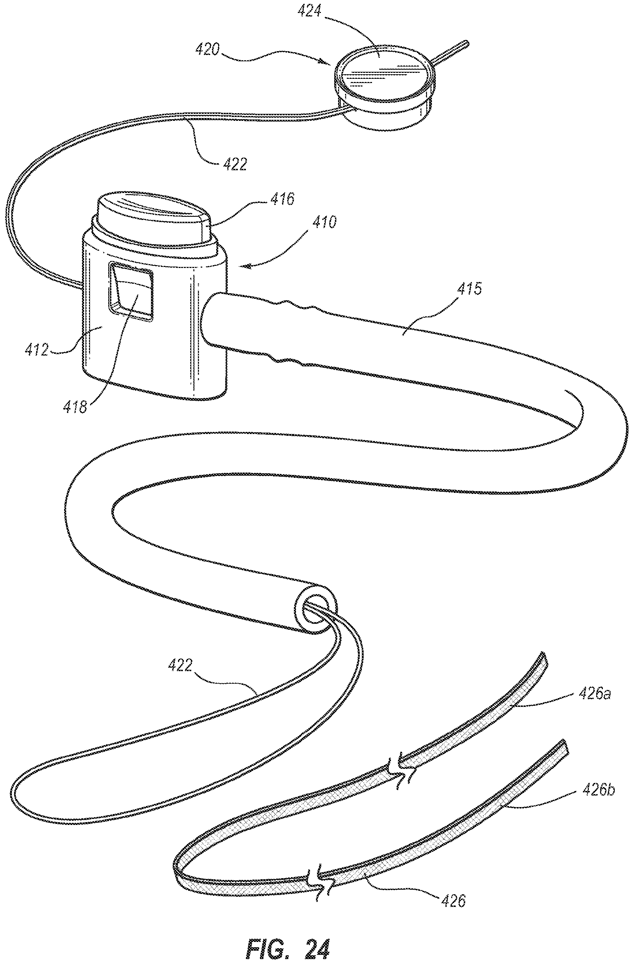

Fig. 24 is a perspective view of a surgical securement apparatus according to another embodiment of the present disclosure. Surgical securement apparatus 410 is utilized to selectively and releasably secure a securement cord 426 which is adapted to secure intracorporeal structures during various types of surgical procedures. Securement cord 426 is threaded around one or more intracorporeal structures within a surgical site and is then threaded through surgical securement apparatus 410. Surgical securement apparatus 410 facilitates tightening of the loop of securement cord 426 around one or more secured intracorporeal structures. Additionally, surgical securement apparatus 410 is adapted to hang outside the surgical site to enable selective securement of securement cord 426 from outside the surgical site. Selective securement of securement cord 426 allows a practitioner to quickly, easily and intuitively adjust the tension of securement cord 426 around the secured structures or to fully release securement cord 426 at any time. As depicted, surgical securement apparatus 410 comprises a body 412, an extender tube 415, a release button 416, and an assembly window 418.

Body 412 comprises a housing for securing the other components of surgical securement apparatus 410. Body 412 provides desired structure to maintain operability of the components of surgical securement apparatus 410. Body 412 can be colored coded or otherwise labeled to enable a practitioner to quickly identify the intracorporeal structures which are secured by a particular surgical securement apparatus 410. For example, in one illustrative embodiment a practitioner can use a surgical securement apparatus with a red body to secure one or more arteries and to provide a clear indication to the practitioner that the structures secured are arteries. Similarly, a blue body 412 may be utilized as an indicator that one or more veins are being secured. A yellow body 412 may be utilized to indicate nerves, a green body 412 may indicate tendons, and an orange body 412 may indicate the target structure of the procedure.

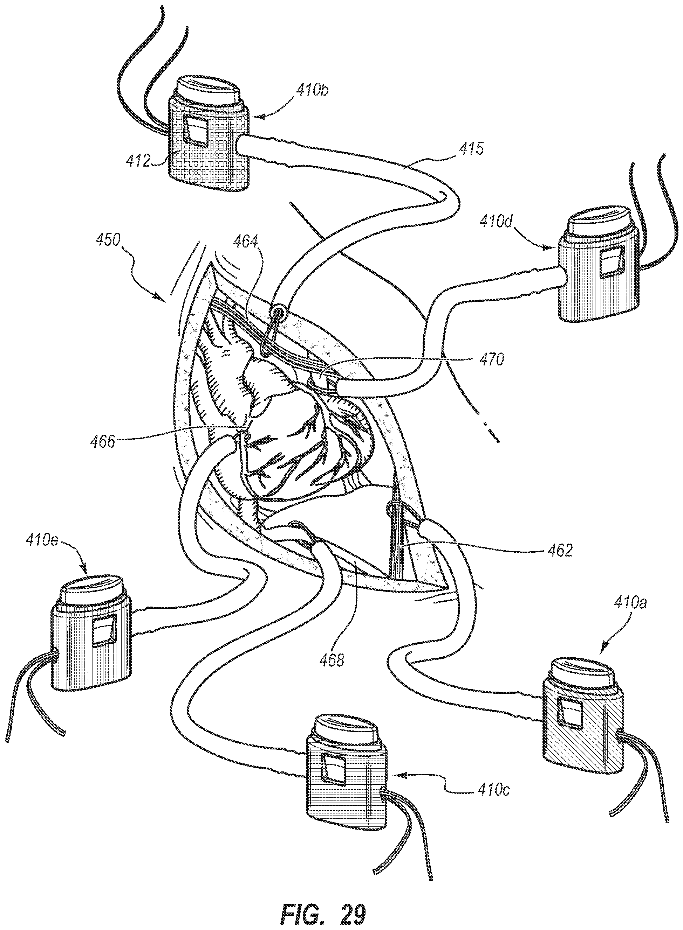

In another exemplary color-coding scheme, two surgical securement apparatus with a red body 412 can be utilized in a bypass surgery to indicate the blood vessels to be connected during the procedure. As can be appreciated by those skilled in the art, a variety of color-coding schemes can be utilized without departing from the scope of the present disclosure. In alternative embodiments, other types of non-textual indicia can be used, including symbols, shapes, and patterns. Moreover, other features such as labels and patterns can be used in connection with body 412 to convey information about the associated intracorporeal structures. Illustrative uses of color-coding and other marking systems in connection with surgical securement apparatus 410 will be discussed in greater detail below with reference to Fig. 29.

In the illustrated embodiment, extender tube 415 is coupled to body 412. Extender tube 415 comprises a resilient tubular member having a lumen formed therethrough. Extender tube 415 can be formed of a flexible material such as flexible PVC, silicone, or other flexible material. Extender tube 415 extends distally from body 412 to access a surgical site. The length of extender tube 415 allows the distal end of extender tube 415 to be positioned within the surgical site and adjacent to intracorporeal structures while body 412 of the surgical securement apparatus remains outside the surgical site. The configuration of extender tube 414 is adapted to facilitate engagement of a securement cord 426 which is utilized during a surgical procedure to secure and/or isolate intercorporeal structures. The securement cord 426 is threaded in a loop or other configuration around one or more intracorporeal structures. The securement cord 426 can then be threaded through the distal end of extender tube 415. The position of extender tube 415 allows surgical securement apparatus 410 to provide a desired degree of tension on the securement cord 426. In this manner, securement cord 426 can be secured from outside the surgical site by surgical securement apparatus 410, thus removing any potential for interference or interruption of the surgical site from body 412 of surgical securement apparatus 410.

Release button 416 is positioned within body 412 of surgical securement apparatus 410. Release button 416 allows a practitioner to selectively secure or release a cord positioned within surgical securement apparatus 410. Selective securement of the cord is accomplished by depressing and releasing release button 416. For example, in the illustrated embodiment when release button 416 is in a released position, surgical securement apparatus securely grips a cord positioned within surgical securement apparatus 410. When the user depresses release button 416, the grip exerted by surgical securement apparatus 410 is loosened, and manipulation of the cord positioned within surgical securement apparatus 410 or adjustment of the surgical securement apparatus 410 relative to the cord can be effectuated. The ability to selectively release and reposition the surgical securement apparatus 410 relative to a cord for which the surgical securement apparatus 410 is to be utilized can be desirable to the extent the practitioner desires to adjust the tension of the cord, reposition the surgical securement apparatus 410 relative to the patient, or perform other desired activities relative to the cord and/or the surgical securement apparatus 410. In particular, at the end of a surgical procedure the practitioner can quickly, simply and efficiently release the cord looped around a secured intracorporeal structures and remove the cord after a given aspect of the procedure is completed or before closing up the surgical site.

Assembly window 418 comprises an aperture in the sidewall of body 412 of surgical securement apparatus 410. Assembly window 418 allows for the quick snap fit assembly of release button 416 relative to body 412 of surgical securement apparatus 410. In the illustrated embodiment, release button 416 includes a biasing flange having a transverse length approximating the width of assembly window 418. During assembly, release button 416 is lowered into body 412 of surgical securement apparatus 410. As release button 416 is urged downward, the biasing flange is received within assembly window 418 preventing inadvertent removal of release button 416 from body 412.

In the illustrated embodiment, surgical securement apparatus 410 is utilized with threading assembly 420. Threading assembly 420 allows a practitioner to quickly and simply thread one or more tails of cord 426 from the exterior of surgical securement apparatus 410 and through a lumen of surgical securement apparatus 410 such that desired engagement of such cord 426 is effectuated. In the illustrated embodiment, threading assembly 420 comprises a threading loop 422 and a clasp 424. Threading loop 422 can comprise a length of nylon suture, or other cord-like material with sufficient strength to pull the tails of cord 426 through the lumen of surgical securement apparatus. Threading loop 422 extends from a proximal portion of surgical securement apparatus 410, along the length of a lumen of body 412 of surgical securement apparatus 410, and extends distally from an end of extender tube 415.

In the illustrated embodiment, clasp 424 includes a first lateral side and second lateral side. Cooperative engagement of the first lateral side and second lateral side secures a portion of threading loop 422 within clasp 424. Clasp 424 secures the end of threading loop 422 positioned on the proximal side of surgical securement apparatus 410. Clasp 424 prevents the accidental pulling of threading loop 422 through surgical securement apparatus 410 as a result of tension exerted on the portion of threading loop 422 positioned distal to surgical securement apparatus 410. Additionally, clasp 424 provides a simple and ergonomic mechanism to allow a user to control functioning of threading assembly 420 and of threading loop 422. According to one embodiment of the present disclosure, clasp 424 includes texturing or finger grips to allow for desired gripping of clasp 424.

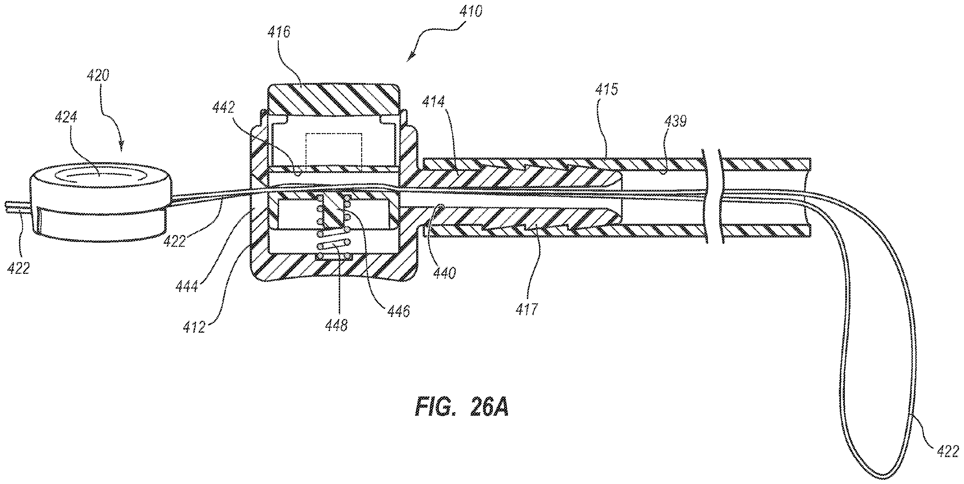

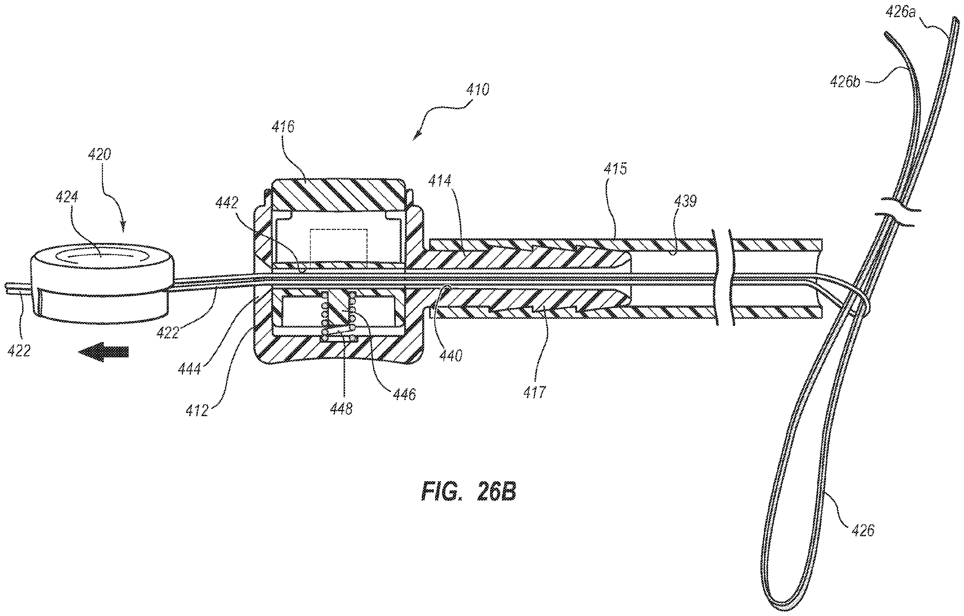

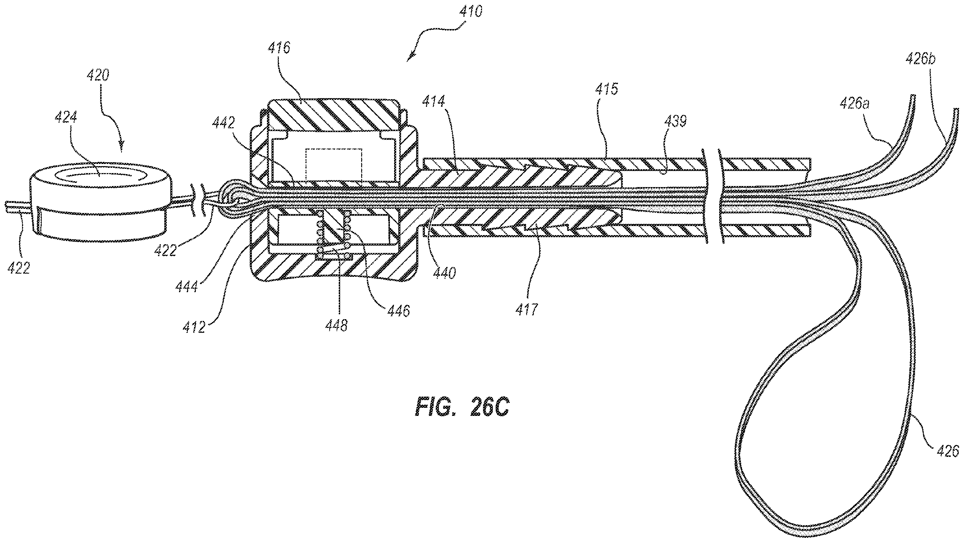

To utilize surgical securement apparatus 410, a user simply threads a first tail 426a and second tail 426b of a securement cord 426 through threading loop 422. Once the first tail 426a and second tail 426b are threaded through threading loop 422, the user grasps clasp 424 and begins to draw threading loop 422 in a proximal direction. As threading loop 422 is retracted in a proximal direction it begins to be drawn into extender tube 415. As threading loop 422 is drawn into extender tube 415, first tail 426a and second tail 426b are also drawn into extender tube 415 thus avoiding the complicated or cumbersome manual introduction of the tips of first tail 426a and second tail 426b into the somewhat small diameter of extender tube 415. A more complete description of the manner in which threading loop 422 draws first tail 426a and second tail 426b of cord 426 into surgical securement apparatus 410 will be described below with reference to Fig. 26.

As will be appreciate by those skilled in the art, a variety of types and configurations of surgical securement apparatus and threading assemblies can be utilized without departing from the scope of the present disclosure. According to one embodiment of the present disclosure, extender tube 415 can be coupled to body 412 via a connector that extends distally from body 412. The connector can further comprise a connector tip adapted to engage and secure extender tube 415 such that the lumen of extender tube 415 is in communication with a lumen of the connector. In another embodiment, extender tube 415 is coupled directly to body 412. In yet another embodiment, extender tube 415 is integrated with body 412. According to another embodiment, an extension mechanism other than an extension tube may be utilized.

According to another embodiment of the present disclosure, a mechanism to secure a cord other than a depressible button can be utilized. For example, a surgical securement apparatus can comprise a mechanism which has a first locking position to secure the cord and a second unlocked position to release securement of the cord. According to another embodiment, the surgical securement apparatus can be utilized with something other than a securement cord. For example, surgical securement apparatus can be used in conjunction with sutures in a surgical site to selectively secure one or more tails of a suture. A practitioner may use a temporary suture to secure a drainage catheter or other instrument within the surgical site. If the temporary suture must be removed at the end of the procedure, a surgical securement apparatus can be used to selectively secure the suture from outside the surgical site, and thereby prevent it from becoming lost within the surgical site.

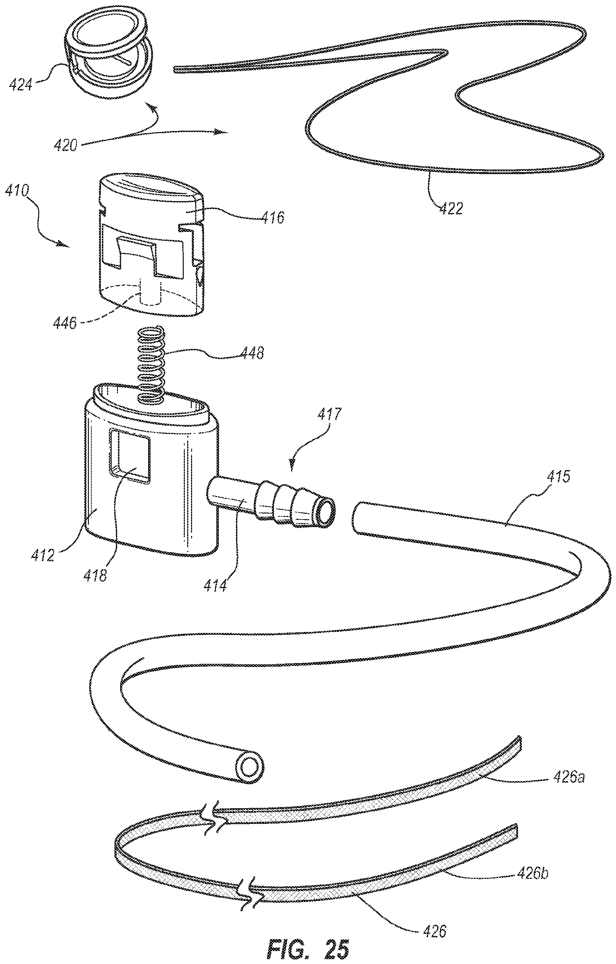

Fig. 25 is an exploded view of a surgical securement apparatus. As discussed with reference to Fig. 24, surgical securement apparatus 410 comprises a body 412, a flexible extender tube 415, a release button 416, and an assembly window 418. Threading loop 422 or sorting cord 426 can be threaded through surgical securement apparatus 410 by extending threading loop 422 through a lumen of body 412, a lumen of release button 416, and a lumen of extender tube 415.

In the illustrated embodiment, a connector 414 of surgical securement apparatus 410 is depicted. Connector 414 is secured to body 412 of surgical securement apparatus such that it that extends distally from body 412. Connector 414 provides a mechanism to connect body 412 to extender tube 415. Connector 414 comprises a lumen that integrates with and/or forms a portion of a lumen through surgical securement apparatus 410. According to one aspect, connector 414 has a tapered mouth, such that there is a smooth transition from the lumen of connector 414 to the lumen of extender tube 415. A tapered mouth facilitates threading of securement cord 426 through the lumen of surgical securement apparatus 410 by guiding first tail 426a and second tail 426b into the lumen of body 412. The tapered mouth of connector also minimizes snagging or interference with proper operation of surgical securement apparatus 410 as a practitioner retracts threading loop 422 and/or tightens securement cord 426 during use of surgical securement apparatus 410.

Connector 414 can further comprise a connector tip 417 that is configured to engage and secure extender tube 415. In the illustrated embodiment, connector tip 417 can include a barbed connector, such as a "Christmas tree" type connector. Connector tip 417 can comprise a plurality of tapered ramps, or barbs. The tapered ramps have a smaller cross-section at their distal end and increase in thickness as each ramp approaches its proximal most extent. The ramps are adapted to allow extender tube 415 to slide onto connector 414. The ramps then engage the interior of extender tube 415 and thereby restrict extender tube 415 from sliding in the opposite or distal direction such that the tube would be removed from connector 414. Connector tip 417 engages extender tube 415 in a manner that allows the lumen of extender tube 415 to be in communication with the lumen of connector 414, thus allowing a continuous lumen through both structures, as discussed more fully below with reference to Fig. 26A.

In the illustrated embodiment, release button 416 is adapted to be positioned within body 412. A biasing member 448 fits within body 412 in abutment with release button 416. The biasing member 448 can comprise a spring. The biasing member is adapted to be positioned around or adjacent to a post 446 of release button 416. Post 446 is positioned on the underside of release button 416 such that it is located within body 412 and in abutment with, and/or adjacent to, biasing member 448. Biasing member 448 exerts a biasing force urging release button 416 in an upward direction. As release button 416 is urged in an upward direction, a cord threaded through surgical securement apparatus 410 is secured by release button 416. A more complete description of the manner in which surgical securement apparatus 410 engages and secures a cord will be described below with reference to Fig. 26A.

As will be appreciated by those skilled in the art, a variety of types and configurations of surgical securement apparatus can be utilized without departing from the scope of the disclosure. For example, in one embodiment, the surgical securement apparatus is provided without a threading assembly. According to yet another embodiment, a connector other than a barbed "Christmas tree" connector can be utilized to secure an extender tube to a connector. For example, the connector may comprise a clip, a fastener, or a detent. In yet another embodiment, the connector tip includes ribs instead of barbs. In still another embodiment, no connector tip is employed, but rather the shape of the connector and/or a friction fit provided between the connector and the extender tube secures the extender tube relative to the body of the surgical securement apparatus.

Fig. 26A is a cross-sectional side view of surgical securement apparatus 410 according to one embodiment of the present disclosure. In the illustrated embodiment, a connector 414 includes a barbed connector tip 417 that engages and secures extender tube 415 relative to the other components of surgical securement apparatus 410. In the illustrated embodiment, barbed connector tip 417 comprises a "Christmas tree" connector. The barbs of connector tip 417 are adapted to allow extender tube 415 to slide onto connector 414. Once the extender tube 415 slides past a barb, the barb engages the inside of the extender tube and thereby restricts extender tube 415 from sliding in an opposite direction such that it is removed from connector 414. Barbed connector tip 417 engages and secures extender tube 415 in a manner that lumen 439 of extender tube 415 is in communication with lumen 440 of connector 414. The connector lumen 440 runs through the center of connector tip 417. Connector tip 417 is inserted and received into extender tube lumen 439, thus allowing a continuous lumen through both structures.

The cross-sectional view of Fig. 26A also illustrates that lumen 440 of connector 414 can have a tapered distal aperture to facilitate threading of securement cord 426 through surgical securement apparatus 410. Tapering of the distal aperture provides a larger opening to receive the threaded first tail 426a and second tail 426b of securement cord 426. Tapering also reduces the surface area of connector tip 417 at the interface of lumen 439 of extender tube 415 and lumen 440 of connector 414. The tapered distal aperture thus minimizes the possibility that cord 426 could get snagged while being pulled through surgical securement apparatus 410 by threading assembly 420.

Fig. 26A further depicts a rear aperture 444. Rear aperture 444 receives threading loop 422 as it is inserted into surgical securement apparatus 410. The tails of a securement cord 426 extend proximally from rear aperture 444 once cord 426 has been threaded through surgical securement apparatus 410. In the illustrated embodiment, rear aperture 444 is positioned in substantial alignment with connector lumen 440. Rear aperture 444 has a tapered configuration which facilitates the loading of threading loop 422 into surgical securement apparatus 410. According to one alternative embodiment of the present disclosure, a tapered extension tube is provided in place of or in addition to a rear aperture 444.

In the illustrated embodiment, release button 416 is depicted in an undepressed position. Threading loop 422 extends through surgical securement apparatus 410 such that a portion of threading loop 422 projects distally from extender tube 415. The portion of threading loop 422 which extends distally of extender tube 415 is utilized to capture a securement cord and thread the securement cord through surgical securement apparatus 410.

In the illustrated embodiment, when release button 416 is in an undepressed position, threading loop 422 is engaged between release button 416 and body 412 restricting movement of threading loop 422 relative to surgical securement apparatus 410. Spring member 448 is positioned adjacent post 446 such that spring member 448 exerts a biasing force on post 446 to urge release button 416 in an upward direction. When release button 416 is urged to its upward most displacement relative to body 412, release button lumen 442 is placed slightly out of alignment with connector lumen 440. In this manner threading loop 422, or anything threaded through the lumen of surgical securement apparatus, is compressed between the bottom surface of release button lumen 442 and both the top of connector lumen 440 and the top surface of rear aperture 444. The cooperative engagement of the surfaces of connector lumen 440, release button 442, and rear aperture 444 provide an effective mechanism for securing a desired degree of tension on threading loop or another cord threaded along the length of surgical securement apparatus 410.

Fig. 26B depicts release button 416 in a depressed position. The practitioner depresses release button 416 when loosening the tension is desired. As the practitioner depresses release button 416, the release button 416 is urged in a downward direction and spring member 448 is depressed. Additionally, as release button 416 is urged in a downward direction, release button lumen 442 aligns with connector lumen 440 and rear aperture 444. As a result, threading loop 422 is no longer cooperatively engaged between opposing surfaces of release button lumen 442, connector lumen 440, and rear aperture 444. When threading loop 422 is not cooperatively engaged, the securement on threading loop 422 is released and threading loop 422 is moveable with respect to surgical securement apparatus 410.

When the release button 416 is depressed and the threading loop 422 is released, clasp 424 can be retracted in a rearward direction to pull threading loop 422 in a proximal direction. In the illustrated embodiment, first tail 426a and second tail 426b are threaded through threading loop 422. As clasp 424 is retracted in a rearward direction, the portion of threading loop 422 projecting distally from extender tube 415 is drawn into an extender tube lumen 439. Because first tail 426a and second tail 426b are threaded through threading loop 422, retraction of threading loop 422 pulls first tail 426a and second tail 426b into extender tube lumen 439. As clasp 424 is further retracted in a rearward direction, threading loop 422 is drawn into a connector lumen 440 of connector 414. Accordingly, first tail 426a and second tail 426b are also pulled into connector lumen 440. The distal end of connector 414 may be tapered, as depicted, to thereby facilitate guidance of first tail 426a and second tail 426b into connector lumen 440.

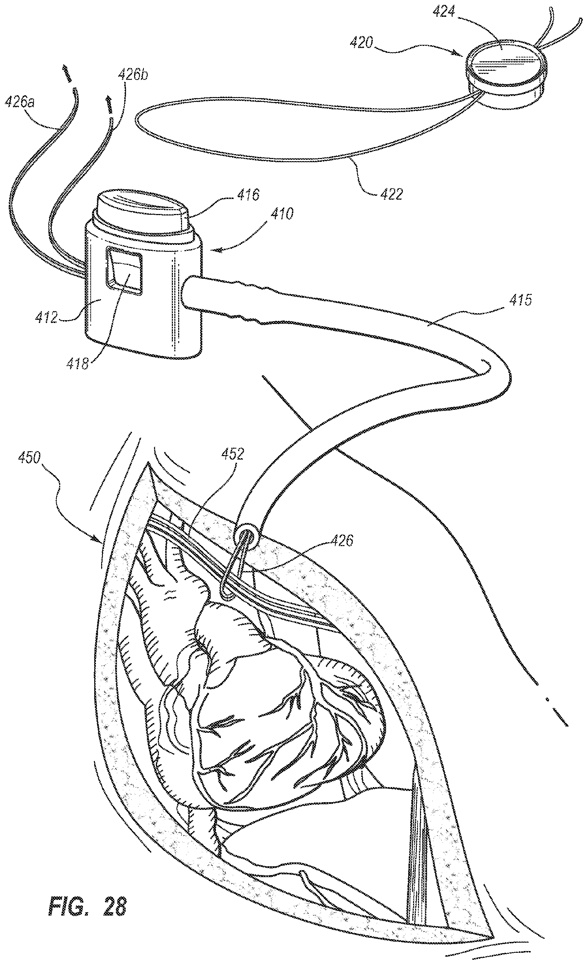

Fig. 26C illustrates the manner in which clasp 424 and threading loop 422 are utilized to draw securement cord 426 into surgical securement apparatus 410. In the illustrated embodiment, release button 416 is in a depressed position similar to the position of release button 416 in Fig. 26B. When release button 416 is in a depressed position, release button lumen 442 aligns with connector lumen 440 and rear aperture 444. As threading loop 422 is pulled in rearward direction, first tail 426a and second tail 426b are also drawn into release button lumen 442. As will be appreciated by those skilled in the art, as the practitioner continues to retract threading assembly 420 in a rearward direction, first tail 426a and second tail 426b are pulled along the entire length of surgical securement apparatus 410 until first tail 426a and second tail 426b extend out of a rear aperture 444 of surgical securement apparatus 410, as depicted in Fig. 28. In this manner, threading assembly 420 facilitates the quick, simple and intuitive threading of first tail 426a and second tail 426b of securement cord 426 through surgical securement apparatus 410.

Once securement cord 426 is completely threaded through surgical securement apparatus 410, a practitioner may desire to secure the position of securement cord 426 within securement apparatus 410. To secure securement cord, the practitioner simply allows button 410 to be released to an undepressed position. As described above with reference to Fig. 26A, release button 416 engages securement cord 426 when release button 416 is in an undepressed position. Release button 426 engages securement cord 426 by compressing securement cord 426 between the bottom surface of release button lumen 442 and both the top of connector lumen 440 and the top surface of rear aperture 444. Thus, release button 416 provides selective securement of securement cord 426.

The practitioner can also release engagement of securement cord 426 by depressing release button 416. As described above with reference to Fig. 26B, as release button 416 is urged in a downward direction, release button lumen 442 becomes aligned with connector lumen 440 and rear aperture 444. Securement cord 426 is no longer cooperatively engaged between opposing surfaces of release button lumen 442, connector lumen 440, and rear aperture 444. Accordingly, the practitioner can move surgical securement apparatus 410 laterally relative to first tail 426a and second tail 426b. Releasing of the securement of securement cord 426 by depression of release button 416 allows the practitioner to loosen, tighten, or make other changes in the juxtaposition of surgical securement apparatus 410 relative to securement cord 426 and the one or more intracorporeal structures thereby secured.

As will be appreciated by those skilled in the art, a variety of types and configurations of surgical securement apparatus can be utilized without departing from the scope of the disclosure. For example, the juxtaposition and mechanisms utilized to secure a cord relative to the surgical securement apparatus can vary without departing from the scope of the present disclosure. For example, a cord which is threaded through the release button may be cooperatively engaged by surfaces other than the bottom surface of the release button and the top of the connector lumen and top of the rear aperture. Additionally, the lumen of the release button may vary in size. A top surface of the lumen may remain fixed, while the bottom surface of the lumen clamps against the top surface as the release button is depressed. The top and bottom surfaces of the release button lumen and other lumens can further comprise one or more protrusions or other non-slip surfaces to facilitate desired engagement of a cord. In one embodiment, the top and bottom surfaces of the lumen can include grooves which receive protrusions from the opposite surface thereby enhancing engagement of the cord by the protrusions.

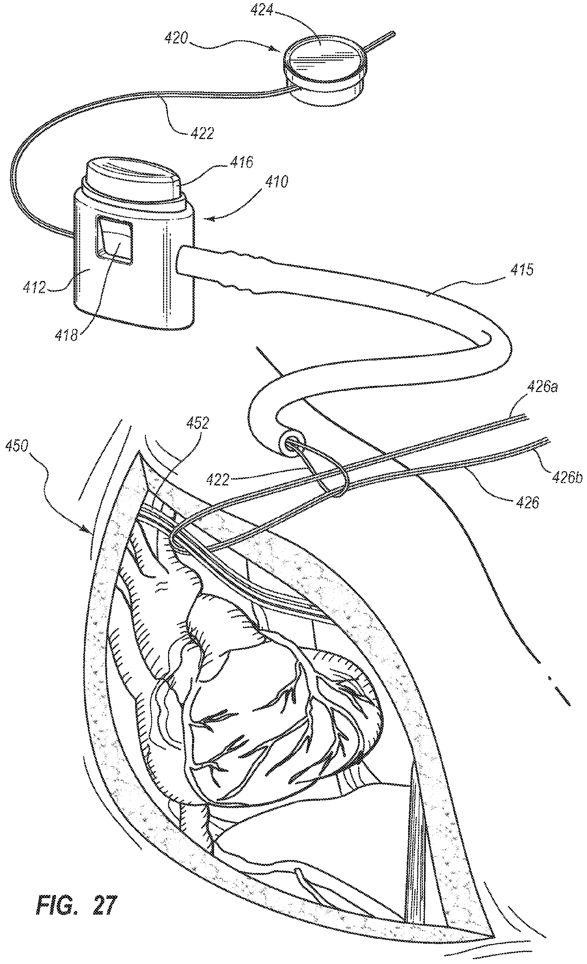

Fig. 27 is a perspective view of a surgical securement apparatus 410 being utilized in connection with a surgical procedure. In the illustrated embodiment, securement cord 426 is threaded around one or more intracorporeal structures 452 within a surgical site 450. First tail 426a and second tail 426b of securement cord 426 have been threaded through threading loop 422. Threading assembly 420 is partially retracted, such that threading loop 422 has been advanced adjacent to the tip of extender tube 415. As threading loop 422 is further retracted, threading loop 422 will draw first tail 426a and second tail 426b into the lumen of extender tube 415.

Securement cord 426 facilitates desired securement of intracorporeal structures 452 within the patient. In the illustrated embodiment, the depicted surgical environment is an open heart surgery. The surgical site 450 is opened exposing a variety of intracorporeal structures including the heart, various vessels and intracorporeal structures 452. As will be appreciated by those skilled in the art, intracorporeal structures 452 are representative of any of plurality of different intracorporeal structures that may need to be secured during a surgical procedure. Securement cord 426 is threaded around intracorporeal structures 452 allowing a practitioner to hold, secure, manipulate or even draw intracorporeal structures 452 from surgical site 450 utilizing securement cord 426.

First tail 426a and second tail 426b have been threaded through threading loop 422. The design of threading loop 422 and threading assembly 420 allows the practitioner to simply and easily draw first tail 426a and second tail 426b through threading loop 422. For example, in the even that a larger diameter of threading loop 422 positioned distal to extension tube 415 is needed, the practitioner can simply advance threading loop 422 in a distal direction. To advance threading loop 422, the user simply depresses release button 416, and advances clasp 424 in the direction of surgical securement apparatus 410. By advancing clasp 424 in the direction of surgical securement apparatus 410, the suture material comprising threading loop 422 is also advanced such that a greater portion of threading loop 422 is positioned distal to the tip of extender tube 415. In this manner, a larger threading loop 422 is provided facilitating a straightforward threading of first tail 426a and second tail 426b through threading loop 422. Once first tail 426a and second tail 426b have been threaded through threading loop 422, a user can simply draw securement cord 426 adjacent the tip of extender tube by retracting clasp 424 away from threading assembly 420.

Fig. 28 is a perspective view of a surgical securement apparatus 410 in which securement cord 426 has been threaded through surgical securement apparatus 410. In the illustrated embodiment, securement cord 426 is positioned around various intracorporeal structures 452 to be secured during a surgical procedure. Threading assembly 420 has been fully retracted, such that threading loop 422 is withdrawn from surgical securement apparatus 410. Accordingly, first tail 426a and second tail 426b of securement cord 426 have been threaded along the entire length of surgical securement apparatus, through extender tube 415, connector 414, and body 412, such that first tail 426a and second tail 426b are extending from rear aperture 444 (See also Figs. 26A-26C). Surgical securement apparatus 410 and extender tube 415 of surgical securement apparatus 410, have been advanced forward along securement cord 426 such that the distal end of extender tube 415 is positioned adjacent intracorporeal structures 452 and the loop in securement cord 426 can be held taut. Although the figure depicts the securement cord 426 as being somewhat loose, it can be appreciated by those skilled in the art that the tension of the securement cord can be adjusted as desired.

Extender tube 415 enables securement of intracorporeal structures 452 within surgical site 450 while allowing the body of surgical securement apparatus 410 to remain outside surgical site 450. Extender tube 415 provides displacement between the tip of extender tube 415 and body 412 of surgery securement apparatus. The length of extender tube 415 can be selected to ensure that the body of surgical securement apparatus 410 remains outside surgical site 450 while the distal tip of extender tube 415 can be placed immediately adjacent one or more intracorporeal structures 452. The displacement from the distal tip of extender tube and body 412 allows desired securement at the tip of extender tube 415 while providing actuation of button 416 at a desired displacement from surgical site 450.

Body 412 can be positioned outside of surgical site 450, so as to not obstruct any structures or otherwise impede the surgical procedure. According to one aspect of the disclosure, surgical securement apparatus 410 is configured to hang outside of surgical site 450 such that body 412 can provide an appropriate amount of weight to stabilize and/or draw ancillary structures to the periphery of surgical site 454. Additionally, the position of body 412 makes body 412 and button 416 readily accessible for a practitioner in the event that a practitioner desires to manipulate a desired structure to which surgical securement apparatus 410 is secured.

In the illustrated embodiment, body 412 of surgical securement apparatus 410 can be colored to provide an indication of the type of intracorporeal structures 452 being secured with the corresponding securement cord 426. For example, body 412 of surgical securement apparatus 410 can be colored blue. Color coding or other sorting indicia allow the practitioner to sort and secure veins. Additionally, the particular color or other sorting indicia can be utilized to communicates to a practitioner the type of structures being secured. In one illustrative embodiment, the color blue can indicate to the practitioner that the secured structures are veins.

Fig. 29 illustrates an exemplary embodiment of an surgical securement and marking system employing multiple color-coded surgical securement apparatus to secure and mark various intracorporeal structures within a surgical site 450 of coronary bypass surgery. The surgical securement apparatus are color-coded to aid practitioners in identifying the intracorporeal structures which are secured by surgical securement apparatus 410a-410e. The color-coding provides an indication of the associated structure so that the practitioner does not need to independently examine the structure to identify it again after the structure is secured. Other non-textual indicia may be utilized in connection with the color-coding, or in alternative to the color-coding, such as symbols and patterns.

In the illustrated embodiment, surgical securement apparatus 410a is colored green indicating that the corresponding sorted and secured intracorporeal structure is a tendon 462. Surgical securement apparatus 410b is colored yellow indicating that the corresponding sorted and secured intracorporeal structure is a nerve 464. Surgical securement apparatus 410c is colored blue indicating that the corresponding structure is a vein 466. Surgical securement apparatus 410d and 410e are colored red indicating that the corresponding structures are arteries 468, 470.

The color coded system can be adapted for various purposes and procedures. For examples, surgical securement apparatus of like color can be used to associate various intracorporeal structures in ways other than structure type. For example, surgical securement apparatus 410d and 410e can both be colored red to indicate that the structures secured by each are to be connected together during a procedure such as coronary bypass. In one exemplary method of performing coronary bypass surgery, the mammary artery is severed and connected to the coronary artery below the blockage, thereby providing oxygen-rich blood to the heart tissue below the blockage. Surgical securement apparatus that are color-coded with like colors can be used to sort and secure the mammary artery and the coronary artery. For example, surgical securement apparatus 410d may secure the mammary artery 468 while surgical securement apparatus 410e may secure the blocked coronary artery 470 to be bypassed. The practitioner is thereby aided in avoiding operating on an incorrect blood vessel, and can quickly identify the connection that needs to be made to complete the bypass.

In another exemplary method of performing coronary bypass surgery, a blood vessel, usually a harvested vein from another part of the patient's body, is connected to the aortic artery at one end, and then to the blocked coronary artery at a position below the blockage, thereby supplying the tissue below the blockage with a supply of oxygen rich blood. The ends of the harvested blood vessels, the aorta, and the coronary artery to be bypassed can be sorted and secured by various securement cords and surgical securement apparatus. The surgical securement apparatus can be color coded to provide easy identification of the various parts to be connected by the procedure. For example, the aorta and one end of the harvested vessel may each be secured by surgical securement apparatus having the same color. Similarly, the other end of the harvested vessel and the coronary artery may each be secured by like colored surgical securement apparatus having a color different than the other surgical securement apparatus. In this manner, the practitioner can easily identify the vessels to be connected in the surgery.

In another embodiment of a surgical securement and marking system, colors and other non-textual indicia may indicate the structures associated with the steps of a surgical procedure. For example, a practitioner may secure and mark one or more intracorporeal structures involved in a first step of the procedure with a particular color. Similarly, the one or more structures of a second step may be marked with another color, and so on. In another embodiment, surgical securement apparatus colored similarly may be used to secure and mark ancillary structures that are not involved in the procedure. For example, gray surgical securement apparatus may indicate the associated structures are not being treated during the procedure and can be moved to the periphery of the surgical site or otherwise ignored by the practitioner.

Other types of non-textual indicia can also be utilized for marking. Examples of other non-textual indicia include symbols and patterns. Particular symbols may be placed on body 412 of surgical securement apparatus to quickly convey information to practitioners. For example, in Fig. 29, a star or other symbol may be integrated on both surgical securement apparatus 410d and surgical securement apparatus 410e. The practitioner can readily recognize that the structures secured by surgical securement apparatus having like symbols, such as stars, are associated. In the case of bypass surgery, the association can be that the structures that are to be connected during the procedure. The symbols can also be colored to provide additional information. Patterns can be used in a similar manner. For example, a red body 412 of a surgical securement apparatus 410d may further comprise a pattern such as three parallel lines or stripes. The practitioner can readily recognize an association of intracorporeal structures secured by such apparatus with structures secured by an apparatus having the same or corresponding pattern. Moreover, other features such as labels can be included on body 412 to convey additional information about the associated intracorporeal structures.

As will be understood by those skilled in the art, utilization of the surgical securement apparatus with a securement cord to secure intracorporeal structures is merely an exemplary embodiment illustrating operation of the surgical securement apparatus relative to securing such intracorporeal structure and should in no means be considered to be limiting in nature. For example, the surgical securement apparatus can be utilized with sutures that are positioned within the surgical site. Additionally, the surgical securement apparatus can be utilized for securing additional medical devices such as a suture ring of a catheter hub to a patient. Moreover, the color-coding examples disclosed are merely exemplary illustrations and should in no means be considered to be limiting in nature. For example, symbols, patterns, texturing or other indicia can be utilized with a sorting and securement system. A desired securement and sorting system can be adapted for particularized procedures, medical specialties, medical facilities, or even individual surgical teams, and available colors can be adapted in a variety of ways to conform to any particular color-coding scheme.

The present invention may be embodied in other specific forms without departing from its scope or essential characteristics. The described embodiments are to be considered in all respects only as illustrative and not restrictive. The scope of the invention is, therefore, indicated by the appended claims rather than by the foregoing description.

## Claims

1. A suture securement apparatus (10, 110) for selectively and releasably securing the ends of a suture utilized in a purse string suture utilized to maintain hemostasis and close a percutaneous catheter puncture site, the suture securement apparatus comprising:
a body (12, 112) having a cavity positioned therein;
a plunger (16, 116) slidably disposed within the cavity of the body and biased to secure the sutures and allow for releasable engagement of the sutures;
an extension tube (14, 114) extending a predetermined distance from the body such that a tip of the extension tube is adaptable to engage the sutures adjacent the suture insertion point without interruption from the body; and
a threading assembly (20, 120) comprising a clasp (24, 122) and a suture engaging portion (22, 124), the suture engaging portion (22, 124) positioned through a lumen of the suture securement apparatus and the clasp (24, 122) configured to secure a portion of the suture engaging portion on a proximal side of the securement apparatus.

2. The suture securement apparatus of claim 1, further comprising a biasing element (48) positioned within the cavity of the body and engaging the plunger (16, 116) to bias the plunger (16, 116) and allow for releasable engagement of the sutures.

3. The suture securement apparatus of claim 1, wherein the suture engaging portion comprises a suture loop (22).

4. The suture securement apparatus of either claim 1 or claim 2, wherein the plunger comprises a release button (16, 116).

5. The suture securement apparatus of claim 2, wherein the extension tube (14, 114) comprises an extension tube lumen (40, 140).

6. The suture securement apparatus of claim 5, wherein the plunger (16, 116) includes a plunger lumen (42, 142).

7. The suture securement apparatus of claim 6, wherein sutures secured by the suture securement apparatus can be moved relative to the suture securement apparatus when the extension tube lumen (40, 140) and the plunger lumen (42, 142) are aligned.

8. The suture securement apparatus of claim 6, wherein sutures secured by the suture securement apparatus are secured relative to the suture securement apparatus when the extension tube lumen (40, 140) and the plunger lumen (42, 142) are not aligned.

9. The suture securement apparatus of claim 2, wherein the extension tube (14, 114) is integrally coupled to the body (12, 112) of the suture securement apparatus and the extension tube (14, 114) is tapered such that the tip of the extension tube has a smaller outside diameter than the outside diameter of the portion of the extension tube positioned adjacent the body.

10. The suture securement apparatus of claim 1, wherein the plunger (16, 116) is biased toward an undepressed position.

11. A suture securement apparatus of claim 1, wherein the suture engaging portion of the threading assembly comprises a wire (124).

12. A suture securement apparatus of claim 11, wherein the wire (124) is configured to form an insertion assembly (126).

13. The suture securement apparatus of claim 12, wherein the insertion assembly (126) comprises a pointed tip (128) and one or more angular portions (127) to facilitate threading the insertion assembly (126) through the lumen of the suture securement apparatus.

14. The suture securement apparatus of claim 1, wherein the plunger (116) comprises a lumen (142) through the plunger, wherein the plunger lumen (142) forms a portion of the lumen of the suture securement apparatus and wherein the plunger lumen (142) is configured to have a cross-section in the shape of a tear drop, and wherein at least a portion of a lumen (140) of the extension tube is configured to have a cross-section in the shape of a tear drop and oriented such that a pointed portion of the tear drop is oriented 180 degrees relative to the pointed portion of the tear drop shape of the plunger lumen cross-section.

15. The suture securement apparatus of claim 14, wherein when the plunger (116) is in a fully depressed position, a portion of the tear drop shape having a larger cross-section and being associated with the plunger (116) is aligned with a portion of the tear drop shape having a larger cross-section and being associated with the extension tube (114) such that a suture threaded through the lumen of the suture securement apparatus is released and moveable relative to the suture securement apparatus, and wherein when the plunger (116) is in a fully undepressed position the pointed portion of the tear drop shape cross-section of the plunger (116) is aligned with a pointed portion of the tear drop shaped cross-section of the extension tube (114) such that a suture threaded through the lumen of the suture securement apparatus is engaged and secured relative to the suture securement apparatus.

## Patentansprüche

1. Fadenfesthaltevorrichtung (10, 110) zum selektiven und lösbaren Festhalten der Enden eines in einer Raffnaht zum Aufrechterhalten von Hämostase und Schließen einer perkutanen Kathetereinstichstelle verwendeten Fadens, wobei die Fadenfesthaltevorrichtung Folgendes umfasst:
einen Körper (12, 112) mit einer darin positionierten Höhlung;
einen Kolben (16, 116), der gleitfähig in der Höhlung des Körpers angeordnet ist und gespannt ist, um die Fäden festzuhalten und den lösbaren Eingriff mit den Fäden zu ermöglichen;
eine Verlängerungsröhre (14, 114), die sich um eine vorgegebene Strecke von dem Körper erstreckt, sodass eine Spitze der Verlängerungsröhre anpassbar ist, um der Fadeneinführstelle benachbart ohne Unterbrechung von dem Körper mit den Fäden in Eingriff zu gelangen; und
eine Einfädelanordnung (20, 120), umfassend eine Klemme (24, 122) und einen Fadeneingriffsabschnitt (22, 124), wobei der Fadeneingriffsabschnitt (22, 124) durch ein Lumen der Fadenfesthaltevorrichtung positioniert ist und die Klemme (24, 122) dazu konfiguriert ist, einen Abschnitt des Fadeneingriffsabschnitts auf einer proximalen Seite der Festhaltevorrichtung festzuhalten.

2. Fadenfesthaltevorrichtung nach Anspruch 1, ferner umfassend ein Spannelement (48), das in der Höhlung des Körpers positioniert ist und sich mit dem Kolben (16, 116) im Eingriff befindet, um den Kolben (16, 116) zu spannen und den lösbaren Eingriff mit den Fäden zu ermöglichen.

3. Fadenfesthaltevorrichtung nach Anspruch 1, wobei der Fadeneingriffsabschnitt eine Fadenschlinge (22) umfasst.

4. Fadenfesthaltevorrichtung nach entweder Anspruch 1 oder Anspruch 2, wobei der Kolben eine Lösetaste (16, 116) umfasst.

5. Fadenfesthaltevorrichtung nach Anspruch 2, wobei die Verlängerungsröhre (14, 114) ein Verlängerungsröhrenlumen (40, 140) umfasst.

6. Fadenfesthaltevorrichtung nach Anspruch 5, wobei der Kolben (16, 116) ein Kolbenlumen (42, 142) umfasst.

7. Fadenfesthaltevorrichtung nach Anspruch 6, wobei von der Fadenfesthaltevorrichtung festgehaltene Fäden relativ zu der Fadenfesthaltevorrichtung bewegt werden können, wenn das Verlängerungsröhrenlumen (40, 140) und das Kolbenlumen (42, 142) aufeinander ausgerichtet sind.

8. Fadenfesthaltevorrichtung nach Anspruch 6, wobei von der Fadenfesthaltevorrichtung festgehaltene Fäden relativ zu der Fadenfesthaltevorrichtung festgehalten werden, wenn das Verlängerungsröhrenlumen (40, 140) und das Kolbenlumen (42, 142) nicht aufeinander ausgerichtet sind.

9. Fadenfesthaltevorrichtung nach Anspruch 2, wobei die Verlängerungsröhre (14, 114) einstückig mit dem Körper (12, 112) der Fadenfesthaltevorrichtung gekoppelt ist und die Verlängerungsröhre (14, 114) verjüngt ist, sodass die Spitze der Verlängerungsröhre einen kleineren Außendurchmesser als den Außendurchmesser des dem Körper benachbart positionierten Abschnitts der Verlängerungsröhre aufweist.

10. Fadenfesthaltevorrichtung nach Anspruch 1, wobei der Kolben (16, 116) in Richtung einer nicht eingedrückten Stellung gespannt ist.

11. Fadenfesthaltevorrichtung nach Anspruch 1, wobei der Fadeneingriffsabschnitt der Einfädelvorrichtung einen Draht (124) umfasst.

12. Fadenfesthaltevorrichtung nach Anspruch 11, wobei der Draht (124) dazu konfiguriert ist, eine Einführanordnung (126) zu bilden.

13. Fadenfesthaltevorrichtung nach Anspruch 12, wobei die Einführanordnung (126) eine spitze Spitze (128) und einen oder mehrere eckige Abschnitte (127) umfasst, um das Einfädeln der Einführanordnung (126) durch das Lumen der Fadenfesthaltevorrichtung zu erleichtern.

14. Fadenfesthaltevorrichtung nach Anspruch 1, wobei der Kolben (116) ein Lumen (142) durch den Kolben umfasst, wobei das Kolbenlumen (142) einen Abschnitt des Lumens der Fadenfesthaltevorrichtung bildet und wobei das Kolbenlumen (142) dazu konfiguriert ist, einen Querschnitt in Form eines Tropfens aufzuweisen, und wobei mindestens ein Abschnitt eines Lumens (140) der Verlängerungsröhre dazu konfiguriert ist, einen Querschnitt in Form eines Tropfens aufzuweisen, und derart orientiert ist, dass ein spitzer Abschnitt des Tropfens unter 180 Grad relativ zu dem spitzen Abschnitt der Tropfenform des Kolbenlumenquerschnitts orientiert ist.

15. Fadenfesthaltevorrichtung nach Anspruch 14, wobei, wenn sich der Kolben (116) in einer ganz eingedrückten Stellung befindet, ein Abschnitt der Tropfenform, der einen größeren Querschnitt aufweist und mit dem Kolben (116) assoziiert ist, auf einen Abschnitt der Tropfenform, der einen größeren Querschnitt aufweist und mit der Verlängerungsröhre (114) assoziiert ist, ausgereichtet ist, sodass ein durch das Lumen der Fadenfesthaltevorrichtung gefädelter Faden gelöst und relativ zu der Fadenfesthaltevorrichtung bewegbar ist, und wobei, wenn sich der Kolben (116) in einer ganz nicht eingedrückten Stellung befindet, der spitze Abschnitt des tropfenförmigen Querschnitts des Kolbens (116) auf einen spitzen Abschnitt des tropfenförmigen Querschnitts der Verlängerungsröhre (114) ausgerichtet ist, sodass sich ein durch das Lumen der Fadenfesthaltevorrichtung gefädelter Faden im Eingriff befindet und relativ zu der Fadenfesthaltevorrichtung festgehalten wird.

## Revendications

1. Appareil de fixation de suture (10, 110) destiné à fixer sélectivement et de manière libérable les extrémités d'une suture utilisée dans une suture en cordon de bourse utilisée pour maintenir l'hémostase et fermer un site de perforation de cathéter percutané, l'appareil de fixation de suture comprenant :
un corps (12, 112) ayant une cavité positionnée dans celui-ci ;
un plongeur (16, 116) disposé de manière coulissante au sein de la cavité du corps et sollicité pour fixer les sutures et permettre l'entrée en prise de manière détachable des sutures ;
un tube d'extension (14, 114) s'étendant sur une distance prédéterminée à partir du corps de sorte qu'une pointe du tube d'extension peut être adaptée pour entrer en prise avec les sutures adjacentes au point d'insertion de suture sans interruption du corps ; et
un ensemble d'enfilage (20, 120) comprenant un fermoir (24, 122) et une partie d'entrée en prise de suture (22, 124), la partie d'entrée en prise de suture (22, 124) positionnée à travers une lumière de l'appareil de fixation de suture et le fermoir (24, 122) configuré pour fixer une partie de la partie d'entrée en prise de suture sur un côté proximal de l'élément de fixation.

2. Appareil de fixation de suture selon la revendication 1, comprenant en outre un élément de sollicitation (48) positionné au sein de la cavité du corps et entrant en prise avec le plongeur (16, 116) pour solliciter le plongeur (16, 116) et permettre l'entrée en prise des sutures de manière détachable.

3. Appareil de fixation de suture selon la revendication 1, dans lequel la partie d'entrée en prise de suture comprend une boucle de suture (22).

4. Appareil de fixation de suture selon soit la revendication 1 ou la revendication 2, dans lequel le plongeur comprend un bouton de libération (16, 116).

5. Appareil de fixation de suture selon la revendication 2, dans lequel le tube d'extension (14, 114) comprend une lumière de tube d'extension (40, 140).

6. Appareil de fixation de suture selon la revendication 5, dans lequel le plongeur (16, 116) inclut une lumière de plongeur (42, 142).

7. Appareil de fixation de suture selon la revendication 6, dans lequel les sutures fixées par l'appareil de fixation de suture peuvent être déplacées par rapport à l'appareil de fixation de suture quand la lumière de tube d'extension (40, 140) et la lumière de plongeur (42, 142) sont alignées.

8. Appareil de fixation de suture selon la revendication 6, dans lequel les sutures fixées par l'appareil de fixation de suture sont fixées par rapport à l'appareil de fixation de suture quand la lumière de tube d'extension (40, 140) et la lumière de plongeur (42, 142) ne sont pas alignées.

9. Appareil de fixation de suture selon la revendication 2, dans lequel le tube d'extension (14, 114) est couplé d'un seul tenant au corps (12, 112) de l'appareil de fixation de suture et le tube d'extension (14, 114) est effilé de sorte que la pointe du tube d'extension a un diamètre extérieur inférieur au diamètre extérieur de la partie du tube d'extension positionnée adjacente au corps.

10. Appareil de fixation de suture selon la revendication 1, dans lequel le plongeur (16, 116) est sollicité vers une position non enfoncée.

11. Appareil de fixation de suture selon la revendication 1, dans lequel la partie d'entrée en prise de suture de l'ensemble d'enfilage comprend un fil (124).

12. Appareil de fixation de suture selon la revendication 11, dans lequel le fil (124) est configuré pour former un ensemble d'insertion (126).

13. Appareil de fixation de suture selon la revendication 12, dans lequel l'ensemble d'insertion (126) comprend une pointe pointue (128) et une ou plusieurs parties angulaires (127) pour faciliter l'enfilement de l'ensemble d'insertion (126) à travers la lumière de l'appareil de fixation de suture.

14. Appareil de fixation de suture selon la revendication 1, dans lequel le plongeur (116) comprend une lumière (142) à travers le plongeur, dans lequel la lumière de plongeur (142) forme une partie de la lumière de l'appareil de fixation de suture et dans lequel la lumière de plongeur (142) est configurée pour avoir une section transversale en forme de goutte, et dans lequel au moins une partie d'une lumière (140) du tube d'extension est configurée pour avoir une section transversale en forme de goutte et orientée de sorte qu'une partie pointue de la goutte est orientée à 180° par rapport à la partie pointue de la forme de goutte de la section transversale de lumière de plongeur.

15. Appareil de fixation de suture selon la revendication 14, dans lequel quand le plongeur (116) est dans une position entièrement enfoncée, une partie de la forme de goutte ayant une plus grande section transversale et étant associée avec le plongeur (116) est alignée avec une partie de la forme de goutte ayant une plus grande section transversale et étant associée avec le tube d'extension (114) de sorte qu'une suture enfilée à travers la lumière de l'appareil de fixation de suture est libérée et mobile par rapport à l'appareil de fixation de suture, et dans lequel quand le plongeur (116) est dans une position entièrement non enfoncée la partie pointue de la section transversale en forme de goutte du plongeur (116) est alignée avec une partie pointue de la section transversale en forme de goutte du tube d'extension (114) de sorte qu'une suture enfilée à travers la lumière de l'appareil de fixation de suture est entrée en prise et fixée par rapport à l'appareil de fixation de suture.
